# EUROPEAN PATENT APPLICATION

(11) **EP 3 767 629 A1**
(43) Date of publication of application: **20.01.2021**
(21) Application number: 19767188.6
(22) Date of filing: 15.03.2019
(51) Int. Cl.: G16B 40/00, A61K 39/00, A61K 39/395, A61P 37/02, G01N 33/53, G16B 15/30

(54) **EFFECTIVE CLUSTERING OF IMMUNOLOGICAL ENTITIES**

(30) Priority: 16.03.2018 JP 2018049440
(71) Applicant: Kotai Bioteechnologies, Inc., Suita-shi, Oaka 565-0871 (JP)
(72) Inventor: YAMASHITA Kazuo, Suita-shi, Osaka 565-0871 (JP); BILLAUD, Joel, Kasuga-shi, Fukuoka 816-0844 (JP); SAX Nicolas Claude Paul, Suita-shi, Osaka 565-0871 (JP)
(74) Representative: Uexküll & Stolberg
(86) International application number: PCT/JP2019/010861
(87) International publication number: WO 2019/177152

(57) **Abstract**

The present invention provides a method for classifying immunological entities. The inventors assume that there are commonalities among antigen specificities for which, without a function being specified in advance, bound immunological entities (antigens, epitopes, etc.) are normally handled individually as separate "functions (for example, whether antigen A has the specificity)," and the inventors have discovered that it is possible to classify immunological entities by evaluating the similarities thereof. This method has a high degree of precision with respect to immunity-related illnesses, and the present invention is clinically applicable.

## Description

### [Technical Field]

The present invention relates to a method for classifying an immunological entity such as an antibody based on an epitope, production of an epitope cluster, and application thereof.

### [Background Art]

Antibodies are proteins that bind specifically and with high affinity to antigens. A human antibody consists of two macromolecular sequences called a heavy chain and a light chain. Each of the heavy chain and the light chain is further divided into two regions, i.e., variable region and constant region. It is known that a variable region brings out diversity, which is important for the physiological activity of antibodies. The variable region is further divided into framework regions and complementarity-determining regions (CDR). A molecule to which an antibody binds as a target is referred to as an antigen. An antibody generally binds specifically or with high affinity to an antigen by a CDR physically interacting with an antigen. The region in an antigen that physically interacts with an antibody is referred to as an "epitope".

Antibodies are highly diverse. Each individual can create 10¹¹ antibodies with different amino acid sequences. With such diversity, a B cell repertoire can bind to diverse antigens, and different epitopes of the same antigen with different affinities. The amino acid sequence of the CDR region is the source of diversity. The third loop of a heavy chain (CDR-H3) is the most diverse among CDRs. Multiple antibodies with very different amino acid sequences can bind to the same or very similar epitopes in some cases. With such "sequence degeneration", it is very difficult to compare antibodies, especially antibodies produced by different individuals, by an antigen or epitope.

Antibodies are highly commercially valuable molecules. Many of the most commercially successful drugs today are antibody drugs. Antibody drug is also the field that is growing most rapidly in the pharmaceutical industry. Antibodies are broadly utilized not only for pharmaceutical industries, but also in industries other than basic research and drug development for their high affinity and specificity.

T cells also express receptors (TCR), which are structurally very similar to B cells. An important difference is that TCRs are not soluble and are always bound to a T cell (B cells produce an antibody that is a soluble receptor, and a BCR bound to a cell membrane). While not as diverse as BCRs, T cells also have been studied very extensively. In particular, cell disruption by cytotoxic T cells is important in the action against malignant tumor.

In recent years, next-generation sequencing technologies have enabled large scale identification of the amino acid sequences of antibodies or TCRs. Meanwhile, identification of antigens and epitopes that bind to such antibodies or TCRs is a problem yet to be solved, which is expected to have significant commercial demand.

Existing antigen identification methods are method for experimentally identifying interaction by having an antibody or TCR interact with one or more antigen candidates (e.g., surface plasmon resonance). Alternative technologies thereof include protein chips and various library methods. Such technologies are relatively low cost and high throughput, but cannot be applied to proteins or peptides that have been modified after translation, which are important in some diseases such rheumatoid arthritis. Further, identification of structural epitopes is challenging.

These experimental screening technologies require that the antigen is identified. In other words, an antigen must be identified before the discovery of an antibody or TCR.

Non Patent Literature 1 discloses a calculation method for predicting an antibody specific B cell epitope by using residue pairing preferences and cross blocking.

### [Citation List]

### [Non Patent Literature]

[NPL 1] Sela-Culang I. et al., Structure 22, 646-657, 2014

### [Summary of Invention]

### [Solution to Problem]

The inventors found that an immunological entity can be classified by assuming that there is generality in antigen specificity or binding mode, which is normally considered as separate "function (e.g., whether there is specificity to antigen A)" for immunological entity binders (antigens or epitopes) individually, without predefining a function and evaluating the similarity thereof. This enables application to functions that were not previously known (e.g., antigen specificity or binding mode). Therefore, the present invention can be generalized by not specifying a function (e.g., specific antigen specificity or binding mode) in advance generally in a reaction of an immunological entity such as an antibody-antigen reaction. In a preferred embodiment, "function" is a specific antigen specificity or binding mode (ability to control antigens). By not specifying a function in advance, the present invention can include functions against various antigens in a learning set and reflect this in the prediction of similarity for each function.

The present invention provides the following.
(1) A method of analyzing a collection of immunological entities, comprising the steps of:
   (i) providing a feature of at least two immunological entities;
   (ii) subjecting analysis of antigen specificity or binding mode of the immunological entities to machine learning without specifying antigen specificity or binding mode based on the feature; and
   (iii) classifying the antigen specificity or binding mode or determining whether the antigen specificity or binding mode is the same/different.
(2) A method of analyzing a collection of immunological entities, the method comprising the steps of:
   (a) extracting a feature for at least a pair of members of the collection of immunological entities;
   (b) computing a distance between antigen specificities or binding modes or judging whether the antigen specificities or binding modes match for the pair by machine learning using the feature;
   (c) clustering the collection of immunological entities based on the distance; and
   (d) optionally analyzing based on a classification by the clustering.
(3) A method of analyzing a collection of immunological entities, the method comprising the steps of:
   (aa) extracting a feature for each sequence constituting at least a pair of members of the collection of immunological entities;
   (bb) projecting the feature onto a high dimensional vector space, wherein a distance on the space between the members reflects functional similarity of the members;
   (cc) clustering the collection of immunological entities based on the distance; and
   (dd) optionally analyzing based on a classification by the clustering.
(4) The method of any one of the preceding items, wherein the feature comprises at least one selected from the group consisting of sequence information, lengths of CDR1-3 sequences, a degree of match between sequences, a degree of match between sequences of framework regions, a total charge/hydrophilicity/hydrophobicity/number of aromatic amino acids of a molecule, a charge/hydrophilicity/hydrophobicity/number of aromatic amino acids of each CDR or framework region, number of each amino acid, a combination of heavy chain-light chain, number of somatic hypermutations, a position of a mutation, presence/degree of match of an amino acid motif, a degree of rarity with respect to a reference sequence set, and odds of bound HLA according to a reference sequence.
(5) The method of any one of the preceding items, wherein the immunological entities are antibodies, antigen binding fragments of an antibody, B cell receptors, fragments of a B cell receptor, T cell receptors, fragments of a T cell receptor, chimeric antigen receptors (CARs), or cells comprising any one or more of the same.
(6) The method of any one of the preceding items, wherein the calculation by machine learning uses the feature as an input and is performed by random forest or boosting, wherein the clustering is performed based on a simple threshold value based on a binding distance, or by a hierarchical clustering method or a non-hierarchical clustering method.
(7) The method of any one of the preceding items, wherein the analysis comprises one or more of identification of a biomarker and identification of an immunological entity that is a therapeutic target or a cell comprising the immunological entity.
(8) The method of any one of the preceding items,
   wherein the high dimensional vector space calculation (bb) is performed by a supervised, semi-supervised (Siamese network), or unsupervised (Auto-encoder) method, and
   wherein the clustering (cc) is performed based on a simple threshold value based on a distance on a high dimensional space, or by a hierarchical clustering method or a non-hierarchical clustering method.
(9) The method of any one of the preceding items, wherein the analysis (dd) comprises one or more of identification of a biomarker and identification of an immunological entity that is a therapeutic target or a cell comprising the immunological entity.
(10) The method of any one of the preceding items, wherein the machine learning is selected from the group consisting of machine learning algorithms such as a regressive scheme, a neural network method, support vector machine, and random forest.
(11) A program for having a computer execute the method of any one of the preceding items.
(12) A recording medium for storing a program for having a computer execute the method of any one of the preceding items.
(13) A system comprising a program for having a computer execute the method of any one of the preceding items.
(14) The method of any one of the preceding items, comprising the step of associating the antigen specificity or binding mode with biological information.
(15) A method of generating a cluster of antigen specificity or binding mode, comprising the step of classifying immunological entities with the same antigen specificity or binding mode to the same cluster using the method of any one of the preceding items.
(16) The method of identifying a disease, disorder, or biological condition, comprising the step of associating a carrier of the immunological entity with a known disease, disorder, or biological condition based on a cluster generated by the method of any one of the preceding items.
(17) A composition for identifying the biological information, comprising an immunological entity with antigen specificity or binding mode identified based on any one of the preceding items.
(18) A composition for diagnosing a disease, disorder, or biological condition, comprising an immunological entity with antigen specificity or binding mode identified based on the method of any one of the preceding items.
(19) A composition for treating or preventing a disease, disorder, or biological condition, comprising an immunological entity to an epitope identified based on the method of any one of the preceding items.
(20) The composition of any one of the preceding items, wherein the composition comprises a vaccine.
(21) An immunological entity (e.g., antibody), epitope, or immunological entity binder (e.g., antigen) having a structure with antigen specificity or binding mode identified by the method of any one of the preceding items.
(22) The method of any one of the preceding items, comprising the step of associating the immunological entity, epitope, or immunological entity binder with biological information.
(23) The method of any one of the preceding items, further comprising the step of identifying the clustered, classified, or analyzed immunological entity, epitope, or immunological entity binder.
(24) The method of any one of the preceding items, wherein the identifying comprises at least one selected from the group consisting of determining an amino acid sequence, identifying a three-dimensional structure, identifying a structure other than a three-dimensional structure, and identifying a biological function.
(25) The method of any one of the preceding items, wherein the identifying comprises determining a structure of the immunological entity, epitope, or immunological entity binder.
(26) A method of generating a cluster of immunological entities, epitopes, or immunological entity binders, comprising the step of classifying immunological entities, epitopes, or immunological entity binders with the same antigen specificity or binding mode to the same cluster using the classification method of any one of the preceding items.
(27) The method of any one of the preceding items, wherein the immunological entities, epitopes, or immunological entity binders are evaluated by at least one endpoint selected from the group consisting of a property and similarity with a known immunological entity, epitope, or immunological entity binder thereof to perform the cluster classification targeting an immunological entity meeting a predetermined baseline.
(28) A method of identifying a disease, disorder, or biological condition, comprising the step of associating a carrier of an immunological entity, epitope, or immunological entity binder with antigen specificity or binding mode identified based on a cluster generated by the method of any one of the preceding items with a known disease, disorder, or biological condition.
(29) A method of identifying a disease, disorder, or biological condition, comprising the step of evaluating a disease, disorder, or biological condition of a carrier of one or more clusters generated by the method of any one of the preceding items by using the cluster.
(30) The method of any one of the preceding items, wherein the evaluation is performed using at least one indicator selected from the group consisting of analysis based on a ranking of quantity and/or a ratio of abundance of the plurality of clusters, and analysis studying a certain number of B cells and quantifying whether there is a cell/cluster similar to a BCR of interest thereamong.
(31) The method of any one of the preceding items, wherein the evaluation is performed using an indicator other than the cluster.
(32) The method of any one of the preceding items, wherein the indicator other than the cluster comprises at least one selected from a disease associated gene, a polymorphism of a disease associated gene, an expression profile of a disease associated gene, epigenetics analysis, and a combination of TCR and BCR clusters.
(33) The method of any one of the preceding items, wherein identification of the disease, disorder, or biological condition comprises at least one selected from the group consisting of diagnosis, prognosis, pharmacodynamics, and prediction of the disease, disorder, or biological condition, determination of an alternative method, identification of a patient group, safety evaluation, toxicological evaluation, and monitoring thereof.
(34) A method for evaluating a biomarker, comprising the step of evaluating the biomarker used as an indicator of a disease, disorder, or biological condition using one or more of immunological entities, epitopes, or immunological entity binders with antigen specificity or binding mode identified by the method of any one of the preceding items and/or clusters generated by the method of any one of the preceding items.
(35) A method for identifying a biomarker, comprising the step of using one or more of immunological entities, epitopes, or immunological entity binders with antigen specificity or binding mode identified by the method of any one of the preceding items and/or clusters generated by the method of any one of the preceding items to determine the biomarker or association with a disease, disorder, or biological condition.
(36) A composition for identifying the biological information, comprising an immunological entity to an immunological entity, epitope, or immunological entity binder with antigen specificity or binding mode identified based on any one of the preceding items.
(37) A composition for identifying the biological information, comprising an immunological entity, epitope, or immunological entity binder with antigen specificity or binding mode identified based on any one of the preceding items or an immunological entity binder (e.g., antigen) comprising the same.
(38) A composition for diagnosing the disease, disorder, or biological condition of any one of the preceding items, comprising an immunological entity, epitope, or immunological entity binder with antigen specificity or binding mode identified based any one of the preceding items.
(39) A composition for diagnosing the disease, disorder, or biological condition of any one of the preceding items, comprising a substance targeting an immunological entity, epitope, or immunological entity binder with antigen specificity or binding mode identified based on any one of the preceding items.
(40) A composition for diagnosing the disease, disorder, or biological condition of any one of the preceding items, comprising an immunological entity, epitope, or immunological entity binder with antigen specificity or binding mode identified based on any one of the preceding items.
(41) A composition for treating or preventing the disease, disorder, or biological condition of any one of the preceding items, comprising an immunological entity, epitope, or immunological entity binder with antigen specificity or binding mode identified based on any one of the preceding items.
(42) The composition of any one of the preceding items, wherein the immunological entity is selected from the group consisting of an antibody, an antigen binding fragment of an antibody, a T cell receptor, a fragment of a T cell receptor, a B cell receptor, a fragment of a B cell receptor, a chimeric antigen receptor (CAR), and a cell comprising one or more of them (e.g., a T cell comprising a chimeric antigen receptor (CAR)).
(43) A composition for treating or preventing the disease, disorder, or biological condition of any one of the preceding items, comprising a substance targeting an immunological entity, epitope, or immunological entity binder with antigen specificity or binding mode identified based on any one of the preceding items.
(44) A composition for treating or preventing the disease, disorder, or biological condition of any one of the preceding items, comprising an immunological entity, epitope, or immunological entity binder with antigen specificity or binding mode identified based on any one of the preceding items.
(45) The composition of any one of the preceding items, wherein the composition comprises a vaccine.
(46) A composition for evaluating a vaccine for treating or preventing a disease, disorder, or biological condition, comprising an immunological entity, epitope, or immunological entity binder with antigen specificity or binding mode identified based on any one of the preceding items.
(47) A computer program for having a computer execute a method of analyzing a collection of immunological entities, the method comprising the steps of:
   (i) providing a feature of at least two immunological entities;
   (ii) subjecting analysis of antigen specificity or binding mode of the immunological entities to machine learning without specifying antigen specificity or binding mode based on the feature; and
   (iii) classifying the antigen specificity or binding mode or determining whether the antigen specificity or binding mode is the same/different.
(48) A computer program for having a computer execute a method of analyzing a collection of immunological entities, the method comprising the steps of:
   (a) extracting a feature for at least a pair of members of the collection of immunological entities;
   (b) computing a distance between antigen specificities or binding modes or judging whether the antigen specificities or binding modes match for the pair by machine learning using the feature;
   (c) clustering the collection of immunological entities based on the distance; and
   (d) optionally analyzing based on a classification by the clustering.
(49) A computer program for having a computer execute a method of analyzing a collection of immunological entities, the method comprising the steps of:
   (aa) extracting a feature for each sequence constituting at least a pair of members of the collection of immunological entities;
   (bb) projecting the feature onto a high dimensional vector space, wherein a distance on the space between the members reflects functional similarity of the members;
   (cc) clustering the collection of immunological entities based on the distance; and
   (dd) optionally analyzing based on a classification by the clustering.
(50) The program of any one of the preceding items, further comprising one or more features of the preceding items.
(51) A recording medium storing a computer program for having a computer execute a method of analyzing a collection of immunological entities, the method comprising the steps of:
   (i) providing a feature of at least two immunological entities;
   (ii) subjecting analysis of antigen specificity or binding mode of the immunological entities to machine learning without specifying antigen specificity or binding mode based on the feature; and
   (iii) classifying the antigen specificity or binding mode or determining whether the antigen specificity or binding mode is the same/different.
(52) A recording medium storing a computer program for having a computer execute a method of analyzing a collection of immunological entities, the method comprising the steps of:
   (a) extracting a feature for at least a pair of members of the collection of immunological entities;
   (b) computing a distance between antigen specificities or binding modes or judging whether the antigen specificities or binding modes match for the pair by machine learning using the feature;
   (c) clustering the collection of immunological entities based on the distance; and
   (d) optionally analyzing based on a classification by the clustering.
(53) A recording medium storing a computer program for having a computer execute a method of analyzing a collection of immunological entities, the method comprising the steps of:
   (aa) extracting a feature for each sequence constituting at least a pair of members of the collection of immunological entities;
   (bb) projecting the feature onto a high dimensional vector space, wherein a distance on the space between the members reflects functional similarity of the members;
   (cc) clustering the collection of immunological entities based on the distance; and
   (dd) optionally analyzing based on a classification by the clustering.
(54) The recording medium of any one of the preceding items, further comprising one or more features of the preceding items.
(55) A system for analyzing a collection of immunological entities, the system comprising:
   (I) a feature providing unit for -providing a feature of at least two immunological entities;
   (II) a machine learning unit for subjecting analysis of antigen specificity or binding mode of the immunological entities to machine learning without specifying antigen specificity or binding mode based on the feature; and
   (III) a classification unit for classifying the antigen specificity or binding mode or determining whether the antigen specificity or binding mode is the same/different.
(56) A system for analyzing a collection of immunological entities, the system comprising:
   (A) a feature providing unit for extracting a feature for at least a pair of members of the collection of immunological entities;
   (B) a judgment unit for computing a distance between antigen specificities or binding modes or judging whether the antigen specificities or binding modes match for the pair by machine learning using the feature;
   (C) a clustering unit for clustering the collection of immunological entities based on the distance; and
   (D) an analysis unit for optionally analyzing based on a classification by the clustering.
(57) A system for analyzing a collection of immunological entities, the system comprising:
   (A) a feature providing unit for extracting a feature for each sequence constituting at least a pair of members of the collection of immunological entities;
   (B') a projection unit for projecting the feature onto a high dimensional vector space, wherein a distance on the space between the members reflects functional similarity of the members;
   (C) a clustering unit for clustering the collection of immunological entities based on the distance; and
   (D) an analysis unit for optionally analyzing based on a classification by the clustering.
(58) The system of any one of the preceding items, further comprising one or more features of the preceding items.
(59) The method, program, recording medium, or system of any of items 1 to 58, characterized by:
   the step (i) or (I) excluding calculating a feature from a three dimensional structural model of the at least two immunological entities;
   the step (ii) or (A) excluding calculating a feature from a three dimensional structural model of the at least a pair; or
   the step (iii) or (A) excluding calculating a feature from a three dimensional structural model of an immunological entity of a sequence constituting the at least a pair.
      (A1) A method of analyzing a collection of immunological entities, comprising the steps of:
         (i) providing a feature of at least two immunological entities, the step excluding calculating a feature from a three dimensional structural model of the at least two immunological entities;
         (ii) subjecting analysis of antigen specificity or binding mode of the immunological entities to machine learning without specifying antigen specificity or binding mode based on the feature; and
         (iii) classifying the antigen specificity or binding mode or determining whether the antigen specificity or binding mode is the same/different.
      (A2) A method of analyzing a collection of immunological entities, the method comprising the steps of:
         (a) extracting a feature for at least a pair of members of the collection of immunological entities, wherein the step excludes calculating a feature from a three dimensional structural model of the at least a pair;
         (b) computing a distance between antigen specificities or binding modes or judging whether the antigen specificities or binding modes match for the pair by machine learning using the feature;
         (c) clustering the collection of immunological entities based on the distance; and
         (d) optionally analyzing based on a classification by the clustering.
      (A3) A method of analyzing a collection of immunological entities, the method comprising the steps of:
         (aa) extracting a feature for each sequence constituting at least a pair of members of the collection of immunological entities, wherein the step excludes calculating a feature from a three dimensional structural model of an immunological entity of a sequence constituting the at least a pair;
         (bb) projecting the feature onto a high dimensional vector space, wherein a distance on the space between the members reflects functional similarity of the members;
         (cc) clustering the collection of immunological entities based on the distance; and
         (dd) optionally analyzing based on a classification by the clustering.
      (A4) The method of any one of items A1 to A3, further comprising one or more features of items 1 to 58.
      (A5) A recording medium storing a computer program for having a computer execute a method of analyzing a collection of immunological entities, the method comprising the steps of:
         (i) providing a feature of at least two immunological entities, the step excluding calculating a feature from a three dimensional structural model of the at least two immunological entities;
         (ii) subjecting analysis of antigen specificity or binding mode of the immunological entities to machine learning without specifying antigen specificity or binding mode based on the feature; and
         (iii) classifying the antigen specificity or binding mode or determining whether the antigen specificity or binding mode is the same/different.
      (A6) A recording medium storing a computer program for having a computer execute a method of analyzing a collection of immunological entities, the method comprising the steps of:
         (a) extracting a feature for at least a pair of members of the collection of immunological entities, wherein the step excludes calculating a feature from a three dimensional structural model of the at least a pair;
         (b) computing a distance between antigen specificities or binding modes or judging whether the antigen specificities or binding modes match for the pair by machine learning using the feature;
         (c) clustering the collection of immunological entities based on the distance; and
         (d) optionally analyzing based on a classification by the clustering.
      (A7) A recording medium storing a computer program for having a computer execute a method of analyzing a collection of immunological entities, the method comprising the steps of:
         (aa) extracting a feature for each sequence constituting at least a pair of members of the collection of immunological entities, wherein the step excludes calculating a feature from a three dimensional structural model of an immunological entity of a sequence constituting the at least a pair;
         (bb) projecting the feature onto a high dimensional vector space, wherein a distance on the space between the members reflects functional similarity of the members;
         (cc) clustering the collection of immunological entities based on the distance; and
         (dd) optionally analyzing based on a classification by the clustering.
      (A8) The recording medium of any one of items A5 to A7, further comprising one or more features of items 1 to 58.
      (A9) A system for analyzing a collection of immunological entities, the system comprising:
         (I) a feature providing unit for providing a feature of at least two immunological entities, the feature providing unit excluding calculating a feature from a three dimensional structural model of the at least two immunological entities;
         (II) a machine learning unit for subjecting analysis of antigen specificity or binding mode of the immunological entities to machine learning without specifying antigen specificity or binding mode based on the feature; and
         (III) a classification unit for classifying the antigen specificity or binding mode or determining whether the antigen specificity or binding mode is the same/different.
      (A10) A system for analyzing a collection of immunological entities, the system comprising:
         (A) a feature providing unit for extracting a feature for at least a pair of members of the collection of immunological entities, the feature providing unit excluding calculating a feature from a three dimensional structural model of the at least a pair;
         (B) a judgment unit for computing a distance between antigen specificities or binding modes or judging whether the antigen specificities or binding modes match for the pair by machine learning using the feature;
         (C) a clustering unit for clustering the collection of immunological entities based on the distance; and
         (D) an analysis unit for optionally analyzing based on a classification by the clustering.
      (A11) A system for analyzing a collection of immunological entities, the system comprising:
         (A) a feature providing unit for extracting a feature for each sequence constituting at least a pair of members of the collection of immunological entities, the feature providing unit excluding calculating a feature from a three dimensional structural model of an immunological entity of a sequence constituting the at least a pair;
         (B') a projection unit for projecting the feature onto a high dimensional vector space, wherein a distance on the space between the members reflects functional similarity of the members;
         (C) a clustering unit for clustering the collection of immunological entities based on the distance; and
         (D) an analysis unit for optionally analyzing based on a classification by the clustering.
      (A12) The system of any one of items A9 to A11, further comprising one or more features of items 1 to 58.
      (Item B1) A method of analyzing a collection of immunological entities, comprising the steps of:
         (i) providing a feature of at least two immunological entities;
         (ii) subjecting analysis of antigen specificity or binding mode of the immunological entities to machine learning without specifying antigen specificity or binding mode based on the feature; and
         (iii) classifying the antigen specificity or binding mode or determining whether the antigen specificity or binding mode is the same/different.
      (Item B2) A method of analyzing a collection of immunological entities, the method comprising the steps of:
         (a) extracting a feature for at least a pair of members of the collection of immunological entities;
         (b) computing a distance between antigen specificities or binding modes or judging whether the antigen specificities or binding modes match for the pair by machine learning using the feature;
         (c) clustering the collection of immunological entities based on the distance; and
         (d) optionally analyzing based on a classification by the clustering.
      (Item B3) A method of analyzing a collection of immunological entities, the method comprising the steps of:
         (aa) extracting a feature for each sequence constituting at least a pair of members of the collection of immunological entities;
         (bb) projecting the feature onto a high dimensional vector space, wherein a distance on the space between the members reflects functional similarity of the members;
         (cc) clustering the collection of immunological entities based on the distance; and
         (dd) optionally analyzing based on a classification by the clustering.
      (Item B4) The method of any one of the preceding items, wherein the feature comprises at least one selected from the group consisting of sequence information, lengths of CDR1-3 sequences, a degree of match between sequences, a degree of match between sequences of framework regions, a total charge/hydrophilicity/hydrophobicity/number of aromatic amino acids of a molecule, a charge/hydrophilicity/hydrophobicity/number of aromatic amino acids of each CDR or framework region, number of each amino acid, a combination of heavy chain-light chain, number of somatic hypermutations, a position of a mutation, presence/degree of match of an amino acid motif, a degree of rarity with respect to a reference sequence set, and odds of bound HLA according to a reference sequence.
      (Item B5) The method of any one of the preceding items, wherein the immunological entities are antibodies, antigen binding fragments of an antibody, B cell receptors, fragments of a B cell receptor, T cell receptors, fragments of a T cell receptor, chimeric antigen receptors (CARs), or cells comprising any one or more of the same.
      (Item B6) The method of any one of the preceding items,
         wherein the calculation by machine learning uses the feature as an input and is performed by random forest or boosting, and
         wherein the clustering is performed based on a simple threshold value based on a binding distance, or by a hierarchical clustering method or a non-hierarchical clustering method.
      (Item B7) The method of any one of the preceding items, wherein the analysis comprises one or more of identification of a biomarker and identification of an immunological entity that is a therapeutic target or a cell comprising the immunological entity.
      (Item B8) The method of any one of the preceding items, wherein the machine learning is selected from the group consisting of machine learning algorithms such as a regressive scheme, a neural network method, support vector machine, and random forest.
      (Item B9) The method of any one of the preceding items, wherein the feature comprises at least one selected from the group consisting of sequence information, lengths of CDR1-3 sequences, a degree of match between sequences, a degree of match between sequences of framework regions, a total charge/hydrophilicity/hydrophobicity/number of aromatic amino acids of a molecule, a charge/hydrophilicity/hydrophobicity/number of aromatic amino acids of each CDR or framework region, number of each amino acid, a combination of heavy chain-light chain, number of somatic hypermutations, a position of a mutation, presence/degree of match of an amino acid motif, a degree of rarity with respect to a reference sequence set, and odds of bound HLA according to a reference sequence.
      (Item B10) The method of any one of the preceding items, wherein the immunological entities are antibodies, antigen binding fragments of an antibody, B cell receptors, fragments of a B cell receptor, T cell receptors, fragments of a T cell receptor, chimeric antigen receptors (CARs), or cells comprising any one or more of the same.
      (Item B11) The method of any one of the preceding items,
         wherein the step of projecting calculation onto a high dimensional vector space (bb) is performed by a supervised, semi-supervised (Siamese network), or unsupervised (Auto-encoder) method, and
         wherein the step of clustering (cc) is performed based on a simple threshold value based on a distance on a high dimensional space, or by a hierarchical clustering method or a non-hierarchical clustering method.
      (Item B12) The method of any one of the preceding items, wherein the analysis comprises one or more of identification of a biomarker and identification of an immunological entity that is a therapeutic target or a cell comprising the immunological entity.
      (Item B13) A program for having a computer execute the method of any one of the preceding items.
      (Item B14) A recording medium storing a program for having a computer execute the method of any one of the preceding items.
      (Item B15) A system comprising a program for having a computer execute the method of any one of the preceding items.
      (Item B16) The method of any one of the preceding items, comprising the step of associating the antigen specificity or binding mode with biological information.
      (Item B17) A method of generating a cluster of antigen specificity or binding mode, comprising the step of classifying immunological entities with the same antigen specificity or binding mode to the same cluster using the method of any one of the preceding items.
      (Item B18) A method of identifying a disease, disorder, or biological condition, comprising the step of associating a carrier of the immunological entity with a known disease, disorder, or biological condition based on a cluster generated by the method of any one of the preceding items.
      (Item B19) A composition for identifying the biological information, comprising an immunological entity with antigen specificity or binding mode identified based on the method of any one of the preceding items.
      (Item B20) A composition for diagnosing a disease, disorder, or biological condition, comprising an immunological entity with antigen specificity or binding mode identified based on the method of any one of the preceding items.
      (Item B21) A composition for treating or preventing a disease, disorder, or biological condition, comprising an immunological entity with antigen specificity or binding mode identified based on the method of any one of the preceding items.
      (Item B22) A composition for diagnosing a disease, disorder, or biological condition, comprising an immunological entity binder corresponding to an epitope identified based on the method of any one of the preceding items.
      (Item B23) A composition for treating or preventing a disease, disorder, or biological condition, comprising an immunological entity binder corresponding to an epitope identified based on the method of any one of the preceding items.
      (Item B24) The composition of any one of the preceding items, wherein the composition comprises a vaccine.
      (Item B25) A method for diagnosing a disease, disorder, or biological condition, comprising the step of diagnosing based on an immunological entity with antigen specificity or binding mode identified based on the method of any one of the preceding items.
      (Item B26) A method for judging an adverse event for a disease, disorder, or biological condition, comprising the step of determining an adverse event based on an immunological entity with antigen specificity or binding mode identified based on the method of any one of the preceding items.
      (Item B27) A method for diagnosing a disease, disorder, or biological condition, comprising the step of diagnosing based on an immunological entity with,antigen specificity or binding mode identified based on the method of any one of the preceding items, wherein the at least two immunological entities or the collection of immunological entities comprise at least one immunological entity derived from a healthy individual.
      (Item B28) A method for treating or preventing a disease, disorder, or biological condition, comprising the step of administering an effective amount of an immunological entity with antigen specificity or binding mode identified based on the method of any one of the preceding items.
      (Item B29) A method for treating or preventing a disease, disorder, or biological condition, comprising the step of administering to a subject an effective amount of an immunological entity with antigen specificity or binding mode identified based on the method of any one of the preceding items, wherein the subject excludes a subject determined as a subject who can have an adverse event based on the method of any one of the preceding claims.
      (Item B30) A method for treating or preventing a disease, disorder, or biological condition, comprising the step of administering an effective amount of an immunological entity with antigen specificity or binding mode identified based on the method of any one of the preceding items, wherein the at least two immunological entities or the collection of immunological entities comprise at least one immunological entity derived from a healthy individual.
      (Item B31) A method for diagnosing a disease, disorder, or biological condition, comprising the step of diagnosing based on an immunological entity binder corresponding to an epitope identified based on the method of any one of the preceding items.
      (Item B32) A method for judging an adverse event for a disease, disorder, or biological condition, comprising the step of determining an adverse event based on an immunological entity binder corresponding to an epitope identified based on the method of any one of the preceding items.
      (Item B33) A method for diagnosing a disease, disorder, or biological condition, comprising the step of diagnosing based on an immunological entity binder corresponding to an epitope identified based on the method of any one of the preceding items, wherein the at least two immunological entities or the collection of immunological entities comprise at least one immunological entity derived from a healthy individual.
      (Item B34) A method for treating or preventing a disease, disorder, or biological condition, comprising the step of administering an effective amount of an immunological entity binder corresponding to an epitope identified based on the method of any one of the preceding items.
      (Item B35) A method for treating or preventing a disease, disorder, or biological condition, comprising the step of administering an effective amount of an immunological entity binder corresponding to an epitope identified based on the method of any one of the preceding items, wherein the subject excludes a subject determined as a subject who can have an adverse event based on the method of any one of the preceding claims.
      (Item B36) A method for treating or preventing a disease, disorder, or biological condition, comprising the step of administering an effective amount of an immunological entity binder corresponding to an epitope identified based on the method of any one of the preceding items, wherein the at least two immunological entities or the collection of immunological entities comprise at least one immunological entity derived from a healthy individual.
      (Item B37) The method of any one of the preceding items, wherein the immunological entity binder comprises a vaccine.
      (Item B38) A method for diagnosing a disease, disorder, or biological condition, comprising the steps of:
         (i) providing a feature of at least two immunological entities;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) judging a disease, disorder, or biological condition based on the immunological entities classified or determined in (iii).
      (Item B38A) The method of item B38, further comprising one or more features of the preceding items.
      (Item B39) A method for diagnosing a disease, disorder, or biological condition, the method comprising the steps of:
         (a) extracting a feature for at least a pair of members of the collection of immunological entities;
         (b) computing a distance between antigen specificities or binding modes or judging whether the antigen specificities or binding modes match for the pair by machine learning using the feature;
         (c) clustering the collection of immunological entities based on the distance;
         (d) analyzing based on a classification by the clustering; and
         (e) judging a disease, disorder, or biological condition based on the immunological entities analyzed in (d).
      (Item B39A) The method of item B39, further comprising one or more features of the preceding items.
      (Item B40) A method for diagnosing a disease, disorder, or biological condition, method comprising the steps of:
         (aa) extracting a feature for each sequence constituting at least a pair of members of the collection of immunological entities;
         (bb) projecting the feature onto a high dimensional vector space, wherein a distance on the space between the members reflects functional similarity of the members;
         (cc) clustering the collection of immunological entities based on the distance;
         (dd) analyzing based on a classification by the clustering; and
         (ee) judging a disease, disorder, or biological condition based on the immunological entities analyzed in (dd).
      (Item B40A) The method of item B40, further comprising one or more features of the preceding items.
      (Item B41) A method for treating or preventing a disease, disorder, or biological condition, comprising the steps of:
         (i) providing a feature of at least two immunological entities;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) administering the immunological entities classified or determined in (iii) or an immunological entity binder corresponding to the immunological entities.
      (Item B41A) The method of item B41, further comprising one or more features of the preceding items.
      (Item B42) A method for treating or preventing a disease, disorder, or biological condition, the method comprising the steps of:
         (a) extracting a feature for at least a pair of members of the collection of immunological entities;
         (b) computing a distance between antigen specificities or binding modes or judging whether the antigen specificities or binding modes match for the pair by machine learning using the feature;
         (c) clustering the collection of immunological entities based on the distance;
         (d) optionally analyzing based on a classification by the clustering; and
         (e) administering the immunological entities analyzed in (d) or an immunological entity binder corresponding to the immunological entities.
      (Item B42A) The method of item B42, further comprising one or more features of the preceding items.
      (Item B43) A method for treating or preventing a disease, disorder, or biological condition, the method comprising the steps of:
         (aa) extracting a feature for each sequence constituting at least a pair of members of the collection of immunological entities;
         (bb) projecting the feature onto a high dimensional vector space, wherein a distance on the space between the members reflects functional similarity of the members;
         (cc) clustering the collection of immunological entities based on the distance;
         (dd) optionally analyzing based on a classification by the clustering; and
         (ee) administering the immunological entities analyzed in
         (dd) or an immunological entity binder corresponding to the immunological entities.
      (Item B43A) The method of item B43, further comprising one or more features of the preceding items.
      (Item B44) A method for diagnosing a disease, disorder, or biological condition, comprising the steps of:
         (i) providing a feature of at least two immunological entities, wherein the at least two immunological entities comprise at least one immunological entity derived from a healthy individual;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) judging a disease, disorder, or biological condition based on the immunological entities classified or determined in (iii).
      (Item B44A) The method of item B44, further comprising one or more features of the preceding items.
      (Item B45) The method of item B44 or B44A, wherein the disease, disorder, or biological condition comprises an adverse event.
      (Item B46) A method for diagnosing a disease, disorder, or biological condition, the method comprising the steps of:
         (a) extracting a feature for at least a pair of members of the collection of immunological entities, wherein the collection of immunological entities comprises at least one immunological entity derived from a healthy individual;
         (b) computing a distance between antigen specificities or binding modes or judging whether the antigen specificities or binding modes match for the pair by machine learning using the feature;
         (c) clustering the collection of immunological entities based on the distance;
         (d) analyzing based on a classification by the clustering; and
         (e) judging a disease, disorder, or biological condition based on the immunological entities analyzed in (d).
      (Item B46A) The method of item B46, further comprising one or more features of the preceding items.
      (Item B47) The method of item B46 or B46A, wherein the disease, disorder, or biological condition comprises an adverse event.
      (Item B48) A method for diagnosing a disease, disorder, or biological condition, the method comprising the steps of:
         (aa) extracting a feature for each sequence constituting at least a pair of members of the collection of immunological entities, wherein the collection of immunological entities comprises at least one immunological entity derived from a healthy individual;
         (bb) projecting the feature onto a high dimensional vector space, wherein a distance on the space between the members reflects functional similarity of the members;
         (cc) clustering the collection of immunological entities based on the distance;
         (dd) analyzing based on a classification by the clustering; and
         (ee) judging a disease, disorder, or biological condition based on the immunological entities analyzed in (dd).
      (Item B48A) The method of item B48, further comprising one or more features of the preceding items.
      (Item B49) The method of item B48 or B48A, wherein the disease, disorder, or biological condition comprises an adverse event.
      (Item B50) A method for treating or preventing a disease, disorder, or biological condition, comprising the steps of:
         (i) providing a feature of at least two immunological entities, wherein the at least two immunological entities comprise at least one immunological entity derived from a healthy individual;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) administering the immunological entities classified or determined in (iii) or an immunological entity binder corresponding to the immunological entities.
      (Item B50A) The method of item B50, further comprising one or more features of the preceding items.
      (Item B51) A method of item B50 or B50A, wherein the disease, disorder, or biological condition comprises an adverse event, or the treatment or prevention comprises treating or preventing while avoiding an adverse event.
      (Item B52) A method for treating or preventing a disease, disorder, or biological condition, the method comprising the steps of:
         (a) extracting a feature for at least a pair of members of the collection of immunological entities, wherein the collection of immunological entities comprises at least one immunological entity derived from a healthy individual;
         (b) computing a distance between antigen specificities or binding modes or judging whether the antigen specificities or binding modes match for the pair by machine learning using the feature;
         (c) clustering the collection of immunological entities based on the distance;
         (d) optionally analyzing based on a classification by the clustering; and
         (e) administering the immunological entities analyzed in (d) or an immunological entity binder corresponding to the immunological entities.
      (Item B52A) The method of item B52, further comprising one or more features of the preceding items.
      (Item B53) The method of item B52 or B52A, wherein the disease, disorder, or biological condition comprises an adverse event, or the treatment or prevention comprises treating or preventing while avoiding an adverse event.
      (Item B54) A method for treating or preventing a disease, disorder, or biological condition, the method comprising the steps of:
         (aa) extracting a feature for each sequence constituting at least a pair of members of the collection of immunological entities, wherein the collection of immunological entities comprises at least one immunological entity derived from a healthy individual;
         (bb) projecting the feature onto a high dimensional vector space, wherein a distance on the space between the members reflects functional similarity of the members;
         (cc) clustering the collection of immunological entities based on the distance;
         (dd) optionally analyzing based on a classification by the clustering; and
         (ee) administering the immunological entities analyzed in
         (dd) or an immunological entity binder corresponding to the immunological entities.
      (Item B54A) The method of item B54, further comprising one or more features of the preceding items.
      (Item B55) The method of item B54 or B54A, wherein the disease, disorder, or biological condition comprises an adverse event, or the treatment or prevention comprises treating or preventing while avoiding an adverse event.
      (Item C19) A method for identifying the biological information, comprising the step of using an immunological entity with antigen specificity or binding mode identified based on the method of any one of the preceding items.
      (Item C20) A method for diagnosing a disease, disorder, or biological condition, comprising the step of diagnosing using an immunological entity with antigen specificity or binding mode identified based on the method of any one of the preceding items.
      (Item C21) A method for treating or preventing a disease, disorder, or biological condition, comprising the step of administering an immunological entity with antigen specificity or binding mode identified based on the method of any one of the preceding items to a subject in need thereof.
      (Item C22) A method for diagnosing a disease, disorder, or biological condition, comprising the step of diagnosing using an immunological entity binder corresponding to an epitope identified based on the method of any one of the preceding items.
      (Item C23) A method for treating or preventing a disease, disorder, or biological condition, comprising the step of administering an immunological entity binder corresponding to an epitope identified based on the method of any one of the preceding items to a subject in need thereof.
      (Item C24) The method of any one of the preceding items, wherein the composition comprises a vaccine.
      (Item D38) A computer program for having a computer execute a method for diagnosing a disease, disorder, or biological condition, the method comprising the steps of:
         (i) providing a feature of at least two immunological entities;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) judging a disease, disorder, or biological condition based on the immunological entities classified or determined in (iii).
      (Item D38A) The program of item D38, further comprising one or more features of the preceding items.
      (Item D39) A computer program for having a computer execute a method for diagnosing a disease, disorder, or biological condition, the method comprising the steps of:
         (a) extracting a feature for at least a pair of members of the collection of immunological entities;
         (b) computing a distance between antigen specificities or binding modes or judging whether the antigen specificities or binding modes match for the pair by machine learning using the feature;
         (c) clustering the collection of immunological entities based on the distance;
         (d) analyzing based on a classification by the clustering; and
         (e) judging a disease, disorder, or biological condition based on the immunological entities analyzed in (d).
      (Item D39A) The program of item D39, further comprising one or more features of the preceding items.
      (Item D40) A computer program for having a computer execute a method for diagnosing a disease, disorder, or biological condition, the method comprising the steps of:
         (aa) extracting a feature for each sequence constituting at least a pair of members of the collection of immunological entities;
         (bb) projecting the feature onto a high dimensional vector space, wherein a distance on the space between the members reflects functional similarity of the members;
         (cc) clustering the collection of immunological entities based on the distance;
         (dd) analyzing based on a classification by the clustering; and
         (ee) judging a disease, disorder, or biological condition based on the immunological entities analyzed in (dd).
      (Item D40A) The program of item D40, further comprising one or more features of the preceding items.
      (Item D41) A computer program for having a computer execute a method for treating or preventing a disease, disorder, or biological condition, the method comprising the steps of:
         (i) providing a feature of at least two immunological entities;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) administering the immunological entities classified or determined in (iii) or an immunological entity binder corresponding to the immunological entities.
      (Item D41A) The program of item D41, further comprising one or more features of the preceding items.
      (Item D42) A computer program for having a computer execute a method for treating or preventing a disease, disorder, or biological condition, the method comprising the steps of:
         (a) extracting a feature for at least a pair of members of the collection of immunological entities;
         (b) computing a distance between antigen specificities or binding modes or judging whether the antigen specificities or binding modes match for the pair by machine learning using the feature;
         (c) clustering the collection of immunological entities based on the distance;
         (d) optionally analyzing based on a classification by the clustering; and
         (e) administering immunological entities analyzed in (d) or an immunological entity binder corresponding to the immunological entities.
      (Item D42A) The program of item D42, further comprising one or more features of the preceding items.
      (Item D43) A computer program for having a computer execute a method for treating or preventing a disease, disorder, or biological condition, the method comprising the steps of:
         (aa) extracting a feature for each sequence constituting at least a pair of members of the collection of immunological entities;
         (bb) projecting the feature onto a high dimensional vector space, wherein a distance on the space between the members reflects functional similarity of the members;
         (cc) clustering the collection of immunological entities based on the distance;
         (dd) optionally analyzing based on a classification by the clustering; and
         (ee) administering immunological entities analyzed in
         (dd) or an immunological entity binder corresponding to the immunological entities.
      (Item D43A) The program of item D43, further comprising one or more features of the preceding items.
      (Item D44) A computer program for having a computer execute a method for diagnosing a disease, disorder, or biological condition, the method comprising the steps of:
         (i) providing a feature of at least two immunological entities, wherein the at least two immunological entities comprise at least one immunological entity derived from a healthy individual;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) judging a disease, disorder, or biological condition based on the immunological entities classified or determined in (iii).
      (Item D44A) The program of item D44, further comprising one or more features of the preceding items.
      (Item D45) The program of item D44 or D44A, wherein the disease, disorder, or biological condition comprises an adverse event.
      (Item D46) A computer program for having a computer execute a method for diagnosing a disease, disorder, or biological condition, the method comprising the steps of:
         (a) extracting a feature for at least a pair of members of the collection of immunological entities, wherein the collection of immunological entities comprises at least one immunological entity derived from a healthy individual;
         (b) computing a distance between antigen specificities or binding modes or judging whether the antigen specificities or binding modes match for the pair by machine learning using the feature;
         (c) clustering the collection of immunological entities based on the distance;
         (d) analyzing based on a classification by the clustering; and
         (e) judging a disease, disorder, or biological condition based on the immunological entities analyzed in (d).
      (Item D46A) The program of item D46, further comprising one or more features of the preceding items.
      (Item D47) The program of item D46 or D46A, wherein the disease, disorder, or biological condition comprises an adverse event.
      (Item D48) A computer program for having a computer execute a method for diagnosing a disease, disorder, or biological condition, the method comprising the steps of:
         (aa) extracting a feature for each sequence constituting at least a pair of members of the collection of immunological entities, wherein the collection of immunological entities comprise at least one immunological entity derived from a healthy individual;
         (bb) projecting the feature onto a high dimensional vector space, wherein a distance on the space between the members reflects functional similarity of the members;
         (cc) clustering the collection of immunological entities based on the distance;
         (dd) analyzing based on a classification by the clustering; and
         (ee) judging a disease, disorder, or biological condition based on the immunological entities analyzed in (dd).
      (Item D48A) The program of item D48, further comprising one or more features of the preceding items.
      (Item D49) The program of item D48 or D48A, wherein the disease, disorder, or biological condition comprises an adverse event.
      (Item D50) A computer program for having a computer execute a method for treating or preventing a disease, disorder, or biological condition, the method comprising the steps of:
         (i) providing a feature of at least two immunological entities, wherein the at least two immunological entities comprise at least one immunological entity derived from a healthy individual;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) administering the immunological entities classified or determined in (iii) or an immunological entity binder corresponding to the immunological entities.
      (Item D50A) The program of item D50, further comprising one or more features of the preceding items.
      (Item D51) The program of item D50 or D50A, wherein the disease, disorder, or biological condition comprises an adverse event, or the treatment or prevention comprises treating or preventing while avoiding an adverse event.
      (Item D52) A computer program for having a computer execute a method for treating or preventing a disease, disorder, or biological condition, the method comprising the steps of:
         (a) extracting a feature for at least a pair of members of the collection of immunological entities, wherein the collection of immunological entities comprises at least one immunological entity derived from a healthy individual;
         (b) computing a distance between antigen specificities or binding modes or judging whether the antigen specificities or binding modes match for the pair by machine learning using the feature;
         (c) clustering the collection of immunological entities based on the distance;
         (d) optionally analyzing based on a classification by the clustering; and
         (e) administering the immunological entities analyzed in (d) or an immunological entity binder corresponding to the immunological entities.
      (Item D52A) The program of item D52, further comprising one or more features of the preceding items.
      (Item D53) The program of item D52 or D52A, wherein the disease, disorder, or biological condition comprises an adverse event, or the treatment or prevention comprises treating or preventing while avoiding an adverse event.
      (Item D54) A computer program for having a computer execute a method for treating or preventing a disease, disorder, or biological condition, the method comprising the steps of:
         (aa) extracting a feature for each sequence constituting at least a pair of members of the collection of immunological entities, wherein the collection of immunological entities comprises at least one immunological entity derived from a healthy individual;
         (bb) projecting the feature onto a high dimensional vector space, wherein a distance on the space between the members reflects functional similarity of the members;
         (cc) clustering the collection of immunological entities based on the distance;
         (dd) optionally analyzing based on a classification by the clustering; and
         (ee) administering the immunological entities analyzed in (dd) or an immunological entity binder corresponding to the immunological entities.
      (Item D54A) The program of item D54, further comprising one or more features of the preceding items.
      (Item D55) The program of item D54 or D54A, wherein the disease, disorder, or biological condition comprises an adverse event, or the treatment or prevention comprises treating or preventing while avoiding an adverse event.
      (Item E38) A recording medium storing a computer program for having a computer execute a method for diagnosing a disease, disorder, or biological condition, the method comprising the steps of:
         (i) providing a feature of at least two immunological entities;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) judging a disease, disorder, or biological condition based on the immunological entities classified or determined in (iii).
      (Item E38A) The recording medium of item E38, further comprising one or more features of the preceding items.
      (Item E39) A recording medium storing a computer program for having a computer execute a method for diagnosing a disease, disorder, or biological condition, the method comprising the steps of:
         (a) extracting a feature for at least a pair of members of the collection of immunological entities;
         (b) computing a distance between antigen specificities or binding modes or judging whether the antigen specificities or binding modes match for the pair by machine learning using the feature;
         (c) clustering the collection of immunological entities based on the distance;
         (d) analyzing based on a classification by the clustering; and
         (e) judging a disease, disorder, or biological condition based on the immunological entities analyzed in (d).
      (Item E39A) The recording medium of item E39, further comprising one or more features of the preceding items.
      (Item E40) A recording medium storing a computer program for having a computer execute a method for diagnosing a disease, disorder, or biological condition, the method comprising the steps of:
         (aa) extracting a feature for each sequence constituting at least a pair of members of the collection of immunological entities;
         (bb) projecting the feature onto a high dimensional vector space, wherein a distance on the space between the members reflects functional similarity of the members;
         (cc) clustering the collection of immunological entities based on the distance;
         (dd) analyzing based on a classification by the clustering; and
         (ee) judging a disease, disorder, or biological condition based on the immunological entities analyzed in (dd).
      (Item E40A) The recording medium of item E40, further comprising one or more features of the preceding items.
      (Item E41) A recording medium storing a computer program for having a computer execute a method for treating or preventing a disease, disorder, or biological condition, the method comprising the steps of:
         (i) providing a feature of at least two immunological entities;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) administering the immunological entities classified or determined in (iii) or an immunological entity binder corresponding to the immunological entities.
      (Item E41A) The recording medium of item E41, further comprising one or more features of the preceding items.
      (Item E42) A recording medium storing a computer program for having a computer execute a method for treating or preventing a disease, disorder, or biological condition, the method comprising the steps of:
         (a) extracting a feature for at least a pair of members of the collection of immunological entities;
         (b) computing a distance between antigen specificities or binding modes or judging whether the antigen specificities or binding modes match for the pair by machine learning using the feature;
         (c) clustering the collection of immunological entities based on the distance;
         (d) optionally analyzing based on a classification by the clustering; and
         (e) administering the immunological entities analyzed in (d) or an immunological entity binder corresponding to the immunological entities.
      (Item E42A) The recording medium of item E42, further comprising one or more features of the preceding items.
      (Item E43) A recording medium storing a computer program for having a computer execute a method for treating or preventing a disease, disorder, or biological condition, the method comprising the steps of:
         (aa) extracting a feature for each sequence constituting at least a pair of members of the collection of immunological entities;
         (bb) projecting the feature onto a high dimensional vector space, wherein a distance on the space between the members reflects functional similarity of the members;
         (cc) clustering the collection of immunological entities based on the distance;
         (dd) optionally analyzing based on a classification by the clustering; and
         (ee) administering the immunological entities analyzed in (dd) or an immunological entity binder corresponding to the immunological entities.
      (Item E43A) The recording medium of item E43, further comprising one or more features of the preceding items.
      (Item E44) A recording medium storing a computer program for having a computer execute a method for diagnosing a disease, disorder, or biological condition, the method comprising the steps of:
         (i) providing a feature of at least two immunological entities, wherein the at least two immunological entities comprise at least one immunological entity derived from a healthy individual;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) judging a disease, disorder, or biological condition based on the immunological entities classified or determined in (iii).
      (Item E44A) The recording medium of item E44, further comprising one or more features of the preceding items.
      (Item E45) The recording medium of item E44 or E44A, wherein the disease, disorder, or biological condition comprises an adverse event.
      (Item E46) A recording medium storing a computer program for having a computer execute a method for diagnosing a disease, disorder, or biological condition, the method comprising the steps of:
         (a) extracting a feature for at least a pair of members of the collection of immunological entities, wherein the collection of immunological entities comprises at least one immunological entity derived from a healthy individual;
         (b) computing a distance between antigen specificities or binding modes or judging whether the antigen specificities or binding modes match for the pair by machine learning using the feature;
         (c) clustering the collection of immunological entities based on the distance;
         (d) analyzing based on a classification by the clustering; and
         (e) judging a disease, disorder, or biological condition based on the immunological entities analyzed in (d).
      (Item E46A) The recording medium of item E46, further comprising one or more features of the preceding items.
      (Item E47) The recording medium of item E46 or E46A, wherein the disease, disorder, or biological condition comprises an adverse event.
      (Item E48) A recording medium storing a computer program for having a computer execute a method for diagnosing a disease, disorder, or biological condition, the method comprising the steps of:
         (aa) extracting a feature for each sequence constituting at least a pair of members of the collection of immunological entities, wherein the collection of immunological entities comprises at least one immunological entity derived from a healthy individual;
         (bb) projecting the feature onto a high dimensional vector space, wherein a distance on the space between the members reflects functional similarity of the members;
         (cc) clustering the collection of immunological entities based on the distance;
         (dd) analyzing based on a classification by the clustering; and
         (ee) judging a disease, disorder, or biological condition based on the immunological entities analyzed in (dd).
      (Item E48A) The recording medium of item E48, further comprising one or more features of the preceding items.
      (Item E49) The recording medium of item E48 or E48A, wherein the disease, disorder, or biological condition comprises an adverse event.
      (Item E50) A recording medium storing a computer program for having a computer execute a method for treating or preventing a disease, disorder, or biological condition, the method comprising the steps of:
         (i) providing a feature of at least two immunological entities, wherein the at least two immunological entities comprise at least one immunological entity derived from a healthy individual;
         (ii) subjecting analysis of antigen specificity or binding mode of the immunological entities to machine learning without specifying antigen specificity or binding mode based on the feature;
         (iii) classifying the antigen specificity or binding mode or determining whether the antigen specificity or binding mode is the same/different; and
         (iv) administering the immunological entities classified or determined in (iii) or an immunological entity binder corresponding to the immunological entities.
      (Item E50A) The recording medium of item E50, further comprising one or more features of the preceding items.
      (Item E51) The recording medium of item E50 or E50A, wherein the disease, disorder, or biological condition comprises an adverse event, or the treatment or prevention comprises treating or preventing while avoiding an adverse event.
      (Item E52) A recording medium storing a computer program for having a computer execute a method for treating or preventing a disease, disorder, or biological condition, the method comprising the steps of:
         (a) extracting a feature for at least a pair of members of the collection of immunological entities, wherein the collection of immunological entities comprises at least one immunological entity derived from a healthy individual;
         (b) computing a distance between antigen specificities or binding modes or judging whether the antigen specificities or binding modes match for the pair by machine learning using the feature;
         (c) clustering the collection of immunological entities based on the distance;
         (d) optionally analyzing based on a classification by the clustering; and
         (e) administering the immunological entities analyzed in (d) or an immunological entity binder corresponding to the immunological entities.
      (Item E52A) The recording medium of item E52, further comprising one or more features of the preceding items.
      (Item E53) The recording medium of item E52 or E52A, wherein the disease, disorder, or biological condition comprises an adverse event, or the treatment or prevention comprises treating or preventing while avoiding an adverse event.
      (Item E54) A recording medium storing a computer program for having a computer execute a method for treating or preventing a disease, disorder, or biological condition, the method comprising the steps of:
         (aa) extracting a feature for each sequence constituting at least a pair of members of the collection of immunological entities, wherein the collection of immunological entities comprises at least one immunological entity derived from a healthy individual;
         (bb) projecting the feature onto a high dimensional vector space, wherein a distance on the space between the members reflects functional similarity of the members;
         (cc) clustering the collection of immunological entities based on the distance;
         (dd) optionally analyzing based on a classification by the clustering; and
         (ee) administering the immunological entities analyzed in (dd) or an immunological entity binder corresponding to the immunological entities.
      (Item E54A) The recording medium of item E54, further comprising one or more features of the preceding items.
      (Item E55) The recording medium of item E54 or E54A, wherein the disease, disorder, or biological condition comprises an adverse event, or the treatment or prevention comprises treating or preventing while avoiding an adverse event.
      (Item F38) A system for diagnosing a disease, disorder, or biological condition, comprising:
         (I) a feature providing unit for providing a feature of at least two immunological entities;
         (II) a machine learning unit for subjecting analysis of antigen specificity or binding mode of the immunological entities to machine learning without specifying antigen specificity or binding mode based on the feature;
         (III) a classification unit for classifying the antigen specificity or binding mode or determining whether the antigen specificity or binding mode is the same/different; and
         (IV) a judging unit for judging a disease, disorder, or biological condition based on the immunological entities classified or determined in (III).
      (Item F38A) The system of item F38, further comprising one or more features of the preceding items.
      (Item F39) A system for diagnosing a disease, disorder, or biological condition, the system comprising:
         (A) a feature providing unit for extracting a feature for at least a pair of members of the collection of immunological entities;
         (B) a judging unit for computing a distance between antigen specificities or binding modes or judging whether the antigen specificities or binding modes match for the pair by machine learning using the feature;
         (C) a clustering unit for clustering the collection of immunological entities based on the distance;
         (D) an analysis unit for optionally analyzing based on a classification by the clustering; and
         (E) a biological condition judging unit for judging a disease, disorder, or biological condition based on the immunological entities analyzed in (D).
      (Item F39A) The system of item F39, further comprising one or more features of the preceding items.
      (Item F40) A system for diagnosing a disease, disorder, or biological condition, the system comprising:
         (A) a feature providing unit for extracting a feature for each sequence constituting at least a pair of members of the collection of immunological entities;
         (B') a projection unit for projecting the feature onto a high dimensional vector space, wherein a distance on the space between the members reflects functional similarity of the members;
         (C) a clustering unit for clustering the collection of immunological entities based on the distance;
         (D) an analysis unit for optionally analyzing based on a classification by the clustering; and
         (E)a biological condition judging unit for judging a disease, disorder, or biological condition based on the immunological entities analyzed in (D).
      (Item F40A) The system of item F40, further comprising one or more features of the preceding items.
      (Item F41) A system for treating or preventing a disease, disorder, or biological condition, the system comprising:
         (I) a feature providing unit for providing a feature of at least two immunological entities;
         (II) a machine learning unit for subjecting analysis of antigen specificity or binding mode of the immunological entities to machine learning without specifying antigen specificity or binding mode based on the feature;
         (III) a classification unit for classifying the antigen specificity or binding mode or determining whether the antigen specificity or binding mode is the same/different; and
         (IV) an administration unit for administering the immunological entities classified or determined in (III) or an immunological entity binder corresponding to the immunological entities.
      (Item F41A) The system of item F41, further comprising one or more features of the preceding items.
      (Item F42) A system for treating or preventing a disease, disorder, or biological condition, the system comprising:
         (A) a feature providing unit for extracting a feature for at least a pair of members of the collection of immunological entities;
         (B) a judging unit for computing a distance between antigen specificities or binding modes or judging whether the antigen specificities or binding modes match for the pair by machine learning using the feature;
         (C) a clustering unit for clustering the collection of immunological entities based on the distance;
         (D) an analysis unit for optionally analyzing based on a classification by the clustering; and
         (E) an administration unit for administering the immunological entities analyzed in (D) or an immunological entity binder corresponding to the immunological entities. (Item F42A) The system of item F42, further comprising one or more features of the preceding items.
      (Item F43) A system for treating or preventing a disease, disorder, or biological condition, the system comprising:
         (A) a feature providing unit for extracting a feature for each sequence constituting at least a pair of members of the collection of immunological entities;
         (B') a projection unit for projecting the feature onto a high dimensional vector space, wherein a distance on the space between the members reflects functional similarity of the members;
         (C) a clustering unit for clustering the collection of immunological entities based on the distance;
         (D) an analysis unit for optionally analyzing based on a classification by the clustering; and
         (E) an administration unit for administering the immunological entities analyzed in (D) or an immunological entity binder corresponding to the immunological entities.
      (Item F43A) The system of item F43, further comprising one or more features of the preceding items.
      (Item F44) A system for diagnosing a disease, disorder, or biological condition, the system comprising:
         (I) a feature providing unit for providing a feature of at least two immunological entities, wherein the at least two immunological entities comprise at least one immunological entity derived from a healthy individual;
         (II) a machine learning unit for subjecting analysis of antigen specificity or binding mode of the immunological entities to machine learning without specifying antigen specificity or binding mode based on the feature;
         (III) a classification unit for classifying the antigen specificity or binding mode or determining whether the antigen specificity or binding mode is the same/different; and
         (IV) an administration unit for administering the immunological entities classified or determined in (III) or an immunological entity binder corresponding to the immunological entities.
      (Item F44A) The system of item F44, further comprising one or more features of the preceding items.
      (Item F45) The system of item F44 or F44A, wherein the disease, disorder, or biological condition comprises an adverse event.
      (Item F46) A system for diagnosing a disease, disorder, or biological condition, the system comprising:
         (A) a feature providing unit for extracting a feature for at least a pair of members of the collection of immunological entities, wherein the collection of immunological entities comprises at least one immunological entity derived from a healthy individual;
         (B) a judging unit for computing a distance between antigen specificities or binding modes or judging whether the antigen specificities or binding modes match for the pair by machine learning using the feature;
         (C) a clustering unit for clustering the collection of immunological entities based on the distance;
         (D) an analysis unit for optionally analyzing based on a classification by the clustering; and
         (E)a biological condition judging unit for judging a disease, disorder, or biological condition based on the immunological entities analyzed in (D).
      (Item F46A) The system of item F46, further comprising one or more features of the preceding items.
      (Item F47) The system of item F46 or F46A, wherein the disease, disorder, or biological condition comprises an adverse event.
      (Item F48) A system for diagnosing a disease,: disorder, or biological condition, the system comprising:
         (A) a feature providing unit for extracting a feature for each sequence constituting at least a pair of members of the collection of immunological entities, wherein the collection of immunological entities comprises at least one immunological entity derived from a healthy individual;
         (B') a projection unit for projecting the feature onto a high dimensional vector space, wherein a distance on the space between the members reflects functional similarity of the members;
         (C) a clustering unit for clustering the collection of immunological entities based on the distance;
         (D) an analysis unit for optionally analyzing based on a classification by the clustering; and
         (E) a biological condition judging unit for judging a disease, disorder, or biological condition based on the immunological entities analyzed in (D).
      (Item F48A) The system of item F48, further comprising one or more features of the preceding items.
      (Item F49) The system of item F48 or F48A, wherein the disease, disorder, or biological condition comprises an adverse event.
      (Item F50) A system for treating or preventing a disease, disorder, or biological condition, the system comprising:
         (I) a feature providing unit for providing a feature of at least two immunological entities, wherein the at least two immunological entities comprise at least one immunological entity derived from a healthy individual;
         (II) a machine learning unit for subjecting analysis of antigen specificity or binding mode of the immunological entities to machine learning without specifying antigen specificity or binding mode based on the feature;
         (III) a classification unit for classifying the antigen specificity or binding mode or determining whether the antigen specificity or binding mode is the same/different; and
         (IV) an administration unit for administering the immunological entities classified or determined in (III) or an immunological entity binder corresponding to the immunological entities.
      (Item F50A) The system of item F50, further comprising one or more features of the preceding items.
      (Item F51) The system of item F50 or F50A, wherein the disease, disorder, or biological condition comprises an adverse event, or the treatment or prevention comprises treating or preventing while avoiding an adverse event.
      (Item F52) A system for treating or preventing a disease, disorder, or biological condition, the system comprising:
         (A) a feature providing unit for extracting a feature for at least a pair of members of the collection of immunological entities, wherein the collection of immunological entities comprises at least one immunological entity derived from a healthy individual;
         (B) a judging unit for computing a distance between antigen specificities or binding modes or judging whether the antigen specificities or binding modes match for the pair by machine learning using the feature;
         (C) a clustering unit for clustering the collection of immunological entities based on the distance;
         (D) an analysis unit for optionally analyzing based on a classification by the clustering; and
         (E) an administration unit for administering the immunological entities analyzed in (D) or an immunological entity binder corresponding to the immunological entities.
      (Item F52A) The system of item F52, further comprising one or more features of the preceding items.
      (Item F53) The system of item F52 or F52A, wherein the disease, disorder, or biological condition comprises an adverse event, or the treatment or prevention comprises treating or preventing while avoiding an adverse event.
      (Item F54) A system for treating or preventing a disease, disorder, or biological condition, the system comprising:
         (A) a feature providing unit for extracting a feature for each sequence constituting at least a pair of members of the collection of immunological entities, wherein the collection of immunological entities comprises at least one immunological entity derived from a healthy individual;
         (B') a projection unit for projecting the feature onto a high dimensional vector space, wherein a distance on the space between the members reflects functional similarity of the members;
         (C) a clustering unit for clustering the collection of immunological entities based on the distance;
         (D) an analysis unit for optionally analyzing based on a classification by the clustering; and
         (E) an administration unit for administering the immunological entities analyzed in (D) or an immunological entity binder corresponding to the immunological entities.
      (Item F54A) The system of item F54, further comprising one or more features of the preceding items.
      (Item F55) The system of item F54 or F54A, wherein the disease, disorder, or biological condition comprises an adverse event, or the treatment or prevention comprises treating or preventing while avoiding an adverse event.

The lack of need to identify an immunological entity binder such as an antigen prior to finding a TCR is an important advantage of the clustering algorithm of the invention. The technology of the invention does not require prior knowledge of an immunological entity binder such as an antigen. One of the fascinating applications of the technology of the invention is in the use of an antibody or TCR cluster for identification of a drug development target candidate or a biomarker of a disease, an antibody drug, or genetically modified T cell therapy as a chimeric antigen receptor. For example, it is known that BCRs and TCRs exhibit a typical sequence pattern in a certain type of leukemia or lymphoma, so that identification thereof can be used in diagnosis of diseases without knowing the immunological entity binder such as an antigen.

The present invention is intended so that one or more of the aforementioned features can be provided not only as the explicitly disclosed combinations, but also as other combinations. Additional embodiments and advantages of the present invention are recognized by those skilled in the art by reading and understanding the following detailed description, as needed.

### [Advantageous Effects of Invention]

Clustering of antibodies or TCRs by epitope yields an actual significant effect. In particular, clusters classified by each immunological entity binder (e.g., antigen), antigen specificity, binding mode, or epitope are themselves valuable, even if an immunological entity binder (e.g., antigen) is not identified. Such clustering has some direct advantages. For example, this enables comparison of antibodies or TCR repertoires from different individuals (e.g., donor X, compared to donor Y, has more expression of cluster Z). Further, there is a possibility for discovery of a disease specific, novel immunological entity binder (e.g., antigen) or epitope. A discovery of a novel immunological entity binder (e.g., antigen) is extremely valuable in drug development. In addition, an antibody to an epitope of interest can be quantitatively evaluated. More quantitative and higher resolution/higher precision information is obtained in combination with an existing protein chip. Moreover, this can facilitate downstream analysis and reduce cost. For example, instead of screening N BCRs or TCRs, if N receptors are contained in M clusters (N > M), analysis can be completed by M rounds of screenings. Furthermore, virtual screening (estimation of an immunological entity binder (e.g., antigen) or epitope by similarity search) using BCRs or TCRs with a known immunological entity binder (e.g., antigen), antigen specificity, binding mode, or epitope can be performed. One feature thereof is that this can be a technology that is complementary to experimental screening.

Since antibodies with different amino acid sequences can recognize the same epitope or have the same antigen specificity or binding mode, conventional bioinformatics tools such as sequence alignment are not methodologies that are suitable for clustering of antibodies by epitope. While structural bioinformatics have docking for predicting the so-called protein complex structure, and methodologies that predict the complex structure based on similarity to the interface of a known protein complex, these are also not methodologies that are suitable for clustering of antibodies by epitope. TCRs also have a similar problem, but the problem is further complicated in that an immunological entity binder (e.g., antigen) is a complex of a one-dimensional peptide and an MHC which is a molecule presenting the peptide, where MHCs are themselves diverse. Therefore, the invention is important in that conventional methodologies are not able to cluster antibodies or TCRs by epitope, antigen specificity, or binding mode with a robust scheme.

### [Brief Description of Drawings]

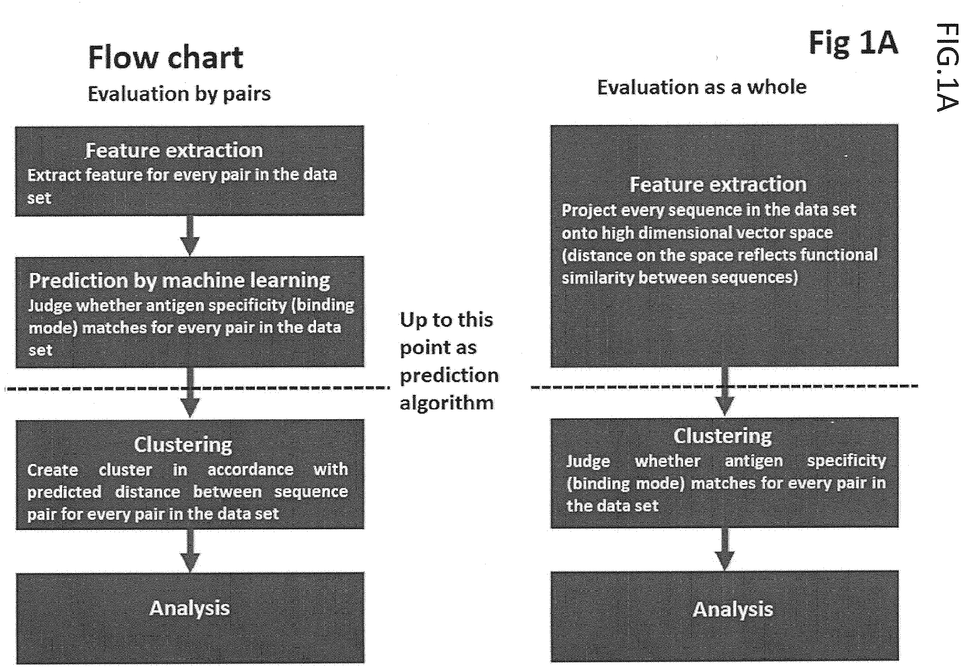

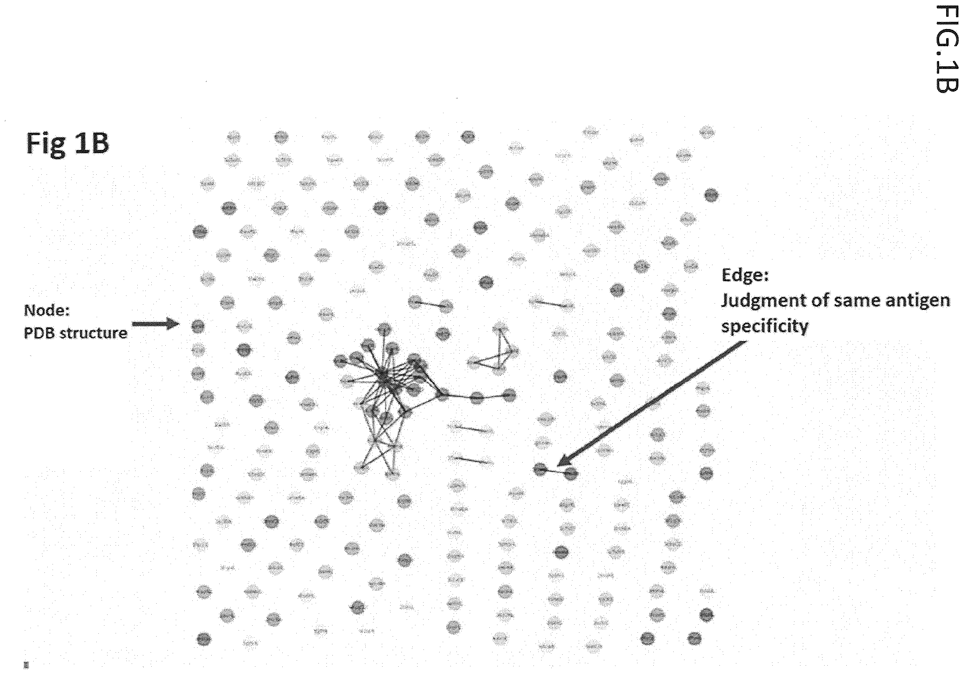

[Figure 1A] Figure **1A** is a flowchart exemplifying the embodiment of the invention. The left side depicts evaluation by pairs, and the right side depicts evaluation from the whole. Projection in accordance with the type of data set is performed, when the distance between each sequence is known in advance, by (for example) the method on the left side by (learning) in advance. If the distance (in terms of antigen specificity) between each sequence is known, a sequence is projected as a vector on a multi-dimensional space so that the distance between the sequences is reproduced (e.g., by using a neural network). Any feature can be extracted from each sequence and inputted into a neural network. (Prediction) If a feature extracted from a sequence is inputted into a model obtained by the learning described above, a prediction result is obtained. If the true/false of antigen specificity of each sequence is known in advance, projection is performed onto a high dimensional space so that true antigen specificity sequence pairs are close and false sequence pairs are close (e.g., by using a neural network). At this time, an input into a neural network is any feature vector extracted from each sequence. An optimal model is constructed through learning in accordance with the distance between each sequence in a high dimensional space. (Prediction) If a feature extracted from a sequence is inputted into a model obtained by the learning described above, a prediction result is obtained.
[Figure 1B] Figure **1B** depicts the result of BCR clustering on a test set. A node represents each PDB structure, and an edge is judged as having the same antigen specificity as a result of prediction.
[Figure 2] Figure **2** depicts results of clustering TCRs that recognize 20 epitopes.
[Figure 3] Figure **3** depicts a crystal structure (left: overwriting the structure obtained from PDB) corresponding to the results of EBV derived epitope (FLRGRAYGL (SEQ ID NO: 1)) specific TCR clustering (right).
[Figure 4] Figure **4** depicts the results of clustering two types of HIV derived peptide specific TCRs and TCRs in a database.
[Figure 5] Figure **5** is a schematic diagram of the system of the invention.
[Figure 6] Figure **6** is a schematic diagram of an exemplary flow chart for implementing the invention. The left side depicts evaluation by pairs, and the right side depicts evaluation from the whole.
[Figure 7] Figure **7** is a schematic diagram of breast cancer diagnosis using clustering using TCRs of the invention.
[Figure 8] Figure **8** is a schematic diagram of TCR clustering using autoencoder of the invention. (Left) shows the schematic diagram of Autoencoder and (right) shows the results of clustering (DBSCAN) parameter optimization.
[Figure 9] Figure **9** is a schematic diagram of diagnosis combining biological information of cells other than TCR/BCR of the invention. (Left) shows the compared cohorts and (right) shows a Venn diagram depicting the results thereof.
[Figure 10] Figure **10** depicts the flow chart of Example 7.
[Figure 11] Figure **11** depicts results of breast cancer diagnosis using clustering using TCRs constructed using only a cluster consisting of a plurality of donors.
[Figure 12] Figure **12** depicts an exemplary diagram for predicting side effects of immune checkpoint inhibitors of the invention.

### [Description of Embodiments]

The present invention is described hereinafter with the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

### (Definitions)

The definitions of the terms and/or the detailed basic technology that are particularly used herein are described hereinafter as appropriate.

As used herein, "immunological entity" refers to any substance responsible for an immune reaction. Immunological entities include antibodies, antigen binding fragments of an antibody, T cell receptors, fragments of a T cell receptor, B cell receptors, fragments of a B cell receptor, chimeric antigen receptors (CARs), cells comprising one or more of them (e.g., T cells comprising a chimeric antigen receptor (CAR) (CAR-T)), and the like. Immunological entities can be a broad concept, similarly including immunologically related entities used in analysis of a phage display or the like (including scFv and nanobodies) artificially imparted with diversity and nanobodies produced by an animal such as an alpaca. As used herein, descriptions of "first", "second", etc. ("third" ... and the like) indicate that entities are different from one another, unless specifically noted otherwise.

As used herein, "antibody" is used in the same meaning that is commonly used in the art and refers to a protein reacting highly and specifically to an antigen, which is made in the immune system when an antigen contacts the biological immune system (antigen stimulation). Each of the antibodies to an epitope used in the present invention may be of any origin, type, shape, or the like, as long as the antibody binds to the specific epitope. The antibodies described herein can be divided into framework regions and antigen binding regions (CDR).

As used herein, "T cell receptor (TCR)" is also called as a T cell antigen receptor. A T cell receptor refers to a receptor recognizing an antigen, expressed on a cell membrane of a T cell that plays a central role in the immune system. TCRs have an α chain, β chain, γ chain, and δ chain, with which an αβ or γδ dimer is constituted. TCRs consisting of the combination of the former are called αβ TCRs, and TCRs consisting of the combination of the latter are called γδ TCRs. T cells having such TCRs are respectively called αβ T cells and γδ T cells. TCRs are structurally very similar to a Fab fragment of an antibody produced by B cells and recognize antigen molecules bound to an MHC molecule. Since a TCR gene of a mature T cell has undergone gene rearrangement, an individual has highly diverse TCRs that enable recognition of various antigens. TCRs also form a complex by binding to a non-variable CD3 molecule at the cell membrane. CD3 has an amino acid sequence called ITAM (immunoreceptor tyrosine-based activation motif) in the intracellular region. This motif is considered to be involved in intracellular signaling. Each TCR chain is comprised of a variable domain (V) and a constant domain (C). A constant domain has a short cytoplasm section penetrating the cell membrane. A variable domain is present outside the cell and binds to an antigen-MHC complex. A variable domain has three hypervariable domains or regions called complementarity-determining regions (CDRs), which bind to an antigen-MHC complex. The three CDRs are called CDR1, CDR2, and CDR3. TCR gene rearrangement is similar to the process of B cell receptors known as immunoglobulins. For gene rearrangement of αβ TCRs, VDJ recombination of a β chain is performed, followed by VJ recombination of an α chain. When the α chain is rearranged, the gene of the δ chain is deleted from the chromosome. Thus, a T cell having an αβ TCR would never have a γδ TCR simultaneously. In contrast, a signal via a γδ TCR in a T cell having the TCR suppresses the expression of a β chain, so that a T cell having a γδ TCR would never have an αβ TCR simultaneously.

As used herein, "B cell receptor (BCR)" is also called a B cell antigen receptor, referring to those comprised of Igα/Igβ (CD79a/CD79b) heterodimer (α/β) associated with a membrane bound immunoglobulin (mIg) molecule. An mIg subunit binds to an antigen to induce aggregation of receptors, while an α/β subunit transmits a signal toward the cell. Aggregation of BCRs is understood to quickly activate Lyn, Blk, and Fyn of an Src family kinase in the same manner as Syk and Btk of tyrosine kinase. Many different results are produced depending on the complexity of BCR signaling. Examples thereof include survival, resistance (anergy; lack of hypersensitive reaction to an antigen) or apoptosis, cell division, differentiation into an antibody producing cell or memory B cell, and the like. Hundreds of millions of types of T cells with different sequences of the variable regions of TCRs are produced, and hundreds of millions of types of B cells with different sequences of the variable regions of BCRs (or antibodies) are produced. Since the individual sequences of TCRs and BCRs vary due to rearrangement or mutation of the genomic sequence, a clue for antigen specificity of a T cell or B cell can be found by determining the sequence of mRNA (cDNA) or the genomic sequence of TCR/BCR.

As used herein, "chimeric antigen receptor (CAR)" is a collective term for chimeric proteins having a single chain antibody (scFv) having a light chain (VL) and a heavy chain (VH) of a tumor antigen specific monoclonal antibody variable region bound in series on the N-terminus side, and a T cell receptor (.TCR) ζ chain on the C-terminus side. A chimeric antigen receptor is an artificial T cell receptor used in gene and cell therapy, in which an artificial T cell receptor that is genetically engineered to defeat the immune evasion mechanism of tumor is transfected into patient T cells, which are amplified and cultured outside the body and then injected into a patient (Dotti G, et al., Hum Gene Ther 20: 1229-1239, 2009). Such a CAR can be produced using an epitope that is identified or clustered by the present invention. Gene and cell therapy can be materialized using the produced CAR or genetically modified T cells comprising such a CAR (see Credit: Brentjens R, et al. "Driving CAR T cells forward." Nat Rev Clin Oncol. 2016 13, 370-383 and the like).

As used herein, "V region" refers to a variable domain (V) region of a variable region of an immunological entity such as an antibody, TCR, or BCR.

As used herein, "D region" refers to a D region of a variable region of an immunological entity such as an antibody, TCR, or BCR.

As used herein, "J region" refers to a J region of a variable region of an immunological entity such as an antibody, TCR, or BCR.

As used herein, "C region" refers to a constant domain (C) region of an immunological entity such as an antibody, TCR, or BCR.

As used herein, "repertoire of a variable region" refers to a collection of V(D)J regions optionally created by gene rearrangement in TCR or BCR. The phrases TCR repertoire, BCR repertoire and the like are used, but they can also be called, for example, T cell repertoire, B cell repertoire, or the like. For example, "T cell repertoire" refers to a collection of lymphocytes characterized by the expression of a T cell receptor (TCR) serving an important role in antigen recognition or recognition of an immunological entity binder. Since a change in a T cell repertoire is a significant indicator of an immune state in a diseased state or physiological state, T cell repertoire analysis has been performed for identification of antigen specific T cells involved in the development of a disease and diagnosis of T lymphocyte abnormalities. TCRs and BCRs create diverse genetic sequences by gene rearrangement of multiple gene fragments of the V region, D region, J region, and C region on the genome.

As used herein, "isotype" refers to IgM, IgA, IgG, IgE, IgD, and the like, which belong to the same type but have different sequences from one another. Isotypes are denoted using various gene abbreviations and symbols.

As used herein, "subtype" is a type within a type in IgA and IgG for BCRs. IgG has IgG1, IgG2, IgG3, and IgG4, and IgA has IgA1 and IgA2. Subtypes are also known to be in β and γ chains for TCRs, having TRBC1 and TRBC2 and TRGC1 and TRGC2, respectively.

As used herein, "immunological entity binder" refers to any substrate that can be specifically bound by an immunological entity such as an antibody, TCR, or BCR. When denoted as "antigen" herein, the antigen can broadly refer to an "immunological entity binder". "Antigen" can be used narrowly in pair with an antibody and refers narrowly to any substrate that can be specifically bound by an "antibody" in the art.

As used herein, "epitope" refers to a site in a molecule of an immunological entity binder (e.g., antigen), to which an immunological entity such as an antibody or a lymphocyte receptor (TCR, BCR, or the like) binds. While an amino acid straight chain can constitute an epitope (linear epitope), separated sites of a protein can also constitute a stereo structure to function as an epitope (conformational epitope). Epitopes of the invention are not limited by such detailed classification of epitopes. It is understood that if certain immunological entities such as antibodies have the same epitope, an immunological entity such as an antibody having another sequence can also be used in the same manner.

As used herein, "antigen specificity" refers to, in the context of an immunological entity, binding specificity to a binding partner thereof (e.g., antigen), referring to a property of binding to a specific binding partner, but does not bind to other binding partners, or binds thereto with a low affinity.

As used herein, "binding mode" refers to the three-dimensional binding form (mode) between an immunological entity and a binding partner thereof, representing a physical concept. Although not wishing to be bound by any theory, a collection of a plurality of binding modes is generally understood to form antigen specificity, but the concept is not limited thereto.

As used herein, whether immunological entities, epitopes, immunological entity binders, antigen specificities, or binding modes are the "same" or "different" can be determined by similarity (amino acid sequence, three-dimensional structure, antigen specificity, binding mode, or the like) in accordance with the classification based on the present invention. "Same" is not limited to complete identity of chemical formulas, amino acid sequences, or the like, but refers to substantially the same quality of function or stereo structure. Typically, this can be determined by antigen specificity or binding mode in the present invention. Immunological entities, epitopes, immunological entity binders, antigen specificities, or binding modes belonging to the same immunological entity, epitope, immunological entity binder, antigen specificity, or binding mode cluster are determined to be the "same" in the present invention. Therefore, "different" immunological entities, epitopes, immunological entity binders, antigen specificities, or binding modes refer to immunological entities, epitopes, immunological entity binders, antigen specificities, or binding modes that do not belong to the "same" cluster. In one embodiment, it can be determined whether immunological entities, epitopes, immunological entity binders, antigen specificities, or binding modes belong to the same cluster depending on whether the immunological entities, epitopes, immunological entity binders, antigen specificities, or binding modes are the "same" or "different". When performing cluster analysis, an immunological entity, epitope, immunological entity binder, antigen specificity, or binding mode is, in comparison to another immunological entity, epitope, immunological entity binder, antigen specificity, or binding mode, determined to be the same if belonging to the same cluster, and determined to be different if belonging to a different cluster. Therefore, immunological entities, epitopes, or immunological entity binders that bind to the same immunological entity, epitope, immunological entity binder, antigen specificity, or binding mode can be classified into the same cluster to generate the cluster. Immunological entities, epitopes, or immunological entity binders can also be evaluated for at least one endpoint selected from the group consisting of properties and similarity with a known immunological entity thereof to perform the cluster classification by targeting an immunological entity, epitope, or immunological entity binder meeting a predetermined baseline. Thus in one embodiment, when the immunological entities, epitopes, immunological entity binders, antigen specificities, or binding modes are the same, the three-dimensional structures of the immunological entities, epitopes, immunological entity binders, antigen specificities, or binding modes can at least partially or completely overlap, or the amino acid sequences of the immunological entities, epitopes, or immunological entity binders (or responsible for antigen specificities or binding modes) or a partial structure of another chemical substance can at least partially or completely overlap. It is suitable to determine a threshold value as an important indicator to be highly compatible with structural data or the like that can be confirmed with certainty, but other threshold values can also be employed when prioritizing statistical significance. Those skilled in the art can determine an appropriate threshold value by referring to the descriptions herein, depending on the situation. For example, a pair with a maximum distance, which is found by clustering analysis using a hierarchical clustering methodology (e.g., group average method (average linkage clustering), nearest neighbor method (NN method), K-NN method, Ward method, furthest neighbor method, or centroid method), of less than a specific value can be deemed to be in the same cluster. Examples of such a value include, but are not limited to, less than 1, less than 0.95, less than 0.9, less than 0.85, less than 0.8, less than 0.75, less than 0.7, less than 0.65, less than 0.6, less than 0.55, less than 0.5, less than 0.45, less than 0.4, less than 0.35, less than 0.3, less than 0.25, less than 0.2, less than 0.15, less than 0.1, less than 0.05, and the like. The clustering methodology is not limited to hierarchical methodologies. A non-hierarchical methodology may also be used.

As used herein, "cluster" of immunological entities, epitopes, immunological entity binders, antigen specificities, or binding modes generally refers to elements that are similar among elements of a population (in this case immunological entities, epitopes, immunological entity binders, antigen specificities, or binding modes) collected from a distribution of the elements in a multi-dimensional space without external standards or designation of the number of groups. As used herein, a cluster refers to a collection with at least one of immunological entity, epitope, immunological entity binder, antigen specificity, and binding mode that is similar. Immunological entities, epitopes, immunological entity binders, antigen specificities, or binding modes belonging to the same cluster bind to similar antibodies. Immunological entities, epitopes, immunological entity binders, antigen specificities, or binding modes can be classified by multivariate analysis. A cluster can be configured using various cluster analysis methodologies. A cluster of immunological entities, epitopes, immunological entity binders, antigen specificities, or binding modes provided by the present invention has been demonstrated to reflect the biological condition (e.g., disease, disorder, or drug efficacy, especially immune state or the like) by showing that an immunological entity, epitope, immunological entity binder, antigen specificity, or binding mode belongs to the cluster.

For example, it is assumed that analysis based on classification by clustering in the present invention deems each cluster as a gene from clustering results and uses the clusters in a similar manner to gene expression analysis. Specifically, it is assumed, for example, that the following can be performed: 1. When following a chronological change, the increase/decrease in sequences belonging to a specific or a plurality of clusters is observed. The number of increased/decreased clusters is observed. The feature (V/D/J gene, length of CDRs, hydrophilicity, hydrophobicity, conserved residue, or the like) of each cluster is found. 2. When interested in a specific layer of a plurality of specimens, a cluster that is predominantly present or increasing/decreasing in a specific layer is identified. The number of increased/decreased clusters is observed. The feature of each cluster is found. 3. When interested in a function (function = antigen specificity or binding mode), focus is placed on a cluster comprising a sequence specific to an antigen of interest (assumed to be obtained by another experiment such as an ELISPOT assay or sorting with a pMHC tetramer) to observe the increase/decrease thereof. (Function = function of a cell) Results of clustering obtained from cells of different subtypes that are separately sorted and sequenced are compared. 4. When comparing with another experiment source, gene expression analysis, omics analysis, correlation with bacterial flora, cytokines, number of cell species, or analysis from 1 to 3 combined therewith can be appropriately used.

As used herein, "machine learning" is understood in the broadest meaning used in the art, referring to learning by a machine (computer). Machine learning is a technology/methodology that attempts to materialize the same function as the ability to learn performed naturally by humans with a computer. For a machine to learn, data that is used as the basis of learning is used as an input value. The input value is passed through processing known as a "machine learning algorithm" to find processing for classifying or recognizing data. Use of such learned processing enables classification or identification of data yet to be learned, which has been inputted after learning. Classification, recognition, identification, or regression (prediction) can be performed by machine learning. Machine learning can be supervised learning or unsupervised learning, or a methodology known as reinforcement learning. Deep learning can be considered as a part of machine learning, and machine learning can be considered as a part of artificial intelligence (AI). Machine learning refers to artificial intelligence in which a developer does not program all operations, but the AI itself analyzes data and finds a regularity or rule, i.e., a specific task can be executed by training. Deep learning refers to one of the machine learning methodologies, which is an advanced form of a neural network and related technologies and is, unlike conventional machine learning, artificial intelligence that reinforces data analysis and learning by stacking multiple layers of neural networks modeled after human nerves. To perform an overall evaluation with an auto-encoder used in deep learning, an immunological entity sequence is projected onto a high dimensional vector space with the sequence itself as an input. Specifically, the auto-encoder itself extracts a feature and projects the feature onto a high dimensional vector space. The feature directly becomes a high dimensional vector space element. Projection can be interpreted as including identity mapping.

As used herein, "classification" refers to, in the context of antigen specificity or binding mode, separation into groups with the same antigen specificity or binding mode property based on a certain standard. In the present invention, classification can be performed by clustering.

As used herein, "same/different" refers to, in the context of antigen specificity or binding mode, whether the antigen specificity or binding mode has the same property or structure.

As used herein, "specifying antigen specificity or binding mode" refers to focusing only on a specific antigen of interest, or a binding mode pertaining to the antigen. This can be considered as specifying a subject of analysis.

As used herein, "not specifying antigen specificity or binding mode" refers to generically (and preferably equally) handling antigen specificity or binding mode with respect to various antigens, instead of a specific antigen of interest or a binding mode pertaining to the antigen.

As used herein, "similarity" refers to the degree similarity in molecules for molecules such as immunological entity binders (e.g., antigens), immunological entities, epitopes, antigen specificities, or binding modes, a part thereof, spatial arrangement formed thereby, or the like. Similarity can be determined based on the difference in lengths, sequence similarity, or the like. Although not wishing to be bound by any theory, it is understood that antibodies, TCRs, BCRs, or the like binding to an epitope belonging to the same cluster can be assigned to a disease, disorder, symptom, physiological phenomenon, or the like in the same category when immunological entities, epitopes, immunological entity binders, antigen specificities, or binding modes are classified based on such similarity in some of the embodiments of the present invention. Therefore, a variety of diagnoses (incidence of cancer, compatibility of administered drug, and the like) is made possible by studying whether there are antibodies, TCRs, BCRs, or the like that react to the same cluster of immunological entities, epitopes, immunological entity binders, antigen specificities, or binding modes by using the methodologies of the invention. Similarity can be used in the analysis of the invention.

As used herein, "similarity score" refers to a specific numerical value indicating similarity. This is also referred to as "similarity". A suitable score can be appropriately employed depending on the technique used in calculating structural similarity. A similarity score can be computed using, for example, a machine learning algorithm such as a regressive scheme, a neural network method, support vector machine, or random forest. Similarity scores can be used in the analysis of the invention.

As used herein, "feature" refers to an element that is considered to affect the result of analysis or calculation of machine learning or the like. Examples of features that are useful in the analysis of an immunological entity include, but are not limited to, sequence information, lengths of CDR1-3 sequences, a degree of match between sequences, a degree of match between sequences of framework regions, a total charge/hydrophilicity/hydrophobicity/number of aromatic amino acids of a molecule, a charge/hydrophilicity/hydrophobicity/number of aromatic amino acids of each CDR or framework region, number of each amino acid, a combination of heavy chain-light chain, number of somatic hypermutations, a position of a mutation, presence/degree of match of an amino acid motif, a degree of rarity with respect to a reference sequence set, odds of bound HLA according to a reference sequence, and the like, and one or a plurality thereof can be used. A feature is used as an input of a machine learning algorithm as a feature vector.

As used herein, "distance" of antigen specificity refers to judgment on whether antigen specificity is a match. The "distance" can be set to any numerical value. Specifically, when the "distance" is designed to be predicted as 0 or 1, clustering is simply an operation of grouping 1. Meanwhile, if the distance is expressed as [0-1], the advantage of clustering is not just the distance (relationship of a pair) . Other parameters such as the density of pairs in the surrounding can also be considered. The present invention can use either type of distance. Information related to distance is information that can be used for providing a feature in the present invention.

As used herein, "complementarity-determining region (CDR)" is a region forming a binding site by actually contacting an immunological entity binder (e.g., antigen) in an immunological entity such as an antibody. Information related to a CDR is information that can be used for providing a feature in the present invention. In general, a CDR is positioned on Fv (including a heavy chain variable region (VH) and light chain variable region (VL)) of an antibody or a molecule corresponding to an antibody (immunological entity). In general, CDRs have CDR1, CDR2, and CDR3 consisting of about 5 to 30 amino acid residues. In addition, it is known that heavy chain CDRs especially contribute to an antibody binding to an antigen in an antigen-antibody reaction. Among the CDRs, CDR3, especially CDR-H3, is known to contribute the most in an antibody binding to an antigen. For example, "Willy et al., Biochemical and Biophysical Research Communications Volume 356, Issue 1, 27 April 2007, Pages 124-128" describes that the binding capability of an antibody was enhanced by modifying a heavy chain CDR3. A plurality of definitions of CDRs and methods for determining the position thereof have been reported. For example, the definition of Kabat (Sequences of Proteins of Immunological Interest, 5th ed., Public Health Service, National Institutes of Health, Bethesda, MD. (1991)) or Chothia (Chothia et al., J. Mol. Biol., 1987; 196: 901-917) may be employed. In one embodiment of the present invention, the definition of Kabat is used as a suitable example, but the definition is not necessarily limited thereto. In some cases, CDRs can be determined by considering both the definition of Kabat and the definition of Chothia (modified Chothia method). For example, a CDR can be the overlapping portion of CDRs according to each definition or a portion comprising both CDRs according to each of the definitions. Alternatively, a CDR can be determined in accordance with IMGT or Honegger. Specific example of such a method includes the method of Martin et al. using Oxford Molecular's AbM antibody modeling software (Proc. Natl. Acad. Sci. USA, 1989; 86: 9268-9272), which is a combination between the definition of Kabat and the definition of Chothia. The present invention can be practiced using such CDR information. As used herein, "CDR3" refers to the third complementarity-determining region (CDR). Herein, CDR is a region, among the variable region, directly contacting an immunological entity binder (e.g., antigen) with a particularly large variation, and is referred to as a hypervariable region. Each of the variable regions of a light chain and a heavy chain has three CDRs (CDR1 to CDR3) and four FRs (FR1 to FR4) surrounding the three CDRs. Since a CDR3 region is understood to straddle the V region, D region, and J region, a CDR3 region is considered to be a key for a variable region and is used as a subject of analysis.

As used herein, "framework region" refers to a region of an Fv region other than CDRs. A framework region generally consists of FR1, FR2, FR3, and FR4, and is considered relatively well conserved among antibodies (Kabat et al., "Sequence of Proteins of Immunological Interest" US Dept. Health and Human Services, 1983.) Therefore, the present invention can employ a methodology that immobilizes the framework region when comparing each sequence. Information related to a framework region is information that can be used for providing a feature in the present invention.

As used herein, "gene region" refers to a framework region, antigen binding region (CDR), and each of the regions such as the V region, D region, J region, and C region. Such gene regions are known in the art and can be appropriately determined by referring to a database or the like. As used herein, "homology" of genes refers to the degree of identity of two or more genetic sequences to one another. Generally, having "homology" refers to having a high degree of identity or similarity. Therefore, two genes having higher homology have higher identity or similarity of the sequences thereof. Whether two genes have homology can be found by direct comparison of sequences, or by hybridization under stringent conditions for nucleic acids. As used herein, "homology search" refers to a search for homology. Preferably, homology can be searched in silico using a computer. Information related to a gene region is information that can be used for providing a feature in the present invention.

As used herein, "identification" of a region such as an amino acid sequence refers to characterization of the amino acid sequence from a certain viewpoint, and refers to determination of a region identified by a characteristic having one property. Identification includes, but is not limited to, specifically identifying a region comprising an amino acid number, linking a characteristic related to these regions, and the like. As used herein, "division" of a region such as an amino acid sequence refers to characterizing the amino acid sequence and then distinguishing each region determined by a characteristic having one property into separate regions. Such identification and division can be performed using any technology used in the field of bioinformatics such as Kabat, Chotia, modified Chotia, IMGT, Honegger, or the like. Identification of a conserved region exemplified by a framework or the like when processing a region such as an amino acid sequence is an important characteristic herein. Decomposition into conserved regions and non-conserved regions (e.g., CDR or the like) as a result of identification is also envisioned. When identifying and superposing parts of conserved regions or non-conserved regions of two or more immunological entities, it is preferable that the parts of each of the immunological entities have a substantially corresponding relationship. As used herein, "corresponding relationship", in the context of a conserved region, is a relationship in which a part of a first immunological entity and a part of a second immunological entity can be superposed on each other when considering the position of a three-dimensional structure. For a non-conserved region, amino acid residues corresponding to each other when considering the position of a three-dimensional structure would be present by defining the same residues described herein. Therefore, "corresponding relationship" can be confirmed by alignment of a sequence or the like, identification of the same residues or the like.

As used herein, "alignment" ((noun) or align (verb)) refers to primary structures of DNA, RNA, or proteins lined up so that a similar region can be identified in bioinformatics. This often provides a hint to find the functional, structural, or evolutionary relationship of sequences. A sequence of aligned amino acid residues or the like is typically expressed as a row in a matrix, and a gap is inserted so that sequences with the same or similar properties are lined up in the same column. When comparing two sequences, this is called a pairwise sequence alignment, which is used when studying the similarity in a part of or whole alignment of two sequences in detail. For alignment, dynamic programming can be typically used. Representative methodologies that can be used include Needleman-Wunsch method for global alignment, and Smith-Waterman method for local alignment. In this regard, global alignment is alignment for all residues in a sequence and is effective for comparison between sequences of approximately the same length. Local alignment is effective when sequences are not similar as a whole, but it is desirable to find partial similarity. As used herein, "mismatch" refers to the presence of a base or amino acid that is not identical to each other when nucleic acid sequences, amino acid sequences or the like are aligned. "Gap" refers to the presence of a base or amino acid that is present in one, but not in the other in an alignment. Information related to alignment is information that can be used for providing a feature in the present invention.

As used herein, "assign" refers to assignment of information such as a specific gene name, function, or characteristic region (e.g., V region, J region, or the like) to a sequence (e.g., nucleic acid sequence, protein sequence, or the like). Specifically, assignment can be accomplished by inputting or linking specific information to a sequence or the like.

As used herein, "specific" refers to binding to a target sequence, but having low binding capability to, preferably not binding to, another sequence in at least a pool of target antibodies, TCRs, or BCRs, or preferably in all existing antibody, TCR, or BCR sequences. A specific sequence is advantageously, but not necessarily limited to being, fully complementary to a target sequence.

As used herein, "protein", "polypeptide", "oligopeptide" and "peptide" are used herein to have the same meaning and refer to a polymer of amino acids with any length. The polymer may be straight, branched, or cyclic. An amino acid may be a naturally-occurring, non-naturally occurring, or modified amino acid. The term may also encompass those assembled into a complex of multiple polypeptide chains. The term also encompasses naturally-occurring or artificially modified amino acid polymers. Examples of such a modification include disulfide bond formation, glycosylation, lipidation, acetylation, phosphorylation, and any other manipulation or modification (e.g., conjugation with a labeling component). The definition also encompasses, for example, polypeptides comprising one or more analogs of an amino acid (e.g., including non-naturally occurring amino acids and the like), peptide-like compounds (e.g., peptoids), and other known modifications in the art.

As used herein, "amino acid" may be naturally-occurring or non-naturally-occurring amino acids as long as the objective of the present invention is met.

As used herein, "polynucleotide", "oligonucleotide" and "nucleic acid" are used herein to have the same meaning, and refer to a polymer of nucleotides with any length. The term also encompasses "oligonucleotide derivative" and "polynucleotide derivative". "Oligonucleotide derivative" or "polynucleotide derivative" refers to an oligonucleotide or polynucleotide that comprises a nucleotide derivative or has a bond between nucleotides which is different from normal. The terms are used interchangeably. Specific examples of such an oligonucleotide include 2'-O-methyl-ribonucleotide, oligonucleotide derivatives having a phosphodiester bond in an oligonucleotide converted to a phosphorothioate bond, oligonucleotide derivatives having a phosphodiester bond in an oligonucleotide converted to an N3'-P5' phosphoramidate bond, oligonucleotide derivatives having ribose and phosphodiester bond in an oligonucleotide converted to a peptide nucleic acid bond, oligonucleotide derivatives having uracil in an oligonucleotide replaced with C-5 propinyluracil, oligonucleotide derivatives having uracil in an oligonucleotide replaced with C-5 thiazoluracil, oligonucleotide derivatives having cytosine in an oligonucleotide replaced with C-5 propinylcytosine, oligonucleotide derivatives having cytosine in an oligonucleotide replaced with phenoxazine-modified cytosine, oligonucleotide derivatives having ribose in DNA replaced with 2'-O-propylribose, oligonucleotide derivatives having ribose in an oligonucleotide replaced with 2'-methoxyethoxyribose, and the like. Unless noted otherwise, specific nucleic acid sequences are also intended to encompass conservatively modified variants (e.g., degenerate codon substitute) and complement sequences in the same manner as the expressly shown sequences. Specifically, degenerate codon substitutes can be achieved by preparing a sequence with the third position of one or more selected (or all) codons substituted with a mixed base and/or deoxyinosine residue (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). As used herein, "nucleic acid" is used interchangeably with a gene, cDNA, mRNA, oligonucleotide, and polynucleotide. As used herein, a "nucleotide" may be naturally-occurring or non-naturally occurring.

As used herein, "gene" refers to an agent defining a genetic trait. A gene is generally arranged in a certain order on a chromosome. A gene defining the primary structure of a protein is referred to as a structural gene, and a gene determining the expression thereof is referred to as a regulator gene. As used herein, "gene" may refer to "polynucleotide", "oligonucleotide", and "nucleic acid". A "gene product" is a substance produced based on a gene and refers to a protein, mRNA, or the like.

Amino acids may be denoted herein by its generally known three character symbol, or a one character symbol recommended by the IUPAC-IUB Biochemical Nomenclature Commission. Nucleotides may similarly be denoted by a commonly recognized one character code. Comparison of similarity, identity, and homology of an amino acid sequence and a base sequence is computed herein using a sequence analysis tool BLAST with default parameters. For example, identity can be searched by using BLAST 2.2.28 (published on April 2, 2013) of NCBI. Herein, values for identity generally refer to a value obtained by alignment under the default condition using the aforementioned BLAST. However, when a higher value is obtained by changing a parameter, the highest value is considered the value of identity. When identity is evaluated in multiple regions, the highest value thereamong is considered the value of identity. Similarity is a numerical value taking into consideration similar amino acid in addition to identity into the calculation.

As used herein, "homology" of genes refers to the degree of identity of two or more genetic sequences with one another. In general, having "homology" refers to having a high degree of identity or similarity. Thus, two genes with higher homology have higher identity or similarity of sequences. It is possible to find whether two types of genes have homology by direct comparison of sequences, or by hybridization under stringent conditions for nucleic acids. When two genetic sequences are directly compared, the genes are homologous when DNA sequences are representatively at least 50% identical, preferably at least 70% identical, and more preferably at least 80%, 90%, 95%, 96%, 97%, 98%, or 99% identical between the genetic sequences. Thus, as used herein, "homolog" or "homologous gene product" refers to a protein in another species, preferably mammal, exerting the same biological function as a protein constituent of a complex which will be further described herein.

As used herein, a "purified" substance or biological agent (e.g., nucleic acid, protein, or the like) refers to a substance or a biological agent from which at least a part of an agent naturally associated with the biological agent has been removed. Thus, the purity of a biological agent in a purified biological agent is generally higher than the purity in the normal state of the biological agent (i.e., concentrated). The term "purified" as used herein refers to the presence of preferably at least 75% by weight, more preferably at least 85% by weight, still more preferably at least. 95% by weight, and most preferably at least 98% by weight of the same type of biological agent. A substance used in the present invention is preferably a "purified" substance. As used herein, "isolation" refers to removing at least one of any accompanying substance in a naturally-occurring state. For example, extraction of a specific genetic sequence from a genomic sequence can also be referred to as isolation.

As used herein, a "marker (substance, protein, or gene (nucleic acid))" refers to a substance which serves as an indicator for tracking whether a subject is in a certain state (e.g., the level or presence of a normal cell state, a transformed state, a disease state, a disorder state, a proliferation ability, or a differentiated state), or whether there is risk thereof. Examples of such a marker include genes (nucleic acid = DNA level), gene products (mRNA, protein, and the like), metabolites, enzymes, and the like. In the present invention, detection, diagnosis, preliminary detection, prediction, or advance diagnosis of a certain state (e.g., a disease such as differentiation disorder) can be materialized using an agent or means specific to a marker associated with the state, or a composition, a kit, a system or the like comprising them. As used herein, "gene product" refers to mRNA or a protein encoded by a gene.

As used herein, "subject" refers to an entity which is to be subjected to diagnosis, detection, or the like in the present invention (e.g., an organism such as a human, an organ or a cell which has been taken out from an organism, or the like).

As used herein, a "sample" refers to any substance obtained from a subject or the like, and includes, for example, a cell or the like. Those skilled in the art can appropriately select a preferable sample based on the descriptions herein.

As used herein, an "agent" is used in a broad sense, and may be any substance or other elements (e.g., energy such as light, radiation, heat, and electricity) as long as the intended object can be attained. Examples of such a substance include, but are not limited to, proteins, polypeptides, oligopeptides, peptides, polynucleotides, oligonucleotides, nucleotides, nucleic acids (e.g., including DNA such as cDNA and genomic DNA, and RNA such as mRNA), polysaccharides, oligosaccharides, lipids, organic small molecules (e.g., hormones, ligands, information transmitting substances, organic small molecules, molecules synthesized by combinatorial chemistry, small molecules which can be utilized as a medicine (e.g., a low molecular weight ligand), and the like), and composite molecules thereof. Representative examples of an agent specific to a polynucleotide include, but are not limited to, a polynucleotide having complementarity with certain sequence homology (e.g., 70% or more sequence identity) relative to the sequence of the polynucleotide, a polypeptide such as a transcription factor binding to a promoter region, and the like. Representative examples of an agent specific to a polypeptide include, but are not limited to, an antibody specifically directed to the polypeptide or a derivative or an analog thereof (e.g., single chain antibody), a specific ligand or receptor when the polypeptide is a receptor or a ligand, a substrate when the polypeptide is an enzyme, and the like.

As used herein, a "detection agent" in a broad sense refers to any agent capable of detecting a subject of interest.

As used herein, a "diagnostic agent" in a broad sense refers to any agent with which a state of interest (e.g., a disease or the like) can be diagnosed.

The detection agent of the invention may be a complex or a composite molecule in which another substance (e.g., label or the like) is bound to a portion enabled to be detected (e.g., antibody or the like). As used herein, a "complex" or a "composite molecule" refers to any construct comprising two or more parts. For example, when one of the parts is a polypeptide, the other part may be a polypeptide or another substance (e.g., a sugar, a lipid, a nucleic acid, a different hydrocarbon, or the like). As used herein, two or more parts constituting the complex may be bound by a covalent bond or another bond (e.g., a hydrogen bond, ionic bond, hydrophobic interaction, Van der Waals force, or the like). When the two or more parts are polypeptides, this can also be called a chimeric polypeptide. Thus, as used herein, a "complex" encompasses molecules obtained by connecting a plurality of kinds of molecules such as a polypeptide, a polynucleotide, a lipid, a sugar, and a small molecule.

As used herein, "interaction", in the context of two substances, refers to a force (e.g., intermolecular force (Van der Waals force), a hydrogen bond, hydrophobic interaction, or the like) being exerted between a substance and the other substance. Generally, the two interacting substances are in an associated or a bound state.

The term "bond" as used herein refers to physical interaction or chemical interaction between two substances or between combinations thereof. The bond includes an ionic bond, a non-ionic bond, a hydrogen bond, a Van der Waals bond, hydrophobic interaction, and the like. Physical interaction (bond) can be direct or indirect, where an indirect bond is formed through or due to the effect of another protein or compound. A direct bond refers to interaction, which is not formed through or due to the effect of another protein or compound and involves substantially no other chemical intermediate. The degree of expression of the marker of the invention or the like can be measured by measuring a bond or interaction.

Thus, as used herein, an "agent" (or a detection agent or the like) which "specifically" interacts with (or binds to) a biological agent such as a polynucleotide or a polypeptide includes an agent whose affinity to the biological agent such as a polynucleotide or a polypeptide is typically equal to or higher than, preferably significantly (e.g., statistically significantly) higher than the affinity to other unrelated polynucleotide or polypeptide (particularly those with less than 30% identity). Such affinity can be measured, for example, by a hybridization assay, a binding assay, or the like.

As used herein, a first substance or agent "specifically" interacting with (or binding to) a second substance or agent refers to the first substance or agent interacting with (or binding to) the second substance or agent with higher affinity than that to a substance or agent other than the second substance or agent (particularly another substance or agent that is present in a sample containing the second substance or agent). Examples of interaction (or bond) specific to a substance or an agent include, but are not limited to, a ligand-receptor reaction, hybridization in nucleic acids, an antigen-antibody reaction in proteins, an enzyme-substrate reaction, and when both a nucleic acid and a protein are involved, a reaction between a transcription factor and a binding site of the transcription factor and the like, protein-lipid interaction, nucleic acid-lipid interaction, and the like.. Thus, when both of the substances or agents are nucleic acids, a first substance or agent "specifically interacting" with a second substance or agent encompasses the first substance or agent having complementarity to at least a part of the second substance or agent. For example, when both of the substances or agents are proteins, examples of "specific" interaction (or bond) of a first substance or agent with a second substance or agent include, but are not limited to, interaction by an antigen-antibody reaction, interaction by a receptor-ligand reaction, enzyme-substrate interaction, and the like. When two kinds of substances or agents include a protein and a nucleic acid, "specific" interaction (or bond) of a first substance or agent with a second substance or agent encompasses interaction (or bond) between a transcription factor and a binding region of a nucleic acid molecule which is a target of the transcription factor.

As used herein, "detection" or "quantification" of polynucleotide or polypeptide expression can be attained, for example, by using an appropriate method including mRNA measurement and an immunological measuring method, which includes binding or interaction with a marker detection agent. This can be measured in the present invention with the amount of PCR product. Examples of a molecular biological measuring method include Northern blotting, dot blotting, PCR, and the like. Examples of an immunological measuring method include, as a method, an ELISA using a microtiter plate, RIA, a fluorescent antibody method, luminescence immunoassay (LIA), immunoprecipitation (IP), single radical immuno-diffusion (SRID), turbidimetric immunoassay (TIA), Western blotting, an immunohistological staining method, and the like. Further, examples of a quantification method include ELISA, RIA, and the like. Detection or quantitation can also be performed by a genetic analysis method using an array (e.g., DNA array or protein array). The DNA array is extensively reviewed in "Saibo Kogaku Bessatsu "DNA maikuroarei to saishin PCR method" [Cell Technology, separate volume, "DNA Microarray and Advanced PCR method], edited by Shujunsha Co., Ltd.). A protein array is described in detail in Nat Genet. 2002 Dec; 32 Suppl: 526-32. Examples of a method for analyzing gene expression include, but are not limited to, RT-PCR, RACE, SSCP, immunoprecipitation, a two-hybrid system, in vitro translation, and the like in addition to the aforementioned methods. Such additional analysis methods are described, for example, in Genomu Kaiseki Jikkenho/Nakamura Yusuke Labo/Manyuaru [Genome Analysis Experimental Method, Nakamura Yusuke Lab. Manual], edited by Yusuke Nakamura, Yodosha Co., Ltd. (2002) and the like. The entire descriptions therein are incorporated herein by reference.

As used herein, "means" refers to anything which can serve as a tool for attaining a certain objective (e.g., detection, diagnosis, or therapy). As used herein, "means for selective recognition (detection)" especially refers to means which can recognize (detect) a certain subject differently from others.

A result detected by the present invention is useful as an indicator of a state of an immune system. Accordingly, the present invention can be used to identify an indicator of a state of an immune system to find the state of a disease.

As used herein, "diagnosis" refers to identification of a variety of parameters associated with a disease, disorder, condition, or the like in a subject to judge the current or future status of such a disease, a disorder, a condition, or the like. By using the method, the apparatus, or the system of the present invention, the condition in the body can be examined. A variety of parameters such as a disease, a disorder, or a condition in a subject, a formulation or a method for the treatment or prevention to be administered can be selected using such information. As used herein, in a narrow sense, "diagnosis" refers to diagnosis of the current status, while encompassing "early diagnosis", "presumptive diagnosis", "advance diagnosis", and the like in a broad sense. Since the diagnosis method of the invention, in principle, can utilize what has come from a body and can be implemented without a healthcare professional such as a doctor, the method is industrially useful. As used herein, "presumptive diagnosis, advance diagnosis, or diagnosis" in particularly may be called "assistance" in order to clarify that the method can be implemented without a healthcare professional such as a doctor.

A procedure of formulating a diagnostic agent or the like of the invention as a drug or the like is known in the art and is described, for example, in the Japanese Pharmacopoeia, U.S. Pharmacopoeia, and other countries' Pharmacopoeias. Thus, those skilled in the art can determine the amount to be used from the descriptions herein without undue experimentation.

### (Description of preferred embodiments>

Preferred embodiments of the present invention are described below. Embodiments described below are provided to facilitate the understanding of the present invention. It is understood that the scope of the present invention should not be limited to the following descriptions. Thus, it is apparent that those skilled in the art can make appropriate modifications within the scope of the present invention by referring to the descriptions herein. Those skilled in the art can appropriately combine any of the embodiments.

### (Binding mode clustering technology)

In one aspect, the present invention provides a method of analyzing antigen specificity or binding mode of immunological entities, comprising the steps of: (i) providing a feature (e.g., sequence information) of at least two immunological entities; (ii) subjecting analysis of the antigen specificity or binding mode of the immunological entities to machine learning based on the feature; and (iii) classifying the antigen specificity or binding mode or determining whether the antigen specificity or binding mode is the same/different.

In one embodiment, the present invention provides a method of analyzing antigen specificity or binding mode of immunological entities, comprising the steps of: (i) providing a feature (e.g., sequence information) of at least two immunological entities; (ii) subjecting analysis of the antigen specificity or binding mode (e.g., "epitope") of the immunological entities to machine learning without specifying antigen specificity or binding mode based on the feature; and (iii) classifying the antigen specificity or binding mode or determining whether the antigen specificity or binding mode is the same/different.

In one embodiment, the present invention relates to a method of evaluating immunological entities in pairs. In this embodiment, the present invention provides a method of analyzing a collection of immunological entities, the method comprising the steps of: (a) extracting a feature for at least a pair of members of the collection of immunological entities; (b) computing a distance between antigen specificities or binding modes or judging whether the antigen specificities or binding modes match for the pair by machine learning using the feature; (c) clustering the collection of immunological entities based on the distance; and (d) optionally analyzing based on a classification by the clustering. Calculation of a feature from a three-dimensional structural model can be excluded from the calculation of the feature.

The following is a representative method of computing "distance" in the present invention. First, learning data is constructed from existing experimental data. Learning data typically includes amino acid sequence information and label information on a pair of immunological entities (information on whether the pair has the same epitope/binding mode, or whether the pair binds to the same antigen molecule). Obtained label information can vary depending on the experimental methodology for obtaining learning data. For example, X-ray crystal structure analysis obtains molecule binding information at an atomic level, so that information on the binding mode is obtained. Next, this experimental data is used for leaning, typically with data for the same epitope/binding mode/antigen as 1 and for different epitope/binding mode/antigen as 0 by machine learning. As a result of learning, machine learning returns the probability of a given immunological entity pair binding to the same binding mode/epitope/antigen. This probability is the distance. The present invention can also use other methodologies that are similar to the exemplified methodology for the computation.

As for the handling of the "feature" in the prediction by machine learning in this embodiment, the feature is used as an input of a machine learning algorithm as a feature vector. The present invention can analyze with either antigen specificity or binding mode. Antigen specificity is a biological definition, and binding mode is a physical definition, referring to substantially the same subject. It is the physical binding mode that is grouped in the prediction method of the invention, where the binding mode can be analyzed unambiguously, but antigen specificity that can comprise a plurality of binding modes can also be analyzed as a result.

In the embodiment of the invention, clustering can be computed based on the distance. For example, the distance is computed in step b) for evaluation by pairs, unlike overall evaluation. Meanwhile, when judging whether antigen specificity or binding mode matches, the distance of antigen specificity or binding mode refers to judgment on whether antigen specificity or binding mode matches. Specifically, in one embodiment, if the "distance" is predicted by 0 or 1, clustering would be a simple operation of grouping 1. Meanwhile, if the distance is expressed as [0-1] in another embodiment, the advantage of clustering is not a just the distance (relationship of a pair). Other parameters such as the density of pairs in the surrounding can also be considered.

Examples of methodology of analysis that can be used in the embodiments of the invention include deeming each cluster as a gene from clustering results and using the clusters in a similar manner to gene expression analysis. Specific examples include: 1. When following a chronological change, the increase/decrease in sequences belonging to a specific or a plurality of clusters is observed. The number of increased/decreased clusters is observed. The feature (V/D/J gene, length of CDRs, hydrophilicity, hydrophobicity, conserved residue, or the like) of each cluster is found. 2. When interested in a specific layer of a plurality of specimens, a cluster that is predominantly present or increasing/decreasing in a specific layer is identified. The number of increased/decreased clusters is observed. The feature of each cluster is found. 3. When interested in a function (function = antigen specificity or binding mode), focus is placed on a cluster comprising a sequence specific to an antigen of interest (assumed to be obtained by another experiment such as an ELISPOT assay or sorting with a pMHC tetramer) to observe the increase/decrease thereof. (Function = function of a cell) Results of clustering obtained from cells of different subtypes that are separately sorted and sequenced are compared. 4. When comparing with another experiment source, gene expression analysis, omics analysis, correlation with bacterial flora, cytokines, number of cell species, or analysis from 1 to 3 combined therewith can be used.

A "pairwise" embodiment computes a feature vector for each pair, and a "whole" embodiment described elsewhere herein computes a feature vector for each sequence.

Therefore, in one embodiment performed by pairs, "extracting a feature for at least a pair of members of the collection of immunological entities" is typically performed as follows. Specifically, genetic information and region information of each sequence is initially obtained; next, the sequences are divided in regions such as CDRs and frameworks; a feature of the whole or each sequence for each region is obtained; match in the feature of each sequence or the difference in the degree of match is found as a feature of a pair; and lastly, features obtained by the series of operations are grouped as one feature vector to extract a feature. For a pairwise embodiment, one feature vector can be computed for each pair.

In one embodiment performed as a whole, "extracting a feature for each sequence constituting at least a pair of members of the collection of immunological entities" is typically performed as follows. First, genetic information and region information of each sequence is obtained; next, the sequences are divided in regions such as CDRs and frameworks; a feature of the whole or each sequence for each region is obtained; and lastly, features obtained by the series of operations is grouped as one feature vector to extract a feature. For an embodiment performed as a whole, a feature vector for the whole can be extracted by computing a feature vector for each sequence and totaling the vectors.

In another embodiment, the present invention provides calculation by the machine learning in a method of analyzing by pairs.

In one embodiment performed in pairs, "computing a distance between antigen specificities or binding modes for the pair by machine learning using the feature" is typically performed as follows. For example, the distance of the pair is computed from a feature (e.g., numerical value extracted in (a)) using a methodology such as random forest or boosting.

In one embodiment performed in pairs, "judgment" of "whether the specificities or binding modes match" can be performed by any methodology such as judgment based on a threshold value (e.g., an appropriate numerical value such as 0.5 or 0.6).

Unlike an embodiment performed in pairs, an embodiment performed in a form of the whole typically performs "projecting the feature onto a high dimensional vector space" as follows. Specifically, a technology known as embedding can be used. Embedding learns to dispose high dimensional vectors consisting of each sequence that recognize the same binding mode/epitope/antigen from the learning data close, and vectors that do not are disposed far away by machine learning. A high dimensional vector space is selected to enable such an arrangement by machine learning.

In an embodiment performed in a form of the whole, unlike an embodiment performed in pairs, "a distance on the space between the members reflects functional similarity of the members" refers to the following. Specifically, members that recognize the same binding mode/epitope/antigen from the learning data close, and members that do not are disposed far apart by machine learning. Therefore, reflection of functional similarity means that a sequence at a closer distance is expected to have a similar function. This step can be performed based on a simple threshold value based on the binding distance, or by hierarchical clustering, non-hierarchical clustering, or a combination thereof.

The same clustering can be used in an embodiment performed in pairs and an embodiment performed as a while. "Clustering the collection of immunological entities based on the distance" typically uses, for example, a method based on a simple threshold value based on the distance, a hierarchical clustering method, a non-hierarchical clustering method, or a combination thereof. Optical clustering parameters can be used to obtain a desired result in accordance with the type of correct answer label of a learning set (binding mode/epitope/antigen) and the objective such as clustering results minimizing false-positive of a learning set, maximization of the Rand Index/Matthews correlation coefficient (MCC), or maximization of the Rand Index/MCC while maintaining false positive to less than a certain ratio.

Specific examples of the step of analyzing based on classification by clustering in an embodiment that can be performed in either as a whole or in pairs include deeming each cluster as a gene from clustering results and using the clusters in a similar manner to gene expression analysis. Specific examples include, but are not limited to: 1. When following a chronological change, the increase/decrease in sequences belonging to a specific or a plurality of clusters is observed. The number of increased/decreased clusters is observed. The feature (V/D/J gene, length of CDRs, hydrophilicity, hydrophobicity, conserved residue, or the like) of each cluster is found. 2. When interested in a specific layer of a plurality of specimens, a cluster that is predominantly present or increasing/decreasing in a specific layer is identified. The number of increased/decreased clusters is observed. The feature of each cluster is found. 3. When interested in a function (function = antigen specificity or binding mode), focus is placed on a cluster comprising a sequence specific to an antigen of interest (assumed to be obtained by another experiment such as an ELISPOT assay or sorting with a pMHC tetramer) to observe the increase/decrease thereof. (Function = function of a cell) Results of clustering obtained from cells of different subtypes that are separately sorted and sequenced are compared. 4. When comparing with another experiment source, gene expression analysis, omics analysis, correlation with bacterial flora, cytokines, number of cell species, or analysis from 1 to 3 combined therewith can be used. In a specific embodiment, typical examples of "analysis" performed "based on a classification by the clustering" include, but are not limited to, analysis comprising one or more of identification of a biomarker and identification of an immunological entity that is a therapeutic target or a cell comprising the immunological entity. For example, statistical evaluation of the presence/absence of expression, expression level, or difference in the expression pattern of an immunological entity belonging to a cluster or a group of clusters specific to a specimen or a group of specimens of interest derived from a patient or the like can identify an immunological entity that should be a target as a biomarker for predicting the presence/absence of a disease, diagnosis, prognosis, possibility of recurrence, severity, vaccine efficacy, or the like, or for a search for a pathogenic immunological entity that would be a therapeutic target of an autoimmune disease or the like or a cell expressing the same, or development of cell therapy or vaccine.

If, for example, evaluation as a whole is performed with an auto-encoder used in deep learning in the embodiment of the invention, the sequence of an immunological entity itself is projected onto a high dimensional vector space as an input. An auto-encoder itself extracts and projects a feature onto a high dimensional vector space. In such a case, the extracted feature directly becomes a high dimensional vector space element.

In one embodiment, the immunological entities are antibodies, antigen binding fragments of an antibody, B cell receptors, fragments of a B cell receptor, T cell receptors, fragments of a T cell receptor, chimeric antigen receptors (CARs), or cells comprising one or more of them.

In one embodiment, the present invention provides analysis of a collection of immunological entities by evaluation as a whole. In an embodiment thereof, the present invention provides a method of analyzing a collection of immunological entities, the method comprising the steps of: (aa) extracting a feature for each sequence constituting at least a pair of members of the collection of immunological entities; (bb) projecting the feature onto a high dimensional vector space, wherein a distance on the space between the members reflects functional similarity of the members; (cc) clustering the collection of immunological entities based on the distance; and (dd) optionally analyzing based on a classification by the clustering. Calculation of a feature from a three-dimensional structural model can be excluded from the calculation of the feature.

In one embodiment performed as a whole, the step of extracting a feature for each sequence constituting at least a pair of members of the collection of immunological entities can be performed in the same manner as an embodiment performed in pairs. Examples thereof include providing a feature with an auto-encoder and the like.

In one embodiment performed as a whole, the step of projecting the feature onto a high dimensional vector space, wherein a distance on the space between the members reflects functional similarity of the members can be performed in the same manner as an embodiment performed in pairs. In this manner, clustering after obtaining the distance on the space and thereafter can be performed in the same manner, either in an embodiment performed in pairs or as a whole.

In one exemplary embodiment, the high dimensional vector space calculation (b), when performed as a whole, can be performed by, but is not limited to, a supervised, semi-supervised (Siamese network), or unsupervised (Auto-encoder) method.

In one embodiment, the step of clustering the collection of immunological entities based on the distance can be specifically performed, for example, based on a simple threshold value based on a distance on a high dimensional space, or by a hierarchical clustering method, a non-hierarchical clustering method, or a combination thereof. This can be performed through processing with various procedures such as using optimal clustering parameters to obtain a desired result in accordance with the type of correct answer label of a learning set (binding mode/epitope/antigen) and the objective such as clustering results minimizing false-positive of a learning set, maximization of the Rand Index/Matthews correlation coefficient (MCC), or maximization of the Rand Index/MCC while maintaining false positive to less than a certain ratio.

In one embodiment, the step of analyzing based on a classification by the clustering is, for example, deeming each cluster as a gene from clustering results and using the clusters in a similar manner to gene expression analysis. Specifically, 1. When following a chronological change, the increase/decrease in sequences belonging to a specific or a plurality of clusters is observed. The number of increased/decreased clusters is observed. The feature (V/D/J gene, length of CDRs, hydrophilicity, hydrophobicity, conserved residue, or the like) of each cluster is found. 2. When interested in a specific layer of a plurality of specimens, a cluster that is predominantly present or increasing/decreasing in a specific layer is identified. The number of increased/decreased clusters is observed. The feature of each cluster is found. 3. When interested in a function (function = antigen specificity or binding mode), focus is placed on a cluster comprising a sequence specific to an antigen of interest (assumed to be obtained by another experiment such as an ELISPOT assay or sorting with a pMHC tetramer) to observe the increase/decrease thereof. (Function = function of a cell) Results of clustering obtained from cells of different subtypes that are separately sorted and sequenced are compared. 4. When comparing with another experiment source, gene expression analysis, omics analysis, correlation with bacterial flora, cytokines, number of cell species, or analysis from 1 to 3 combined therewith can be used.

The description above is merely an example. A more complex function type comprising more terms can also be used to practice the invention.

In one embodiment, the machine learning is selected from the group consisting of machine learning algorithms such as a regressive scheme, a neural network method, support vector machine, and random forest.

In a special case where an immunological entity binder (e.g., antigen) is already known or some of the antibody targets are known, the evaluation step of the invention can include these known cases to clustering as an application. In other words, an immunological entity binder (e.g., antigen)/epitope (antigen specificity or binding mode) of an immunological entity (e.g., antibody) can be predicted by using an immunological entity (e.g., antibody) with a known immunological entity binder (e.g., antigen)/epitope (antigen specificity or binding mode).

Epitopes classified into acluster described herein can be associated with biological information. For example, a carrier of the antibody can be associated with a known disease, disorder, or biological condition based on one or more clusters of epitopes identified based on the classification method of the invention.

Examples of diseases, disorders, or biological conditions that can be involved in the present invention include infections by a foreign object (e.g., bacteria, virus, or the like), as well as self entities recognized as non-self (e.g., neoplasm (cancer or tumor) and entities associated with autoimmune diseases). An immune system functions to distinguish molecules endogenous to an organism ("self" molecule) from substances exogenous or foreign to the organism ("non-self molecule"). The immune system has two types of adaptive responses (humoral response and cell-mediated response) to a foreign object based on the constituent component mediating the response. A humoral response is mediated by an antibody, while cellular immunity involves cells classified as lymphocytes. In recent anticancer and antiviral strategies, use of the host immune system as means of anticancer or antiviral treatment of therapy is an important strategy. The classification or clustering technologies of the invention can also be applied in both humoral response and cellular response strategies.

The immune system functions through three stages (recognition, activation, and effector) in defending the host from a foreign object. In the recognition stage, the immune system recognizes the presence of an exogenous antigen or an intruder in the body and notifies its presence. An exogenous antigen can be, for example, a foreign object (cell surface marker from a viral protein or the like), a cell surface marker of a cell (cancer cell) that can be recognized as non-self, or the like. When the immune system recognizes an intruder, antigen-specific cells of the immune system proliferate and differentiate in response to an intruder-induced signal (activation stage). The final stage is the effector stage for the effector cells of the immune system to neutralize the detected intruder in response thereto. Effector cells play the role of carrying out an immune response. Examples of effector cells include B cells, T cells, natural killer (NK) cells, and the like. B cells produce an antibody against an intruder, and the antibody, in combination with a complement system, guides the cell or organism comprising or associated with a specific target immunological entity, epitope, antigen specificity, or binding mode (including immunological entity binders such as an antigen) to its destruction. T cells are categorized into types such as helper T cells, regulatory T cells, and cytotoxic T cells (CTL cells). Helper T cells secrete a cytokine and stimulate the growth of other cells or the like to enhance the efficacy of an immune response. Regulatory T cells downregulate an immune response. CTL cells directly dissolves/melts and destroys cells presenting an exogenous antigen on the surface. NK cells are understood to recognize and destroy virally infected cells, malignant tumor cells, or the like. Therefore, classification of an immunological entity, epitope, antigen specificity, or binding mode targeted by or highly associated with these effector cells and linking the immunological. entity, epitope, antigen specificity, or binding mode to a disease, disorder, or biological condition plays a very important role in the efficacy of therapy or diagnosis.

In this manner, T cells are antigen specific immune cells that function in response to a specific antigen signal. B lymphocytes and antibodies produced thereby are also antigen specific objects. The present invention enables these specific immunological entity binders (e.g., antigen) to be classified and clustered using an immunological entity, epitope, antigen specificity, or binding mode cluster by the final function (association with a specific disease, disorder, or biological condition).

As described above, B cells respond to free or soluble antigens, but T cells do not. For the T cells to response to an antigen, the antigen needs to be processed by a peptide and linked to a presentation structure encoded by a major histocompatibility complex (MHC) (called "MHC restriction"). T cells distinguish self cells from non-self cells by this mechanism. If an antigen is not presented by a recognizable MHC molecule, T cells do not recognize an antigen signal. T cells specific to a peptide bound to a recognizable MHC molecule bind to an MHC peptide complex, and an immune response progresses. MHC has two classes (MHC class I and MHC class II). It is understood that CD4⁺ T cells preferentially interact with MHC class II proteins, while cytotoxic T cells (CD8⁺) preferentially interact with MHC class I. These MHC proteins of both classes are transmembrane proteins comprising the majority of the structure thereof on the external surface of a cell, having a peptide bond space on the outside thereof. Fragments of both endogenous and exogenous proteins are bound and presented to the extracellular environment in this space. At this time, cells known as professional antigen presenting cells (pAPCs) present an antigen to T cells using an MHC protein, and induce a pathway for differentiation and activation of T cells using various specific costimulatory molecules to materialize the effect of the immune system. The classification and clustering technologies for immunological entities, epitopes, immunological entity binders, antigen specificities, or binding modes of the invention provide an applied method that could not be provided with conventional art for the therapy or diagnosis involving such MHCs.

For non-self entities, an applied method for therapy or diagnosiscan be provided by sufficiently utilizing a conventional immune system, but further creativity could be required for self entities. This is because cancer cells and the like have the same origin as normal cells and are substantially the same as normal cells at gene levels. However, cancer cells are known to present tumor associated antigens (TuAA). In addition, the immune system of a subject can be utilized to attack cancer cells by utilizing the antigen or another immunological entity binder. Such tumor associated antigens can also classify and cluster immunological entities, epitopes, antigen specificities, or binding modes to an indicator with the technology of the invention. For example, a tumor associated antigen can be applied to an anticancer vaccine or the like. For example, a conventional technology using the entire activated tumor cell is disclosed in US Patent No. 5,993,828. Alternatively, a technology applying a composition comprising an isolated tumor antigen has been attempted (e.g., Krishnadas DK et al., Cancer Immunol Immunother. 2015 Oct; 64(10): 1251-60). A genetically modified T .cell (also called CAR-T) using a chimeric antigen receptor (CAR) that recognizes an identified epitope can also be used. An immunotherapy using an immune checkpoint inhibitor or the like based on the action related to an immune checkpoint such as PD-1 or PD-L1 has also drawn attention recently. PD-1 binds to a PD-1 ligand (PD-L1 and PD-L2) expressed in an antigen presenting cell and transmits a suppressive signal to lymphocytes to downregulate the activation state of lymphocytes. PD-1 ligands are expressed in various human tumor tissues other than antigen presenting cells. It is understood that there is a negative correlation between the expression of PD-L1 in resected tumor tissue and post-op survival period in malignant melanoma. It is understood that the cytotoxic activity recovers by inhibiting binding of PD-1 and PD-L1 wiht PD-1 antibodies or PD-L1 antibodies. A sustained antitumor effect can be exhibited by activation of antigen specific T cells and enhancement of cytotoxic activity to cancer cells (e.g., nivolumab or the like). The epitope classification or clustering method of the invention can also be applied to the mechanism of restoring the downregulation mechanism of immune activity.

In one embodiment of the invention, the immunological entity, epitope, immunological entity binder, antigen specificity, or binding mode classification or clustering method of the invention can be applied to viral diseases for vaccines. As a vaccine for a virus, attenuated virus, inactivated vaccine, subunit vaccine, and the like are utilized. While the success rate of subunit vaccines is not high, successful examples in a recombinant hepatitis B vaccine based on an envelope protein and the like have been reported. Since a biological condition can be suitably associated using the immunological entity, epitope, immunological entity binder, antigen specificity, or binding mode classification or clustering method of the invention, it is understood that efficacy with a subunit vaccine or the like is also improved. It is also understood that suitable quantitative evaluation of clusters leads to evaluation of efficacy of vaccines. Stratification is also possible by comparison with cases where a vaccine is effective. It is understood that the efficacy is improved or the possibility of distribution in the market is improved as a result. In fact, a result of identifying a cluster reacting to a vaccine in silico using the methodology of the invention has been shown.

In one embodiment, examples of immunological entities that can be used in the immunological entity, epitope, immunological entity binder, antigen specificity, or binding mode classification or clustering method of the invention include an antibody, an antigen binding fragment of an antibody, a B cell receptor, a fragment of a B cell receptor, a T cell receptor, a fragment of a T cell receptor, a chimeric antigen receptor (CAR), a cell comprising one or more of them (e.g., T cell comprising a chimeric antigen receptor (CAR) (CAR-T)), and the like.

In this regard, the present invention provides a method of generating a cluster of immunological entities, epitopes, immunological entity binders, antigen specificities, or binding modes classified based on the methodology of the invention, the method comprising the step of classifying immunological entities binding to the same epitope to the same cluster. In one embodiment, immunological entities, epitopes, or immunological entity binders are evaluated by at least one endpoint selected from the group consisting of a property and similarity with a known immunological entity, epitope, or immunological entity binder thereof to perform the cluster classification targeting an immunological entity, epitope, or immunological entity binder meeting a predetermined baseline. If a plurality of the immunological entities, epitopes, or immunological entity binders are the same, at least a part or all of the three-dimensional structures of the immunological entities, epitopes, immunological entity binders, antigen specificities, or binding modes overlap in some cases. If the plurality of the epitopes are the same, at least a part or all of amino acids associated with the immunological entities, epitopes, immunological entity binders, antigen specificities, or binding modes overlap in some cases.

### <Antigen specificity/binding mode and antigen classification>

In yet another aspect, the present invention provides an immunological entity, epitope, immunological entity binder, antigen specificity, binding mode, an antigen (or a corresponding immunological entity binder) having the antigen specificity or binding mode or a structure based thereon identified by the method of the invention, or a cluster thereof. The immunological entity, epitope, immunological entity binder, antigen specificity, binding mode, antigen, and the like defined in this regard can have any characteristic described in <(Binding mode clustering technology)> herein, or can be an immunological entity, epitope, immunological entity binder, antigen specificity, binding mode, antigen, and the like identified, classified, or clustered by such technologies. In this regard, a method for generating a cluster can include the step of classifying immunological entities binding to the same epitope, immunological entity binder, antigen specificity, or binding mode to the same cluster or the step of classifying epitopes or immunological entity binders binding to the same immunological entity, antigen specificity, or binding mode to the same cluster. In a preferred embodiment, the immunological entities, epitopes, or immunological entity binders can be evaluated by at least one endpoint selected from the group consisting of a property and similarity with a known immunological entity, epitope, or immunological entity binder to perform the cluster classification targeting an immunological entity meeting a predetermined baseline. For example, as the baseline that can be employed therein, three-dimensional structures of the immunological entities, epitopes, immunological entity binders, antigen specificities, or binding modes can at least partially overlap when a plurality of the immunological entities, epitopes, immunological entity binders, antigen specificities, or binding modes are the same, or amino acid sequences or chemical structures of the epitopes or immunological entity binders can at least partially overlap when the antigen specificity of binding mode of a plurality of the immunological entities, epitopes, or immunological entity binders are the same.

As used herein, "corresponding" (immunological entity binder or the like) (to an epitope) refers to an immunological entity binder sufficiently reflecting the structure or a feature of an epitope when a specific epitope is selected. When the epitope is an amino acid sequence, representative examples thereof include antigen peptides, proteins, and the like comprising the sequence. A vaccine comprising them is intended as a representative example.

One embodiment of the invention relates to a classified immunological entity, epitope, immunological entity binder, antigen specificity, or binding mode, or a clustered immunological entity, epitope, immunological entity binder, antigen specificity, or binding mode, and an immunological entity binder (e.g., antigen) or a polypeptide comprising or associated with the immunological entity, epitope, antigen specificity, or binding mode described above.

In this regard, examples of the method for describing (identifying) a classified immunological entity, epitope, immunological entity binder, antigen specificity, or binding mode or clustered immunological entity, epitope, immunological entity binder, antigen specificity, or binding mode include the following. Specifically, a cluster of immunological entities (e.g., antibodies), epitopes, or immunological entity binders identified by the methodology of the invention is understood as recognizing the same partner such as an immunological entity, epitope, or immunological entity binder with high accuracy, so that an immunological entity, epitope, immunological entity binder, antigen specificity, or binding mode recognized by the cluster can be identified by similarity evaluation on an immunological entity (e.g., antibody with a known antigen) with a known epitope or immunological entity binder (e.g., antigen), experimental antigen screening (or screening of another immunological entity binder), more desirably a mutation experiment of an antigen-antibody pair (or another immunological entity-immunological entity binder), NMR chemical shift, crystal structure analysis, identification of an immunological entity, epitope, immunological entity binder, antigen specificity, or binding mode associated with interaction, or functional evaluation by an in vitro or in vivo experiment. Therefore, even if a known immunological entity, epitope, immunological entity binder, antigen specificity, or binding mode and an immunological entity based thereon are provided, those clustered or classified as in the present invention have specific information, and can be used in a specific application and considered as having a specific effect and function. In this regard, a new characteristic that is absent in conventional epitopes or immunological entity binders (e.g., antigens) and immunological entities based thereof is provided, such that technical matter with a novel and significant characteristic is provided.

### <Program, medium, and system configuration>

In one aspect, the present invention provides a program for executing the method of invention. Any characteristic that can be employed herein can be any of the characteristics described in <Binding mode clustering technology> herein or a combination thereof.

In one aspect, the present invention provides, for example, a program for executing a method of analyzing a collection of immunological entities, comprising the steps of: (i) providing a feature of at least two immunological entities; (ii) subjecting analysis of antigen specificity or binding mode of the immunological entities to machine learning without specifying antigen specificity or binding mode based on the feature; and (iii) classifying the antigen specificity or binding mode or determining whether the antigen specificity or binding mode is the same/different.

Alternatively in one aspect, the present invention provides, for example, a program for executing a method of analyzing a collection of immunological entities, the method comprising the steps of:
(a) extracting a feature for at least a pair of members of the collection of immunological entities;
(b) computing a distance between antigen specificities or binding modes or judging whether the antigen specificities or binding modes match for the pair by machine learning using the feature;
(c) clustering the collection of immunological entities based on the distance; and
(d) optionally analyzing based on a classification by the clustering. Calculation of a feature from a three-dimensional structural model can be excluded from the calculation of the feature.

Alternatively in one aspect, the present invention provides, for example, a program for executing a method of analyzing a collection of immunological entities, the method comprising the steps of:
(aa) extracting a feature for each sequence constituting at least a pair of members of the collection of immunological entities;
(bb) projecting the feature onto a high dimensional vector space, wherein a distance on the space between the members reflects functional similarity of the members;
(cc) clustering the collection of immunological entities based on the distance; and
(dd) optionally analyzing based on a classification by the clustering. Calculation of a feature from a three-dimensional structural model can be excluded from the calculation of the feature.

In the program described above, any of the characteristics that can be employed therein can be any of the characteristics described in <Binding mode clustering technology> herein or a combination thereof.

In another aspect, the present invention provides a recording medium storing a program for executing the method of the invention. In one embodiment, the recording medium can be a ROM, HDD, or magnetic disk that can be stored internally, or an external storage apparatus such as flash memory such as a USB memory. Any of the characteristics that can be employed therein can be any of the characteristics described in <Binding mode clustering technology> herein or a combination thereof. The recording medium of the invention can be a recording medium storing the computer program of the invention described above.

In another aspect, the present invention provides a system comprising a program for executing the method of the invention. Any of the characteristics that can be employed therein can be any of the characteristics described in <Binding mode clustering technology> herein or a combination thereof. In one embodiment, the system of the invention provides a system for analyzing a collection of immunological entities, comprising: (I) a feature providing unit for providing a feature of at least two immunological entities; (II) a machine learning unit for subjecting analysis of antigen specificity or binding mode of the immunological entities to machine learning without specifying antigen specificity or binding mode based on the feature; and (III) a classification unit for classifying the antigen specificity or binding mode or determining whether the antigen specificity or binding mode is the same/different. Any of the characteristics that can be employed therein can be any of the characteristics described in <Binding mode clustering technology> herein or a combination thereof. Each of these parts can be materialized by separate constituent elements, or two or more can be materialized with a single constituent element. Calculation of a feature from a three-dimensional structural model can be excluded from the calculation of the feature.

In another embodiment, the present invention provides a system for analyzing a collection of immunological entities, comprising: (A) a feature extraction unit or a feature providing unit for extracting a feature for at least a pair of members of the collection of immunological entities; (B) a judgment unit for computing a distance between antigen specificities or binding modes or judging whether the antigen specificities or binding modes match for the pair by machine learning using the feature; (C) a clustering unit for clustering the collection of immunological entities based on the distance; and (D) an analysis unit for optionally analyzing based on a classification by the clustering. Any of the characteristics that can be employed therein can be any of the characteristics described in <Binding mode clustering technology> herein or a combination thereof. Each of these parts can be materialized by separate constituent elements, or two or more can be materialized with a single constituent element. Calculation of a feature from a three-dimensional structural model can be excluded from the calculation of the feature.

In another aspect, the present invention provides a system for analyzing a collection of immunological entities, the system comprising: (A) a feature extraction unit or a feature providing unit for extracting a feature for each sequence constituting at least a pair of members of the collection of immunological entities; (B') a projection unit for projecting the feature onto a high dimensional vector space, wherein a distance on the space between the members reflects functional similarity of the members; (C) a clustering unit for clustering the collection of immunological entities based on the distance; and (D) analysis unit for optionally analyzing based on a classification by the clustering. Any of the characteristics that can be employed therein can be any of the characteristics described in <Binding mode clustering technology> herein or a combination thereof. Each of these parts can be materialized by separate constituent elements, or two or more can be materialized with a single constituent element. Calculation of a feature from a three-dimensional structural model can be excluded from the calculation of the feature.

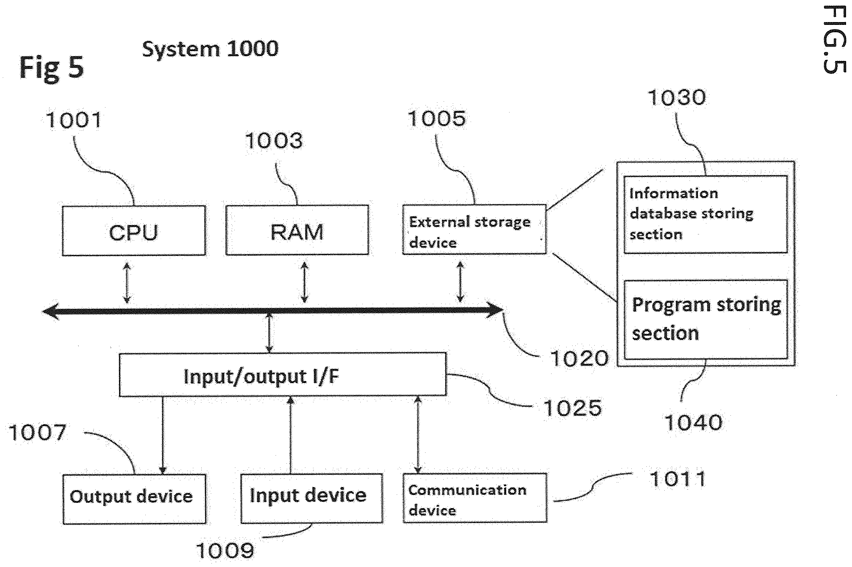

The configuration of system **1000** of the invention is now described with reference to the function block diagram in Figure **5****.** While the figure depicts a case where the invention is materialized with a single system, it is understood that cases where the invention is materialized with a plurality of systems are also encompassed in the scope of the invention.

The system **1000** of the invention is constituted by connecting a RAM **1003,** a ROM or HDD, or a magnetic disk, an external storage device **1005** such as flash memory such as a USB memory, and an input/output interface (I/F) **1025** to a CPU **1001** built into a computer system via a system bus **1020.** An input device **1009** such as a keyboard or a mouse, an output device **1007** such as a display, and a communication device **1011** such as a modem are each connected to the input/output I/F **1025.** The external storage device **1005** comprises an information database storing section **1030** and a program storing section **1040.** Both are a certain storage area secured within the external storage apparatus **1005.**

In such a hardware configuration, various instructions (commands) are inputted via the input device **1009** or commands are received via the communication I/F, communication device **1011,** or the like to call up, deploy, and execute a software program installed on the storage device **1005** on the RAM **1003** by the CPU **1001** to accomplish the function of the invention in cooperation with an OS (operating system). Of course, the present invention can be implemented with a mechanism other than such a cooperating setup.

In the implementation of the present invention, the amino acid sequences or information equivalent thereto (e.g., nucleic acid sequences encoding the same or the like) of immunological entities (which can be antibodies, B cell receptors, T cell receptors, or the like) and other features can be inputted via the input device **1009,** inputted via the communication I/F, communication device **1011,** or the like, or stored in the database storing section **1030.** The steps of subjecting analysis of antigen specificity or binding mode of the immunological entities to machine learning without specifying antigen specificity or binding mode based on the feature; computing a distance between antigen specificities or binding modes or judging whether the antigen specificities or binding modes match for the pair by machine learning using the feature; clustering the collection of immunological entities based on the distance; optionally analyzing based on a classification by the clustering; projecting the feature onto a high dimensional vector space, wherein a distance on the space between the members reflects functional similarity of the members; clustering the collection of immunological entities based on the distance; and optionally analyzing based on a classification by the clustering can be executed with a program stored in the program storing section **1040,** or a software program installed in the external storage device **1005** by inputting various instructions (commands) via the input device **1009** or by receiving commands via the communication I/F, communication device **1011,** or the like. Obtained data or divided data can be outputted through the output device **1007** or stored in the external storage device **1005** such as the information database storing section **1030.** Data can be outputted through the output device **1007** or stored in the external storage device **1005** such as the information database storing section **1030**.

The data or calculation result or information obtained via the communication device **1011** or the like is written and updated immediately in the database storing section **1030.** Information attributed to samples subjected to accumulation can be managed with an ID defined in each master table by managing information such as each of the sequences in each input sequence set and each genetic information ID of a reference database.

The above calculation result can be associated with known information such as a disease, disorder, or biological information and stored in the database storing section **1030.** Such association can be performed directly to data available through a network (Internet, Intranet, or the like) or as a link to the network.

A computer program stored in the program storing section **1040** is configured to use a computer as the processing system described above, i.e., a system for performing calculation or processing such as machine learning, analysis, projection, distance calculation, classification, or division. Each of these functions is an independent computer program, a module thereof, or a routine, which is executed by the CPU **1001** to use a computer as each system or device. It is assumed hereinafter that each function in each system cooperates to constitute each system.

In one aspect, the present invention provides a method for analyzing an epitope of a subject or a cluster thereof using a database, and/or administering diagnosis or therapy based on a diagnostic result. This method and methods comprising one or more additional characteristics described herein are called "efficient clustering of the immunological entities of the invention" herein. A system materializing the repertoire analysis method of the invention is also called "system for analyzing efficient clustering of the immunological entities of the invention".

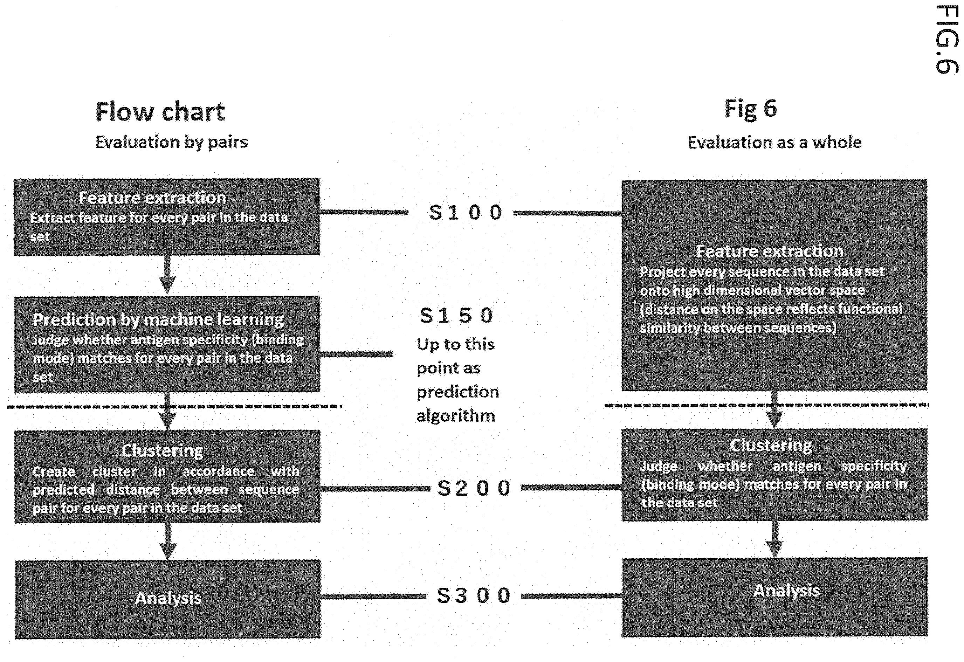

Figure **5** shows a system for efficient clustering of the immunological entities of the invention. Figure **6** shows an example of a system for analyzing efficient clustering of the immunological entities of the invention, which is the specific algorithm thereof.

In Figure **6****,** a feature is provided or extracted at **S100** (step (1)). When performed in pairs, a feature is extracted for every pair in a data set. When performed as a whole, every sequences in a data set is projected onto a high dimensional vector space (the distance in this space reflects the functional similarity between sequences).

At **S150** (step (1A)), prediction is performed using machine learning when performed in pairs. In this regard, every pair in the data set is judged as to whether antigen specificity (binding mode) matches.

At **S200** (step (2)), clustering is performed. When evaluating in pairs, a cluster is created in accordance with the distance between predicted sequence pairs for every pair in the data set. For the whole, clustering judges whether antigen specificity (binding mode) matches for every pair in the data set.

At **S300** (step (3)), analysis is performed.

Provided data can be data stored in the external storage device **1005,** but can be generally obtained as a publicly available database through the communication device **1011.** Alternatively, this can be inputted using the input device **1009** and recorded in the RAM **1003** or external storage device **1005** as needed. A database comprising sequence information of an immunological entity or other features is provided herein. Sequence information or other features can also be obtained by determining the sequence of an actually obtained sample. Sequence information can be obtained by isolating RNA or DNA from tumor and healthy tissue, and poly A+ RNA from each tissue, to prepare cDNA, and sequencing the cDNA using a standard primer. Such a technology is well known in the art. Full or partial sequencing of the genome of a patient is also well known in the art. High throughput DNA sequencing methods are known in the art, including, for example, systems of the MiSeq™ series using the Illumina® sequencing technology. This uses a large scale parallel SBS methodology to generate a high quality DNA sequence with several billion bases in one process. Alternatively, an amino acid sequence of an antibody can be determined by mass spectrometry. The portion materializing **S100** in the system of the invention is also called a feature providing unit.

### <Composition, therapy, diagnosis, drug, and the like>

The present invention also comprises, as an embodiment, the aforementioned classified or clustered immunological entity, epitope, polypeptide, immunological entity binder (e.g., antigen; antigen includes peptides comprising an epitope and the like, as well as those comprising a post-translational modification of glycan or the like, nucleic acids such as DNA/RNA, and lower molecule) and polypeptide having substantial similarity to an immunological entity or immunological entity binder or cluster or associated with antigen specificity or binding mode belonging to the same cluster. Another preferred embodiment comprises a polypeptide having functional similarity to one of the above. In still another embodiment, the present invention comprises a nucleic acid encoding the aforementioned classified or clustered epitope, polypeptide, immunological entity binder (e.g., antigen), or cluster, and a polypeptide having substantial similarity thereto or polypeptide associated with antigen specificity or binding mode belonging to the same cluster. Any of the characteristics that can be employed therein can be any of the characteristics described in <Binding mode clustering technology> herein or a combination thereof, or any characteristic identified, classified, or clustered by said technology.

In one embodiment, an immunological entity, epitope, or immunological entity binder which is a polypeptide, an immunological entity, epitope, or immunological entity binder comprising antigen specificity or binding mode, a cluster, or polypeptide comprising the same of the invention can have affinity to an HLA-A2 molecule. Affinity can be determined by a binding assay, epitope recognition limit assay, prediction algorithm, or the like. The epitope, cluster, or polypeptide comprising the same can have affinity to an HLA-B7 molecule, HLA-B51 molecule, or the like.

In another embodiment of the invention, the present invention provides a pharmaceutical composition comprising a polypeptide, including an immunological entity, epitope, or immunological entity binder that has been classified or clustered in the present invention, an immunological entity, epitope, or immunological entity binder comprising antigen specificity or binding mode, a cluster or polypeptide comprising or associated with the same, and a pharmaceutically acceptable adjuvant, carrier, diluent, excipient, or the like. An adjuvant can be a polynucleotide. A polynucleotide can comprise a dinucleotide. An adjuvant can be encoded by a polynucleotide. An adjuvant can be a cytokine.

In still another embodiment, the present invention relates to a pharmaceutical composition comprising one of the nucleic acids described herein including a nucleic acid encoding a polypeptide comprising an immunological entity, epitope, antigen specificity, binding mode, or immunological entity binder (e.g., antigen) that has been classified or clustered in the present invention. Said composition can comprise a pharmaceutically acceptable adjuvant, carrier, diluent, excipient, or the like.

In still another embodiment, the present invention relates to an isolated and/or purified antibody, an antigen binding fragment, or another immunological entity (e.g., a B cell receptor, a fragment of a B cell receptor, a T cell receptor, a fragment of a T cell receptor, a chimeric antigen receptor (CAR), or a cell comprising one or more of them) specifically binding to at least one immunological entity, epitope, or immunological entity binder that has been classified or clustered in the present invention or having antigen specificity or binding mode belonging to the same cluster. In another embodiment, the present invention relates to an isolated and/or purified antibody or another immunological entity specifically binding to a peptide-MHC protein complex specifically binding to at least one of immunological entity and epitope that has been classified or clustered in the present invention, having antigen specificity or binding mode belonging to the same cluster, or comprising any other suitable epitope. An antibody of any of the embodiments can be a monoclonal antibody or a polyclonal antibody. These compositions can comprise a pharmaceutically acceptable adjuvant, carrier, diluent, excipient, or the like.

In still another embodiment, the present invention relates to a T cell receptor (TCR) and/or B cell receptor (BCR) specifically interacting with at least one of immunological entity, epitope, and immunological entity binder that has been classified or clustered in the present invention or having antigen specificity or binding mode belonging to the same cluster or a fragment thereof, or an isolated protein molecule comprising a binding domain thereof, or TCR and/or BCR repertoire, chimeric antigen receptor (CAR), or a cell comprising one or more of them (e.g., genetically modified T cell comprising a chimeric antigen receptor (CAR) (also referred to as CAR-T cell), or the like) or another immunological entity. In another embodiment, the present invention relates to an isolated and/or purified antibody or another immunological entity specifically binding to a peptide-MHC protein complex comprising an epitope that has been classified or clustered in the present invention or any other suitable epitope. These compositions can comprise a pharmaceutically acceptable adjuvant, carrier, diluent, excipient, or the like.

In still another aspect, the present invention provides a method of identifying a disease, disorder, or a biological condition, comprising the step of associating a carrier of the immunological entity with a known disease, disorder, or biological condition based on a cluster generated by the method of the invention. Alternatively in another aspect, the present invention provides a method of identifying a disease, disorder, or biological condition, comprising the step of evaluating, by using one or more clusters generated by the method of the invention, a disease, disorder or biological condition of a carrier of the cluster. Any of the characteristics that can be employed therein can be any of the characteristics described in <Binding mode clustering technology> herein or a combination thereof, or any characteristic identified, classified, or clustered by said technology. In this regard, the evaluating can use, but is not limited to, at least one indicator selected from analysis based on a ranking of quantity or a ratio of abundance of the plurality of clusters, analysis studying a certain number of B cells and quantifying whether there is a cell/cluster similar to a BCR of interest thereamong, and the like. In still another embodiment, the evaluation is performed using an indicator other than the cluster (e.g., a disease associated gene, a polymorphism of a disease associated gene, an expression profile of a disease associated gene, epigenetics analysis, a combination of TCR and BCR clusters, and the like). By using the present invention, specifically a disease specific gene that is important in the immune system (HLA allele or the like), a polymorphism of a disease associated gene or an expression profile of the gene (RNA-seq or the like), or epigenetics analysis (methylation analysis or the like) can be combined.

In one embodiment, identification of the disease, disorder, or biological condition identifiable by the present invention can be diagnosis, prognosis, pharmacodynamics, and prediction of the disease, disorder, or biological condition, determination of an alternative method, identification of a patient group, safety evaluation, toxicological evaluation, monitoring thereof, or the like..

In another aspect, the present invention provides a method for evaluating a biomarker, comprising the step of evaluating the biomarker used as an indicator of a disease, disorder, or biological condition using one or more epitopes or immunological entity binders identified or classified, or clusters refined, by the present invention. Alternatively, the present invention provides a method for identifying a biomarker, comprising the step of using one or more epitopes identified or classified, or clusters refined, by the present invention to determine the biomarker associated with a disease, disorder, or biological conditions. In this regard, the following methodology can be used for the method for identifying a biomarker. For example, the presence, size, share, or the like of a cluster of interest of B cell repertoire read by a sequencer can be identified and used as a marker.

In still another embodiment, the present invention relates to a host cell expressing a recombinant construct described herein comprising a construct encoding a polypeptide specifically interacting with at least one of an immunological entity, epitope, and an immunological entity binder that has been classified or clustered in the present invention or having antigen specificity or binding mode belonging to the same cluster. A host cell can be a dendritic cell, macrophage, tumor cell, tumor derived cell, bacteria, fungus, protozoa, or the like. This embodiment also provides a pharmaceutical composition comprising such a host cell and a pharmaceutically acceptable adjuvant, carrier, diluent, excipient, or the like.

In another aspect, the present invention provides a composition for identifying the biological information, comprising an immunological entity, epitope, or immunological entity binder identified based on the present invention, or an antigen or an immunological entity binder comprising the same or having antigen specificity or binding mode belonging to the same cluster. Alternatively, the present invention provides a composition for diagnosing the disease, disorder, or biological condition, comprising an immunological entity, epitope, or immunological entity binder identified based on the present invention, or an antigen or an immunological entity binder comprising the same or having antigen specificity or binding mode belonging to the same cluster. Any of the characteristics that can be employed therein can be any of the characteristics described in <Binding mode clustering technology> herein or a combination thereof, or any characteristic identified, classified, or clustered by said technology.

In another aspect, the present invention provides a composition for diagnosing the disease, disorder, or biological condition, comprising a substance targeting an immunological entity to an epitope or immunological entity binder identified based on the present invention. Alternatively, the present invention provides a composition for diagnosing the disease, disorder, or biological condition, comprising an immunological entity, epitope, or immunological entity binder identified based on the present invention, or an immunological entity, epitope, or immunological entity binder such as an antigen comprising the same or having antigen specificity or binding mode belonging to the same cluster. Any of the characteristics that can be employed therein can be any of the characteristics described in <Binding mode clustering technology> herein or a combination thereof, or any characteristic identified, classified, or clustered by said technology. Therefore, examples of the immunological entity include an antibody, an antigen binding fragment of an antibody, a T cell receptor, a fragment of a T cell receptor, a B cell receptor, a fragment of a B cell receptor, a chimeric antigen receptor (CAR), a cell comprising one or more of them (e.g., T cell comprising a chimeric antigen receptor (CAR)), and the like.

In still another embodiment, the present invention provides a composition for treating or preventing a disease, disorder, or biological condition, comprising an immunological entity, epitope, or immunological entity binder identified based on the present invention, or an immunological entity comprising the same or having antigen specificity or binding mode belonging to the same cluster. Any of the characteristics that can be employed therein can be any of the characteristics described in <Binding mode clustering technology> herein or a combination thereof, or any characteristic identified, classified, or clustered by said technology. Further, immunological entities that can be used include, but are not limited to, an antibody, an antigen binding fragment, a chimeric antigen receptor (CAR), a T cell comprising a chimeric antigen receptor (CAR), and the like.

In another aspect, the present invention provides a composition for treating or preventing a disease, disorder, or biological condition, comprising a substance targeting an an immunological entity, epitope, or immunological entity binder identified based on the present invention, or an immunological entity comprising the same or having antigen specificity or binding mode belonging to the same cluster. Any of the characteristics that can be employed therein can be any of the characteristics described in <Binding mode clustering technology> herein or a combination thereof, or any characteristic identified, classified, or clustered by said technology. Examples of the substance that can be used include, but are not limited to, a peptide, polypeptide, protein, nucleic acid, sugar, lower molecule, macromolecule, metal ion, and a complex thereof.

In another aspect, the present invention provides a composition for treating or preventing a disease, disorder, or biological condition, comprising an immunological entity, epitope, or immunological entity binder identified based on the present invention, or an immunological entity binder (e.g., antigen) comprising the same or having antigen specificity or binding mode belonging to the same cluster. Any of the characteristics that can be employed therein can be any of the characteristics described in <Binding mode clustering technology> herein or a combination thereof, or any characteristic identified, classified, or clustered by said technology.

In still another embodiment, the present invention relates to a vaccine or an immunotherapeutic composition comprising at least one constituent component such as an immunological entity, epitope, or immunological entity binder identified based on the present invention, an immunological entity binder (e.g., antigen) or polypeptide comprising the same or having antigen specificity or binding mode belonging to the same cluster, composition described above or herein, or T cell or host cell described above or herein.

The present invention also relates to a diagnostic method or therapeutic method. The method can comprise the step of administering a pharmaceutical composition such as an immunotherapeutic composition or an immunological entity binder (e.g., vaccine) comprising a component disclosed herein to an animal (including humans herein). These methods can be for treating or preventing a disease, disorder, or biological condition. Examples of administration can include transdermal, intranodular, perinodal, oral, intravenous, intradermal, intramuscular, intraperitoneal, mucosal, aerosol inhalation, instillation, and other delivery modes. The method can further comprise the step of assaying to determine a characteristic indicating a condition of a target cell. The method can further comprise a first assaying step and a second assaying step, wherein the first assaying step is performed before a step of administering a therapeutic drug or the like, and the second assaying step is performed after the step of administering a therapeutic drug or the like. In this case, the method can further comprise the step of comparing a characteristic determined by the first assaying step with a characteristic determined by the second assay step, thereby obtaining a result. The result can be, for example, an indication of an immune response, decrease in the target cell count, decrease in the mass or size of tumor comprising a target cell, decrease in the number or concentration of intracellular parasite infected target cells or the like. The result can be judged based on an immunological entity or epitope that has been classified, identified, or clustered by the method of the invention, or that comprising the same, or antigen specificity or binding mode.

### <Antibody/Cell therapy × diagnosis>

In one aspect, the present invention provides a composition for diagnosing a disease, disorder, or biological condition, comprising an immunological entity with antigen specificity or binding mode identified based on the analysis method of the invention. The present invention also provides a method for diagnosing a disease, disorder, or biological condition, comprising the step of diagnosing based on an immunological entity with antigen specificity or binding mode identified based on the analysis method of the invention. Such a method can be applied to, for example, antibody drug or diagnosis when performing cell therapy.

In another aspect, the present invention provides a method for diagnosing a disease, disorder, or biological condition, comprising the step of diagnosing based on an immunological entity with antigen specificity or binding mode identified based on the method of the invention. Alternatively, the present invention provides a method for judging an adverse event for a disease, disorder, or biological condition, comprising the step of determining an adverse event based on an immunological entity with antigen specificity or binding mode identified based on the method of the invention. The present invention also provides a method for diagnosing a disease, disorder, or biological condition, comprising the step of diagnosing based on an immunological entity with antigen specificity or binding mode identified based on the method of the invention, wherein the at least two immunological entities or the collection of immunological entities comprise at least one immunological entity derived from a healthy individual. In this regard, the present invention is a surprising discovery in that the invention can effectively identify an adverse event while further including a healthy individual in the subject of analysis, i.e., at least two immunological entities or the collection of immunological entities.

The diagnosis targeted by the invention is understood to use a cluster of immunological entities as an indicator of, for example, therapeutic efficacy, prognosis, risk of side effect (adverse event, severe adverse event, or the like), pathology, recurrence, or the like. Candidate selection can be obtained as a cluster, which: 1. is manifested significantly in one group in a comparison between groups of interest such as patient of a specific disease/healthy individual or patient of another disease, drug responder/non-responder, or presence/absence of side effect, or 2. comprises a sequence of an immunological entity demonstrated to be associated with the indicator (therapeutic efficacy or the like) by an in vitro/ex vivo/in vivo test or the like, or a combination thereof. These indicators can be combined with other indicators such as the peripheral cytokine volume, cancer cell count, circulating DNA, HLA type, SNPs (genetic mutations), gene expression, epigenome, metagenome, or other indicators that are different from immune cells, or the cell count of a specific cell type, or an indicator such as gene expression or surface marker of an immune cell. In this regard, combine includes testing in parallel with simply an immunological entity cluster, a responder patient selection indicator, for the purpose of limiting the cell type of a subject of clustering. For example, the quantity/number of immunological entities determined as cancer specific at or above a certain number before therapy or after a certain period from therapy, or an increase thereof compared to before therapy can be an indicator for determining therapeutic efficacy. With regard to side effects, if the number of sequences with T cell receptors/B cell receptors determined to have a specific HLA type and/or a relationship with a risk of a specific side effect is at a certain number, risk is determined to be high so that measures can be taken such as avoiding the therapy or reducing the dosage. In a cluster reflecting a pathological condition, the change in the cluster during therapy can be considered as an indicator for judging therapeutic efficacy. If, for example, said cluster reflects the activity of an autoimmune disease, it can be determined that the disease is in remission when the cluster disappears due to therapy.

In one embodiment of the invention, a disease, disorder, or biological condition targeted by the present invention can comprise an adverse event. A therapy that avoids side effects (adverse event, severe adverse event, or the like) in advance can be administered in view of being able to determine an adverse event.

In one embodiment of the invention, a sample of a healthy individual can be included in subjects of analysis. Unexpectedly, the attributes of a patient suffering from a disease (e.g., breast cancer patient) can be analyzed in detail by including a healthy individual. This has resulted in the analysis results that are correct or very likely.

In such a case, the present invention provides a method for diagnosing a disease, disorder, or biological condition, comprising the steps of: (i) providing a feature of at least two immunological entities, wherein the at least two immunological entities comprise at least one immunological entity derived from a healthy individual; (ii) subjecting analysis of antigen specificity or binding mode of the immunological entities to machine learning without specifying antigen specificity or binding mode based on the feature; (iii) classifying the antigen specificity or binding mode or determining whether the antigen specificity or binding mode is the same/different; and (iv) judging a disease, disorder, or biological condition based on the immunological entities classified or determined in (iii).

In one embodiment, a targeted disease, disorder, or biological condition comprises an adverse event. The present invention can administer high quality treatment or prevention of diseases, disorders, or various conditions by utilizing the present invention that can determine an adverse event and provide a result at an unexpectedly very high probability.

Alternatively in another embodiment, the present invention provides a method for diagnosing a disease, disorder, or biological condition, the method comprising the steps of: (a) extracting a feature for at least a pair of members of the collection of immunological entities, wherein the collection of immunological entities comprises at least one immunological entity derived from a healthy individual; (b) computing a distance between antigen specificities or binding modes or judging whether the antigen specificities or binding modes match for the pair by machine learning using the feature; (c) clustering the collection of immunological entities based on the distance; (d) analyzing based on a classification by the clustering; and (e) judging a disease, disorder, or biological condition based on the immunological entities analyzed in (d).

In one specific embodiment, a disease, disorder, or biological condition targeted by the present invention comprises an adverse event.

In another embodiment, the present invention provides a method for diagnosing a disease, disorder, or biological condition, the method comprising the steps of: (aa) extracting a feature for each sequence constituting at least a pair of members of the collection of immunological entities, wherein the collection of immunological entities comprises at least one immunological entity derived from a healthy individual; (bb) projecting the feature onto a high dimensional vector space, wherein a distance on the space between the members reflects functional similarity of the members; (cc) clustering the collection of immunological entities based on the distance; (dd) analyzing based on a classification by the clustering; and (ee) judging, a disease, disorder, or biological condition based on the immunological entities analyzed in (dd).

In one specific embodiment, a disease, disorder, or biological condition targeted by the present invention comprises an adverse event.

### <Antibody/Cell therapy × therapy/prevention>

In another aspect, the present invention provides a composition for treating or preventing a disease, disorder, or biological condition, comprising an immunological entity with antigen specificity or binding mode identified based on the analysis method of the invention. The present invention also provides a method for treating or preventing a disease, disorder, or biological condition, comprising the step of administering an effective amount of an immunological entity with antigen specificity or binding mode identified based on the analysis method of the invention. Such methods can be applied to antibody drugs, cell therapy, and the like.

In another aspect, the present invention provides a method for treating or preventing a disease, disorder, or biological condition, comprising the step of administering an effective amount of an immunological entity with antigen specificity or binding mode identified based on the method of the invention. Alternatively, the present invention provides a method for treating or preventing a disease, disorder, or biological condition, comprising the step of administering to a subject an effective amount of an immunological entity with antigen specificity or binding mode identified based on the method of the invention, wherein the subject excludes a subject determined as a subject who can have an adverse event based on the method of the invention. Alternatively, the present invention provides a method for treating or preventing a disease, disorder, or biological condition, comprising the step of administering an effective amount of an immunological entity with antigen specificity or binding mode identified based on the present invention, wherein the at least two immunological entities or the collection of immunological entities comprise at least one immunological entity derived from a healthy individual. In this regard, the present invention is a surprising discovery in that the invention can effectively identify an adverse event and administer an effective therapy or prevention while further including a healthy individual in the subject of analysis, i.e., at least two immunological entities or collection of immunological entities.

Selection of a candidate of an immunological entity (e.g., antibody drug or cell drug) that can be used in therapy/prevention is expected to include administering an immunological entity obtained as a result of clustering. The selection can additionally use another indicator.
(1) A specific cluster found in a disease responder or drug responsive patient (including the so-called exceptional responder; https://peoplepoweredmedicine.org/neer) or found at a significantly higher probability/ratio compared to some type of comparative cohort is selected.
(2) If found in a cell group expressing another indicator, e.g., a specific surface marker such as a surface marker/gene expression (CD103, CD39 or the like) that is considered cancer specific or an immune checkpoint molecule (PD-1, LAG3, CTLA-4, TIM-3 or the like) (or a combination thereof), or rather the expression of such markers is significantly high in a cell group in the same cluster, such a result is selected as an indicator.
(3) A sequence selected from a cluster confirmed to bind to an antigen in an in vitro/ex vivo/in vivo experiment or the like, exhibit cytotoxicity, exhibit suppression of inflammation,:or the like is selected.
(4) a selection combining two of (1) to (3) ((1) and (2), (2) and (3), and (3) and (1)) or three of (1) to (3), or the like.

In one embodiment of the invention, a disease, disorder, or biological condition targeted by the present invention can comprise an adverse event. A therapy that avoids side effects (adverse event, severe adverse event, or the like) in advance can be administered in view of being able to determine an adverse event.

In one embodiment of the invention, a sample of a healthy individual can be included in subjects of analysis. Unexpectedly, the attributes of a patient suffering from a disease (e.g., breast cancer patient) can be analyzed in detail by including a healthy individual. This has resulted in the analysis results that is correct or very likely.

In one embodiment, the present invention provides a method for treating or preventing a disease, disorder, or biological condition, comprising the steps of: (i) providing a feature of at least two immunological entities, wherein the at least two immunological entities comprise at least one immunological entity derived from a healthy individual; (ii) subjecting analysis of antigen specificity or binding mode of the immunological entities to machine learning without specifying antigen specificity or binding mode based on the feature; (iii) classifying the antigen specificity or binding mode or determining whether the antigen specificity or binding mode is the same/different; and (iv) administering the immunological entities classified or determined in (iii) or an immunological entity binder corresponding to the immunological entities.

In a specific embodiment, the disease, disorder, or biological condition targeted by the present invention comprises an adverse event, or the treatment or prevention comprises treating or preventing while avoiding an adverse event.

In another embodiment, the present invention provides a method for treating or preventing a disease, disorder, or biological condition, the method comprising the steps of: (a) extracting a feature for at least a pair of members of the collection of immunological entities, wherein the collection of immunological entities comprises at least one immunological entity derived from a healthy individual; (b) computing a distance between antigen specificities or binding modes or judging whether the antigen specificities or binding modes match for the pair by machine learning using the feature; (c) clustering the collection of immunological entities based on the distance; (d) optionally analyzing based on a classification by the clustering; and (e) administering the immunological entities analyzed in (d) or an immunological entity binder corresponding to the immunological entities.

In a specific embodiment, the disease, disorder, or biological condition targeted by the present invention comprises an adverse event, or the treatment or prevention comprises treating or preventing while avoiding an adverse event.

In still another embodiment, the present invention provides a method for treating or preventing a disease, disorder, or biological condition, the method comprising the steps of: (aa) extracting a feature for each sequence constituting at least a pair of members of the collection of immunological entities, wherein the collection of immunological entities comprises at least one immunological entity derived from a healthy individual; (bb) projecting the feature onto a high dimensional vector space, wherein a distance on the space between the members reflects functional similarity of the members; (cc) clustering the collection of immunological entities based on the distance; (dd) optionally analyzing based on a classification by the clustering; and (ee) administering the immunological entities analyzed in (dd) or an immunological entity binder corresponding to the immunological entities.

In a specific embodiment, the disease, disorder, or biological condition targeted by the present invention comprises an adverse event, or the treatment or prevention comprises treating or preventing while avoiding an adverse event.

### <Vaccine × diagnosis>

In another aspect, the present invention provides a composition for diagnosing a disease, disorder, or biological condition, comprising an immunological entity binder corresponding to an epitope identified based on the analysis method of the invention. The present invention also provides a method for diagnosing a disease, disorder, or biological condition, comprising the step of diagnosing based on an immunological entity binder corresponding to an epitope identified based on the analysis method of the invention. For example, such a method can be applied to diagnosis or the like when implementing vaccine therapy. Alternatively, the present invention provides a method for judging an adverse event for a disease, disorder, or biological condition, comprising the step of determining an adverse event based on an immunological entity binder corresponding to an epitope identified based on the method of the invention. Alternatively, the present invention also provides a method for diagnosing a disease, disorder, or biological condition, comprising the step of diagnosing based on an immunological entity binder corresponding to an epitope identified based on the method of the invention, wherein the at least two immunological entities or the collection of immunological entities comprise at least one immunological entity derived from a healthy individual. In this regard, the present invention is a surprising discovery in that the invention can effectively identify an adverse event while further including a healthy individual in the subject of analysis, i.e., at least two immunological entities or collection of immunological entities.

A cluster of immunological entities can be used as an indicator for predicting efficacy before and after vaccination. For example, the cluster can be used before vaccination as an indicator for whether a vaccine can induce the immunity of interest, or after vaccination as an indicator for whether a vaccine was able to induce the immunity of interest.

Candidate cluster selection can be obtained as a cluster, which: 1. is manifested significantly in a vaccine responder group in a comparison between groups of interest such as vaccine response/non-response before and after vaccination, or 2. comprises a vaccine identified by an in vitro/ex vivo/in vivo test or the like comprising a sequence of a useful immunological entity of interest, or a combination thereof.

These indicators can be combined with other indicators such as the peripheral cytokine volume, cancer cell count, circulating DNA, HLA type, SNPs (genetic mutations), gene expression, epigenome, metagenome, or other indicators that are different from immune cells, or the cell count of a specific cell type, or an indicator such as gene expression or surface marker of an immune cell. In this regard, combine includes testing in parallel with simply an immunological entity cluster, a responder patient selection indicator, for the purpose of limiting the cell type of a subject of clustering.

For example, the quantity/number of immunological entities associated with vaccine efficacy at or above a certain number before vaccination or after a certain period from vaccination, or an increase thereof compared to before vaccination can be an indicator for determining vaccine efficacy.

In practical use of a vaccine, a sample derived from a healthy individual can be used in analysis, or an adverse event can be predicted and diagnosed to administer prevention or treatment in a manner that avoids the adverse event in advance.

### <Vaccine × therapy/prevention>

In another aspect, the present invention provides a composition for treating or preventing a disease, disorder, or biological condition, comprising an immunological entity binder corresponding to an epitope identified based on the analysis method of the invention. The present invention also provides a method for treating or preventing a disease, disorder, or biological condition, comprising the step of administering an effective amount of an immunological entity binder corresponding to an epitope identified based on the analysis method of the invention. Examples of immunological entity binders include, but are not limited to, vaccines.

In one embodiment, the present invention provides a method for treating or preventing a disease, disorder, or biological condition, comprising the step of administering an effective amount of an immunological entity binder corresponding to an epitope identified based on the method of the invention, wherein the subject excludes a subject determined as a subject who can have an adverse event based on the method of the invention. The present invention also provides a method for treating or preventing a disease, disorder, or biological condition, comprising the step of administering an effective amount of an immunological entity binder corresponding to an epitope identified based on the method of the invention, wherein the at least two immunological entities or the collection of immunological entities comprise at least one immunological entity derived from a healthy individual. In this regard, the present invention is a surprising discovery in that the invention can effectively identify an adverse event and as a result attain a highly effective therapeutic or preventive effect while further including a healthy individual in the subject of analysis, i.e., at least two immunological entities or collection of immunological entities.

Such a method can be applied, for example, when administering vaccine therapy. For selection of a candidate of an immunological entity binder such as a vaccine, the immunological entity binder corresponding to an epitope obtained as a result of clustering can itself be administered, but the selection can additionally use another indicator.
(1) A specific cluster found in a disease responder or drug responsive patient (including the so-called exceptional responder; https://peoplepoweredmedicine.org/neer) or found at a significantly higher probability/ratio compared to some type of comparative cohort is selected.
(2) If found in a cell group expressing another indicator, e.g., a specific surface marker (or a combination thereof) such as a cancer specific T cell marker (CD103 or CD39) or an immune checkpoint molecule, or rather the expression of such markers is significantly high in a cell group in the same cluster, such a result is selected as an indicator.
(3) A sequence selected from a cluster confirmed to bind to an antigen in an *in vitro*/*ex vivo*/*in vivo* experiment or the like, exhibit cytotoxicity, exhibit suppression of inflammation, readily induced (epitope with high immunogenicity), or the like is selected.
(4) a selection combining two of (1) to (3) ((1) and (2), (2) and (3), and (3) and (1)) or three of (1) to (3), or the like.

In practical use of a vaccine, a sample derived from a healthy individual can be used in analysis, or an adverse event can be predicted and diagnosed to administer treatment or prevention or treatment in a manner that avoids the adverse event in advance.

The present invention relates to a method for making a passive/adoptive immunotherapeutic drug with an immunological entity, epitope, or immunological entity binder identified based on the present invention, a cluster comprising the same, or immunological entity binder (e.g., antigen) or polypeptide comprising the epitope. The method can comprise combining a T cell or host cell described in other parts herein with a pharmaceutically acceptable adjuvant, carrier, diluent, excipient, or the like. A buffer, binding agent, blasting agent, diluent, flavoring agent, lubricant, or the like can be included as the excipient.

In one aspect, the present invention relates to a method for diagnosing a disorder, disease, or biological condition using an immunological entity, epitope, or immunological entity binder identified based on the present invention, or immunological entity binder (e.g., antigen) or polypeptide comprising the same or having antigen specificity or binding mode belonging to the same cluster, or the like. The method can comprise contacting subject tissue with at least one constituent component comprising, for example, a T cell, host cell, antibody, and protein, including any one of the components described above or in other parts herein, and diagnosing a disease based on a characteristic of the tissue or constituent component. The contacting step can be performed, for example, *in vivo* or *in vitro.* The present invention further comprises the step of identifying a classified epitope. Such an identification step comprises determining of the structure thereof as well as, but not limited to, determining an amino acid sequence, identifying a three-dimensional structure, identifying of another structure, identifying a biological function, and the like.

In still another embodiment, the present invention relates to a method for making a vaccine. This method can comprise combining at least one constituent component including an epitope, immunological entity binder, composition, construct, T cell, and host cell including any of the components described in other parts herein with a pharmaceutically acceptable adjuvant, carrier, diluent, excipient, or the like. In another embodiment, the present invention can evaluate or improve a vaccine using the clustering and classification method of the invention and an immunological entity, epitope, or immunological entity binder identified therewith or an immunological entity, epitope, or immunological entity binder with antigen specificity or binding mode that has been identified. The present invention can also evaluate and/or generate or improve a biomarker using an identified epitope or an immunological entity binder, an immunological entity, epitope, or immunological entity binder with antigen specificity or binding mode that has been identified, or the cluster itself. In this regard, "improve" means providing a methodology for improving vaccine performance that can more appropriately evaluate neutralizing antibody production upon vaccination by identifying a cluster whose antibody titer is desirably increased by clustering, by performing the methodology in parallel with a normal experiment. Examples of "evaluation" of a biomarker include a method for at first identifying a cluster (e.g., cluster correlated with a state of a disease) that can be a biomarker itself and investigating whether a more simple experimentation (e.g., can be performed using an ELISA binding assay or the like) is able to suitably follow an expected change in the cluster. Such a case presumes that the cluster itself can function as a marker, but this can also be made in the same manner (to reflect information of the cluster).

The present invention also provides a composition for evaluating a vaccine for treating or preventing a disease, disorder, or biological condition, comprising an immunological entity, epitope, or immunological entity binder identified based on the present invention or an immunological entity comprising the same, or having or against antigen specificity or binding mode belong to the same cluster. For such evaluation, an example of influenza viruses, for example, can be applied. In another aspect, the present invention relates to a method for treating or preventing a disease using an immunological entity, epitope, or immunological entity binder identified based on the present invention, or immunological entity binder (e.g., antigen) or polypeptide comprising the same or having antigen specificity or binding mode belonging to the same cluster, or the like. The method can comprise combining a therapeutic method of an animal comprising administering a vaccine or immunotherapeutic composition described in other parts herein to the animal with at least one therapeutic mode including, for example, radiation therapy, chemotherapy, biochemical therapy, and surgery.

The present invention also relates to a vaccine or immunotherapeutic product comprising an immunological entity, epitope, or immunological entity binder identified based on the present invention, or an epitope that has been classified or clustered in the present invention comprising the same or having antigen specificity or binding mode belonging to the same cluster, a cluster comprising the epitope, immunological entity binder (e.g., antigen) or polypeptide comprising the epitope or having antigen specificity or binding mode belonging to the same cluster, or the like. A still another embodiment relates to an isolated polynucleotide encoding a polypeptide described in other parts herein. Another embodiment relates to a vaccine or immunotherapeutic product comprising such a polynucleotide. A polynucleotide can be a DNA, RNA, or the like.

In one embodiment, the present invention also relates to a kit comprising a delivery device and any one of the embodiments described in other parts herein. A delivery device can be a catheter, syringe, internal or external pump, reservoir, inspiratory, microinjector, patch, or any other similar device suitable for any route of delivery. As discussed above, a kit can also comprise any one of the embodiments disclosed herein in addition to a delivery device. For example, a kit can comprise, but not limited to, an isolated epitope, polypeptide, cluster, nucleic acid, immunological entity binder (e.g., antigen), pharmaceutical composition comprising any one of the above, antibody, T cell, T cell receptor, epitope-MHC complex, vaccine, immunotherapeutic drug, or the like. A kit can also comprise a construct such as a detailed user manual or any other similar item.

A particularly desirable strategy for including an immunological entity, epitope, or immunological entity binder and/or epitope cluster with the same binding mode or antigen specificity in a vaccine or a pharmaceutical composition is disclosed in US Patent Application Publication No. 09/560,465 entitled "EPITOPE SYNCHRONIZATION IN ANTIGEN PRESENTING CELLS" filed on April 28, 2000.

The vaccine that can be used in the present invention comprises an epitope or an immunological entity binder (e.g., antigen) at a concentration effective to present an epitope or immunological entity binder that has been classified, identified, or clustered in the present invention or an epitope or immunological entity binder with antigen specificity or binding mode that has been identified. Preferably, the vaccine of the invention can comprise a plurality of the epitope of the invention or cluster thereof in combination with any one or more immunological epitopes. The vaccine formulation of the invention comprises a peptide and/or nucleic acid at a concentration that is sufficient to present an epitope to a target. The formulation of the invention preferably comprises an epitope at a total concentration of about 1 µg to 1 mg/(100 µl of vaccine preparation) or a peptide comprising the same. Conventional dosage and dosing related to a peptide vaccine and/or nucleic acid vaccine can be used with the present invention. Such a dosing regimen is thoroughly understood in the art. In one embodiment, a single dose for adults is suitably about 1 to 5000 µl of composition, which is administered as a single or multiple doses, such as two, three, four or more doses separated in 1 week, 2 weeks, 1 month, or more. The vaccine of the invention can comprise a recombinant organism such as a virus, bacteria, or protozoa genetically engineered to express an epitope in a host.

The vaccine, composition, and method of the invention can blend an adjuvant to a formulation to enhance the performance of the vaccine. Specifically, an adjuvant can be designed to enhance the delivery and intake of an epitope. Adjuvants intended by the present invention are known to those skilled in the art. Examples thereof include GM-CSF, GCSF, IL-2, IL-12, BCG, tetanus toxoid, osteopontin, and ETA-1.

The vaccine or the like of the invention can be administered by any suitable method. The vaccine of the invention is administered to a patient in a mode consistent with a standard vaccine delivery protocol known in the art. Examples of epitope delivery methods include, but are not limited to, transdermal, intranodular, perinodal, oral, intravenous, intradermal, intramuscular, intraperitoneal, and mucosal administration, including delivery by injection, instillation, or inhalation. Particularly useful methods of vaccine delivery for inducing a CTL response are disclosed in AU Patent No. 739189 published on January 17, 2002, US Patent Application Publication No. 09/380,534 filed on September 1, 1999, and partially simultaneously pending US Patent Application Publication No. 09/776,232 filed on February 2, 2001, which are incorporated herein by reference.

In one embodiment, the present invention can also comprise a protein, antibody, cell that can express them, specific B cell and T cell, or the like, which specifically binds to an immunological entity, epitope, or immunological entity binder (e.g., antigen) at a concentration effective to present an immunological entity, epitope, or immunological entity binder that has been classified, identified, or clustered in the present invention or immunological entity, epitope, or immunological entity binder with antigen specificity or binding mode that has been identified. These reagents are in a form of an immunoglobulin, i.e., a polyclonal serum or monoclonal antibody whose production method is well known in the art. Production of mAb having specificity related to a peptide-MHC molecule complex is known in the art (Aharoni et al. Nature 351: 147-150, 1991 and the like). General construct and use are also discussed in US Patent No. 5,830,755 entitled "T CELL RECEPTORS AND THEIR USE IN THERAPEUTIC AND DIAGNOSTIC METHODS".

In one embodiment, one of immunological entity, epitope, or immunological entity binder (e.g., antigen) having binding mode or antigen specificity at a concentration effective to present binding mode, antigen specificity, immunological entity, epitope, or immunological entity binder that has been classified, identified, or clustered in the present invention can be bound to an enzyme, radioactive chemical substance, fluorescent tag, and toxin for use in diagnosing (imaging or other detection), monitoring, and treating a binding mode, antigen specificity, immunological entity, epitope, or immunological entity binder associated pathogenic state. Therefore, a toxin conjugate can be administered to kill tumor cells, and a radiolabel can facilitate imaging of binding mode, antigen specificity, immunological entity, epitope, or immunological entity binder-positive tumor, and an enzyme conjugate can be used in an ELISA-like assay to diagnose cancer and confirm epitope expression in biopsy tissue. In still another embodiment, T cells described above can be administered to a patient as an adoptive immunotherapy after proliferation achieved by stimulation with a cytokine and/or epitope of binding mode or antigen specificity.

In another embodiment, the present invention provides a complex of an epitope or immunological entity binder with binding mode or antigen specificity that has been classified, identified, or clustered in the present invention and MHC, or a peptide-MHC complex as an epitope or immunological entity binder with binding mode or antigen specificity. In a particularly suitable embodiment, a complex can be a soluble multimer protein described in US Patent No. 5,635,363 (tetramer) or US Patent No. 6,015,884 (Ig-dimer). Such a reagent is useful for detecting and monitoring a specific T cell response and purifying said T cell.

In another embodiment, an immunological entity, epitope, or immunological entity binder with binding mode or antigen specificity that has been classified, identified, or clustered in the present invention can be used to perform a functional assay, evaluate endogenous immunity level or a response to immunological stimulation (e.g., vaccine), and monitor the immune state due to the path of therapy and the disease. Except when measuring an endogenous immunity level, each of these assays can presume a preliminary step for immunity *in vivo* or *in vitro* depending on the nature of the problem to be addressed. Such immunity can be performed using various embodiments of the invention, or immunogen in other forms that can induce the same immunity. Except for tetramer/Ig-dimer analysis and PCR that can detect the expression of homologous TCRs, these assays can generally benefit from the step of *in vitro* antigenic stimulation that can suitably use various aforementioned embodiments of the invention in order to detect a specific functional activity (can be directly detected for a high cytolytic response). Finally, detection of cytolytic activity requires a substance with binding mode or antigen specificity belonging to the same cluster or epitope presenting target cells, which can be produced using various embodiments of the invention. The specific embodiment selected for any specific step is dependent on the problem to be addressed, ease of use, cost, or the like, but the advantage of one embodiment over another embodiment related to any specific set of circumstances is evident to those skilled in the art.

Such a functional assay can be associated with the binding mode or antigen specificity of the invention, or use an activation step or a reading step or both in a form of an immunological entity, epitope, or immunological entity binder or a complex with an MHC molecule. Two categories of assays, i.e., assay for measuring a response of a cell pool and an assay for measuring a response of individual cells, can be practiced among the many assays of T cell functions known in the art (detailed procedures can be found in standard immunological reference documents such as Current Protocols in Immunology 1999 John Wiley & Sons Inc., N.Y). The former can measure the overall strength of responses, while the latter can determine the relative frequency of responsive cells. Examples of the assay for measuring an overall response include cytotoxic assay, ELISA, and proliferation assay for detecting cytokine secretion. Examples of the assay for measuring a response of individual cells include limiting dilution analysis (LDA), ELISPOT, flow cytometric detection of unsecreted cytokines (described in US Patent No. 5,445,939, US Patent No. 5,656,446, and US Patent No. 5,843,689, and reagents therefor are sold by Becton, Dickinson & Company under the product name "FASTIMMUNE"), detection of specific TCR with a tetramer or Ig-dimer as discussed and cited above (see also Yee, C. et al. Current Opinion in Immunology, 13: 141-146, 2001).

The present invention can be provided as a kit. As used herein, "kit" refers to a unit providing parts to be provided (e.g., test drug, diagnostic drug, therapeutic drug, antibody, label, user manual, and the like) which are generally separated into two or more segments. Such a kit form is preferred when providing a composition, which should not be provided in a mixed state for stability or the like and is preferably used by mixing immediately prior to use. Such a kit preferably comprises an instruction or manual describing how the provided portions (e.g., test drug, diagnostic drug, or therapeutic drug) are used or how a reagent should be processed. When a kit is used as a reagent kit herein, the kit generally comprises an instruction or the like describing the method of use of a test drug, diagnostic drug, therapeutic drug, antibody, or the like.

In this manner, in still another aspect of the invention, the present invention relates to a kit having (a) a container comprising the pharmaceutical composition of the invention in a solution or lyophilized form, (b) optionally a second container comprising a diluent or reconstitution solution for the lyophilized formulation, and (c) optionally a manual for the (i) use of the solution or (ii) reconstitution and/or use of the lyophilized formulation. The kit further has one or more of (iii) buffer, (iv) diluent, (v) filter, (vi) needle, or (v) syringe. The container is preferably a bottle, vial, syringe, or test tube, and the container may be a multi-purpose container. The pharmaceutical composition is preferably lyophilized.

The kit of the invention preferably has the lyophilized formulation of the invention and manual for reconstitution and/or use thereof in a suitable container. Examples of the suitable container include a bottle, vial (e.g., dual chamber vial), syringe (dual chamber syringe or the like), and test tube. The container can be made of various materials such as glass or plastic. Preferably, the kit and/or container comprises a manual showing the method of reconstitution and/or use on the container or accompanying the container. For example, the label thereof can have an explanation showing that the lyophilized formulation is reconstituted to have the above peptide concentration. The label can further have an explanation showing that the formulation is useful for, or is for subcutaneous injection.

The container of the formulation can be a multi-purpose vial that can be used for repeated dosing (e.g., 2 to 6 dosing). The kit can further have a second container having a suitable diluent (e.g., sodium bicarbonate solution).

The final peptide concentration of a reconstituted formulation made by mixing the diluent and the lyophilized formulation is preferably, but is not limited to, at least 0.15 mg/mL/peptide (when = 75 µg, 0.5 ml) and preferably 3 mg/mL/peptide (when = 1500 µg, 0.5 ml) or less. The kit can further comprise other materials (including other buffer, diluent, filter, needle, syringe, and user manual inserted into the package) that are desirable from the commercial viewpoint or user viewpoint.

The kit of the invention can have a single container comprising a formulation of the pharmaceutical composition of the invention with or without other constituent elements (e.g., other compounds or pharmaceutical composition of such other compounds) or have another container for each constituent element.

The kit of the invention preferably comprises a formulation of the invention which is packaged for use as a combination with concomitant administration of a second compound (adjuvant (e.g., GM-CSF), chemotherapeutic agent, naturally-occurring product, hormone or antagonist, other drugs, or the like) or a pharmaceutical composition thereof. Constituent elements of the kit can be constituents made in advance as a complex, or each constituent element placed in separate containers until administration to a patient. The constituent elements of the kit can be provided as one or more liquid solutions, preferably an aqueous solution, and more preferably sterilized aqueous solution. The constituent elements of the kit can also be provided as a solid. Preferably, a suitable solution provided in a separate different container can be added thereto to convert the solid into a liquid.

A container of a therapeutic kit can be a vial, test tube, flask, bottle, syringe, or any other means for sealing a solid or liquid. Generally, the kit comprises a second vial or another container when there are a plurality of constituent elements so that the elements can be administered separately. The kit can also comprise another container for a pharmaceutically acceptable solution. Preferably, a therapeutic kit comprises an instrument (e.g., one or more needles, syringes, eye droppers, pipettes, or the like) enabling the administration of the agent of the invention, which is a constituent element of the kit.

The pharmaceutical composition of the invention is suitable for administering the peptide through any acceptable route, such as oral (enteral), nasal, ocular, subcutaneous, intradermal, intramuscular, intravenous, or transdermal route. Preferably, the administration is subcutaneous administration and most preferably intradermal administration. The pharmaceutical composition can be administered by an injection pump.

As used herein, "instruction" is a document with an explanation of the method of use of the present invention for a physician or other users. The instruction describes a detection method of the invention, how to use a diagnostic drug, or a description instructing administration of a drug or the like. Further, an instruction may have a description instructing oral administration, or administration to the esophagus (e.g., by injection or the like) as the site of administration. The instruction is prepared in accordance with a format specified by a regulatory authority of the country in which the invention is practiced (e.g., Ministry of Health, Labour and Welfare in Japan, Food and Drug Administration (FDA) in the U.S., or the like), with an explicit description showing approval by the regulatory authority. The instruction is a so-called package insert, and is generally provided in, but not limited to, paper media. The instructions may also be provided in a form such as electronic media (e.g., web sites provided on the Internet or emails) .

As used herein, "or" is used when "at least one or more" of the listed matters in the sentence can be employed. When explicitly described herein as "within the range" of "two values", the range also includes the two values themselves.

### (General technology)

Any molecular biological methodologies, biochemical methodologies, microbiological methodologies, and bioinformatics used herein that is known in the art, well known, or conventional can be used.

Reference literatures such as scientific literatures, patents, and patent applications cited herein are incorporated herein by reference to the same extent that the entirety of each document is specifically described.

As described above, the present invention has been described while showing preferred embodiments to facilitate understanding. The present invention is described hereinafter based on Examples. The above descriptions and the following Examples are not provided to limit the present invention, but for the sole purpose of exemplification. Thus, the scope of the present invention is not limited to the embodiments and Examples specifically described herein and is limited only by the scope of claims.

### [Examples]

The Examples are described hereinafter. When necessary, all experiments were conducted in compliance with the guidelines approved by the ethics committee of the Osaka University in the following Examples. For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science INC, or the like).

### (Example 1: Antigen specific clustering of antibodies)

Antibody sequences were clustered based on antigen epitope specificity from the crystal structure of antibody-antigen complexes.

### (Data set)

An antigen-antibody complex crystal structure list was downloaded from SAbDab (http://opig.stats.ox.ac.uk/webapps/sabdab-sabpred/Welcome.php, March 16, 2017 version). A heavy atom of an antigen in contact with an antibody was searched, with a threshold value of 3.5 Å. Antigens with a residue length of 3 or greater were kept, and overlaps in the sequences of antigen-antibody were removed using CD-HIT. The antigen sequences were locally aligned with FASTA (using the default settings), and the matching portion was extracted. Sequences with 65% or more of the residues in contact with an antibody sequence on each antigen sequence and with 5 or more residues in contact with the same antigen residue, with RMSD of the contacting residues of less than 5.0A, were considered as recognizing the same epitope. Lastly, the heavy chain and light chain sequences of the antibodies were connected, and those with sequence homology of 90% or greater were eliminated using CD-HIT. Overall, 23,220 pairs were obtained, among which 465 were in the true data set and the rest were in the false data set. 80% were randomly used therefrom in a learning set, and the remaining 20% were used in a test set (Tables 1 and 2).

**[Table 1-1]**

| | | | |
|---|---|---|---|
| lahwBA | 2x71AB | 4fp8IM | 4xvsHL |
| 1bj1HL | 2xraHL | 4fqjHL | 4xwgHL |
| IbogBA | 2yc1AB | 4g6fHL | 4y5yAB |
| lbvkBA | 3b2uCD | 4g6jHL | 4yblHL |
| IdoeFE | 3baeHL | 4hixHL | 4ydlBC |
| 1e4xHL | 3bkyHL | 4hkxAB | 4ydvHL |
| 1fd1HL | 3bn9DC | 4hs6HL | 4zffHL |
| 1fnsHL | 3eyfBA | 4hs8HL | 4zptAB |
| lfrgHL | 3g5vBA | 4i9wED | 5acoGJ |
| 1ggiHL | 3gbmHL | 4ij3CB | 5c7xMN |
| 1himLH | 3gi8HL | 4jb9HL | 5cbaCD |
| lhysDC | 3gjfHL | 4jdtHL | 5cd5CD |
| 1jpsHL | 3grwHL | 4jg0HL | 5dlqBA |
| 1kc5HL | 3h42HL | 4jolIM | 5dlqCD |
| 1n64HL | 3hb3CD | 4jpvHL | 5dlxBA |
| 1nmaHL | 3ixtAB | 4jznIP | 5eocHL |
| lorsBA | 3j5mDC | 4jzoCF | 5f3bAB |
| 1otsCD | 3jabHL | 4k4mHL | 5f3hAB |
| 1q1jIM | 3jwoHL | 4ki5CD | 5f9oHL |
| ls5hBA | 3l5wHL | 4kv5HL | 5fecAB |
| 1tziBA | 3lexHL | 4lspHL | 5fgcEB |
| luj3BA | 3ma9HL | 41suHL | 5gjsHL |
| lv7mHL | 3mlrHL | 41vhHI | 5h37KL |
| 1xgpBA | 3mlwHL | 4m48HL | 5hhvHL |
| lyy9DC | 3mnwBA | 4mhhJK | 5i8cAB |
| 2aepHL | 3o4lAB | 4py8IJ | 5i9qBC |
| 2b0sHL | 3pjsBA | 4r2gJI | 5igxHL |
| 2b2xIM | 3q3gDC | 4ravAB | 5kaqQR |
| 2dtgAB | 3s88HL | 4rdqIH | 5kelCD |
| 2dtgCD | 3sm5HL | 4rfoHL | 5kveHL |
| 2eizBA | 3so3CB | 4slqHL | 5sx4JI |
| 2i91HG | 3u7yHL | 4slrHL | 5te4HL |
| 2ipuHL | 3ujiHL | 4slsHL | 5tljDC |
| 2j4wHL | 3ujjHL | 4tvpDE | 5tlkHG |

**[Table 1-2]**

| | | | |
|---|---|---|---|
| 2nyyDC | 3ulvDC | 4u6hAB | 5tpwHL |
| 2os1HL | 3wxvHL | 4ut6HL | 5u3kHL |
| 2otuBA | 4d9qED | 4utaHL | 5u3oHL |
| 2r4rHL | 4edwHL | 4utbHL | 5wt9HL |
| 2uziHL | 4edxBA | 4xmpHL | |
| 2vdrHL | 4ffvDC | 4xnmHL | |
| 2virBA | 4ffyHL | 4xnyHL | |

### Table 1. Learning set

### [Table 2]

**Table 2. Test set**

| | | | |
|---|---|---|---|
| 1g9mHL | 3mlyHL | 4k9eHL | 4yflHL |
| 1kcsHL | 3rvvDC | 4m62IM | 5b8cBA |
| 1mlcBA | 3se8HL | 4o58HL | 5c7kEF |
| 1ncaHL | 3uyrHL | 4onfHL | 5drzBA |
| 1tjgHL | 3ztjGH | 4pljDC | 5esvHL |
| 1tpxBC | 4al8HL | 4r8wHL | 5eszAB |
| 1yqvHL | 4dgiHL | 4rfnHL | 5f96HL |
| 2fd6HL | 4fqiHL | 4tsaHL | 5f9wBC |
| 2q8aHL | 4hhaBA | 4tvpHL | 5iq9HL |
| 3be1HL | 4hlzGH | 4u0rBC | 5kvfHL |
| 3ifoAB | 4hpoHL | 4uu9AB | 5u3jHL |
| 3ifpAB | 4iofCD | 4xnzHL | 5u3mHL |
| 315xHL | 4j6rHL | 4y5vGH | |
| 31h2IM | 4jpwHL | 4ydiHL | |

### (Feature extraction)

Three CDRs and four FR (framework) regions were identified for each of the heavy chain and light chain for each antibody pair. The features described above were obtained for each region.
*Sequence homology score based on BLOSUM62
*Difference in the lengths of amino acid sequences
*Number of aligned residues.

Grid search was performed so that the mean MCC (Matthews correlation coefficient) would be maximized as a result of 5 cross validation on number of trees of random forest and the number of leaves of each tree by using GridSearchCV of sklearn, which is python's machine learning library. The hyperparameters yielding the maximum MCC were (number of trees, number of leaves of a tree) = (9, 60).

Learning was performed again using the entire learning set by using the optimal hyperparameters. The results were applies to the test set. This resulted in an MCC of 0.85.

### (Conclusion)

It was found that antibody sequences can be clustered based on antigen epitope specificity from the crystal structure of antibody-antigen complexes.

### (Example 2: Antigen specific clustering of TCRs)

This Example clusters TCRs from only TCR-pMHC binding information to show that the clusters reflect different binding specificity (mode).

### (Data set)

TCR sequence data was acquired from the following three databases (data acquired on October 2, 2017)
*ATLAS: https://zlab.umassmed.edu/atlas/web/help.php
*VDJdb: https://vdjdb.cdr3.net/
*McPAS-TCR: http://friedmanlab.weizmann.ac.il/McPAS-TCR/

Only TCRs derived from humans and mice were extracted therefrom. Duplicate entries (with the same V gene, J gene, CDR3 sequence) were deleted. As a result, a data set of 10727 unique TCR beta chains (each with information on pMHC) was created.

### (Feature extraction)

The following features were used in machine learning.

### (1) Feature based on V- or J-gene

Information on the amino acid sequences of human and mouse TRAV, TRBV, TRAJ, and TRBJ genes was obtained from IMGT (http://www.imgt.org/vquest/refseqh.html), and each gene family was globally aligned to attain multiple sequence alignment. CDR1, CDR2, FR1, FR2, FR3, and FR4 based on the definition of IMGT were extracted. The CDR2.5 region (Dash, P., Fiore-Gartland, A. J., Hertz, T., Wang, G. C., Sharma, S., Souquette, A., ... Thomas, P. G. (2017). Quantifiable predictive features define epitope-specific T cell receptor repertoires. Nature. https://doi.org/10.1038/nature22383) defined by the 81^{st} to 86^{th} (based on the definition of IMGT) amino acids was also extracted.

### (2) Feature based on CDR3

The sequence of the CDR3 region (105^{th} to 117^{th} amino acids based on the definition of IMGT) was extracted. Sequences described in the database were directly used instead of extracting the sequence from a full length sequence. Truncated CDR3 (those with deletion of the first 3 amino acids and the last 2 amino acids of CDR3) was also obtained.

### (3) Physical amount of total charge/feature

The charge on the side chain contained in each region at ph of 7.5 were added up for each (CDR1, CDR2, CDR2.5, CDR3, FR1, FR2, FR3, and FR4).
*The index of hydrophobicity of the CDR3 region was calculated on the Kyte & Doolittle scale.

### (4) Feature based on comparison of pairs

In addition to the feature for each TCR described above, features for each pair of all TCRs were also calculated.

Distance between sequences: The distance between sequences was calculated for each region (CDR1, CDR2, CDR2.5, CDR3, FR1, FR2, FR3, and FR4) based on BLOSUM62 substitution matrix (Henikoff, S., & Henikoff, J. G. (1992). Amino acid substitution matrices from protein blocks. Proceedings of the National Academy of Sciences, 89(22), 10915-10919. https://doi.org/10.1073/pnas.89.22.10915), based on the generated multiple sequence alignment described above, for TCR-A and TCR-B.

Non-sequence feature: Whether the charge of each region (CDR1, CDR2, CDR2.5, CDR3, FR1, FR2, FR3, and FR4) has the same sign (+ or -) as a Boolean feature, and the absolute value of the difference in hydrophobicity of the CDR3 region were considered.

### (Machine learning algorithm and hyperparameter optimization)

### (1) Machine learning prediction model

Open source LightGBM gradient boosting framework (https://github.com/Microsoft/LightGBM) was used for learning whether a pair of TCRs binds to the same epitope. At this time, the following hyperparameters were optimized: number of trees, number of leaves for each tree, learning rate, and relative weighting of true/false.

### (2) Clustering algorithm

Clustering was performed using a hierarchical clustering method based on the prediction results. In doing so, a fixed threshold value is set for a prediction value, but the threshold value is also optimized upon optimization of hyperparameters.

### (3) Learning/test set division and evaluation

For information on pairs generated from a data set, 80% of epitopes are assigned to a learning set, and 20% are assigned to a test set, based on the epitope to which they bind. The assignment is repeated 10 times.

Scoring: A prediction model created by learning based on various hyperparameters is applied to a test set for evaluation. Evaluation was performed using an MCC score, modified Rand index, and homogeneity score. Learning/prediction/clustering/evaluation was repeated 10 times. The model with a homogeneity score greater than 0.9 which has the highest mean MCC score was selected.

### (Results)

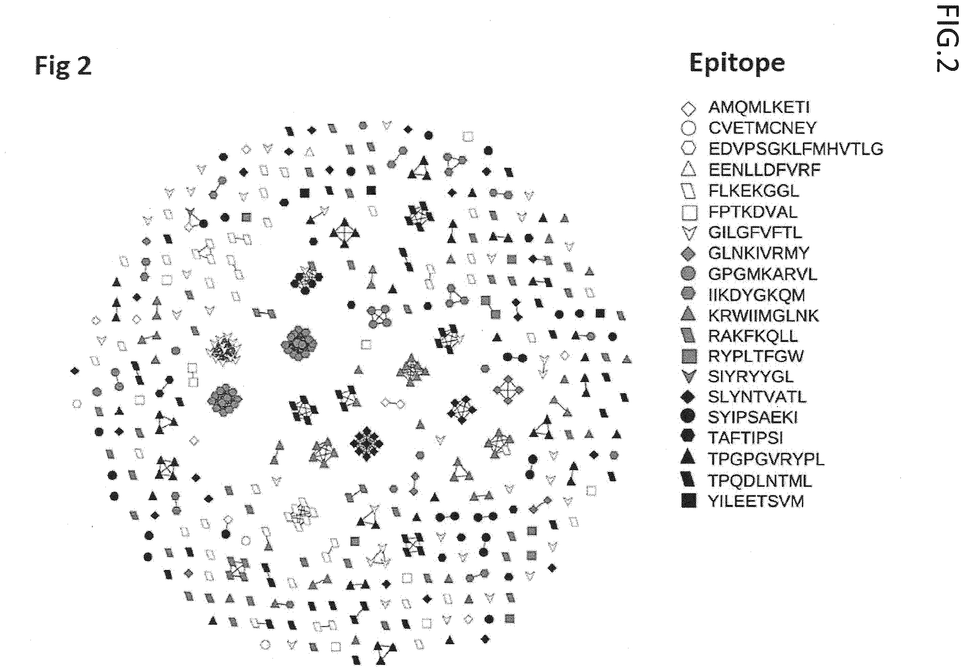

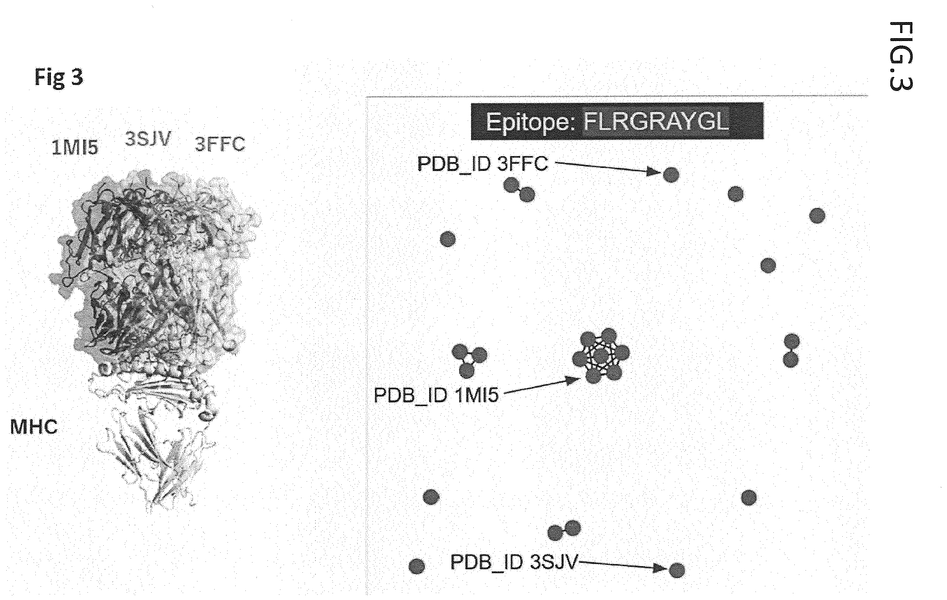

Hyperparameters were optimal at (number of trees, number of leaves for each tree, learning rate, relative weighting of true/false) = (50, 30, 0.1, 1.6). The threshold value for hierarchical clustering was set to 0.6. (Figure **2****)** An optimized model was applied to a TCR recognizing an epitope derived from EBV (Epstein-Barr Virus) with a known TCR-pMHC crystal structure. It was found as a result thereof that TCRs recognizing different positions, even in the same pMHC, were separated into separate clusters, and the clustering results reflect the binding mode (Figure **3****).**

### (Example 3: Prediction of presented MHC and antigen peptide of HIV derived antigen specific TCRs)

This Example performed clustering of antigen unknown TCR and antigen known TCR sequences to show that the antigen of an antigen unknown TCR sequence can be predicted from information'on an antigen known TCR sequence.

### (Data set)

115 HIV derived peptide A specific TCR sequences derived from 14 human specimens obtained at the National Institute of Infection Diseases, 82 HIV derived peptide B specific TCR sequences derived from 7 human specimens, and 236 duplicate free HIV antigen (7 types) specific TCRs contained in the data set used in Example 2 were used.

### (Prediction)

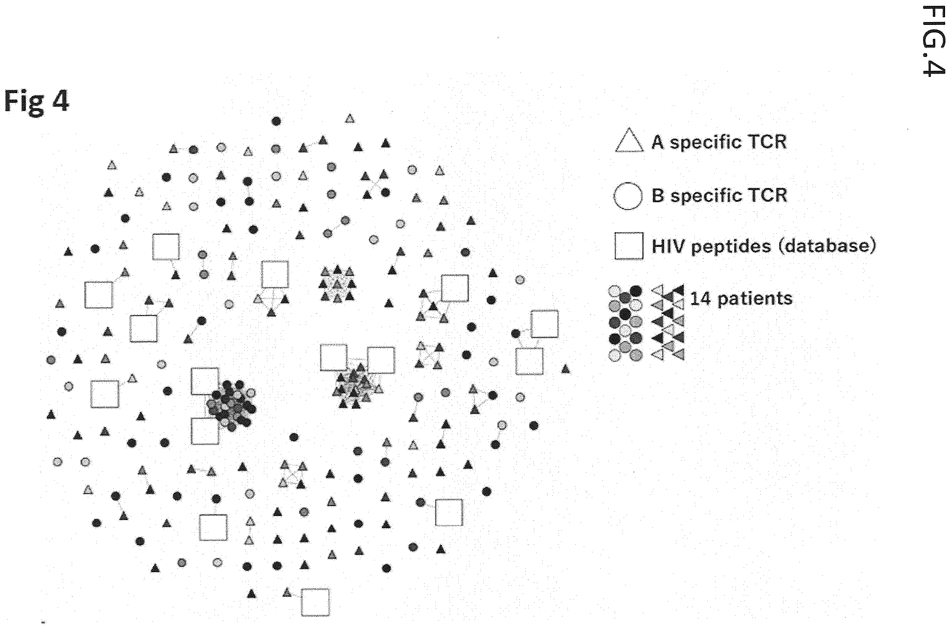

A machine learning model obtained by using the optimal hyperparameters in Example 2 was applied to the data set. The threshold value for hierarchical clustering was also the same (0.6). Figure **4** shows the results of clustering. It can be seen that the peptide A specific sequences and the B specific sequences are separated. For the clusters in the data set used in Example 2, the antigen that would be recognized was predicted from the information on pMHC recognized by a TCR sequence in the cluster.

### (Example 4: Breast cancer diagnosis using clustering with TCRs)

This Example extracted TCRs that are characteristic to breast cancer patients from information on peripheral CD8+ T cell TCR-β chain obtained from breast cancer patients and healthy individuals to find an immune response associated with breast cancer.

### (Data set)

Peripheral blood CD8+ T cell receptor β chain sequence information on 20 breast cancer patients and 6 healthy individuals that was reported in D.J. Munson, et al., PNAS 113(29) 8272-8277, 2016 and uploaded to and published on the Gene Expression Omnibus (GEO) database was used.

### (Results)

The machine learning model optimized in Example 2 was applied to the data set. Since the number of sequences varies for each sample (donor), sampling was performed 100 times that matches the number of sequences of the smallest sample, and the number of instances of expression of sequences belong to each cluster was counted. Clusters with a low number of instances (0 to 1/26 persons) were excluded from the study. A vector was constructed using the resulting cluster.

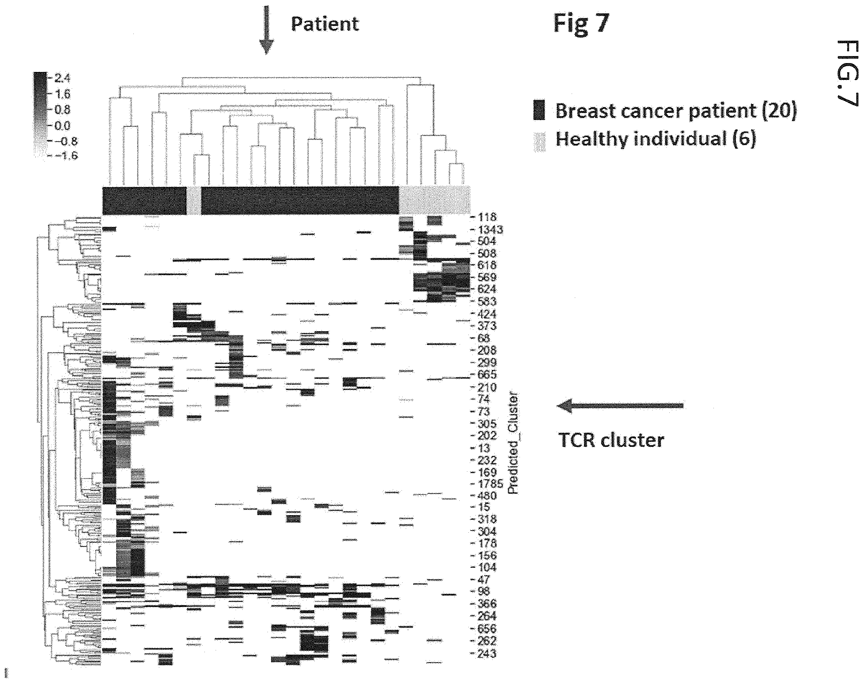

As a result, the breast cancer patients and healthy individuals were able to be divided using information on the peripheral blood CD8+ T cell receptor β chain sequence (Figure **7****).**

### (Example 5: TCR:clustering using autoencoder)

This Example extracted a feature using autoencoder to perform clustering.

### (Data set)

Peripheral T cell receptor β chain sequence information used in R. O. Emerson, et al., Nature Genetics, 49(5), 659-665, 2017 and published in ImmunoSEQ of Adaptive Biotechnologies, Inc was used for the implementation of autoencoder. The overall number of sequences used was about 10 million sequences.

Clustering results were evaluated using T cell receptor (TCR) β chain sequence information stored in the database VDJdb published in M. Shugay, et al., Nucleic Acids Research, 46(D1), D419-D427, 2018.

### (Implementation)

TensorFlow was used for the implementation. The input was a V gene sequence or an amino acid sequence of a CDR3 region (based on the definition of IMGT). Autoencoder was comprised of three layers of symmetric fully-connected layers. Hidden layers were comprised of 100, 200, and 500 hidden units, respectively. For each hidden layer, batch normalization and ReLU activation function were used. An embedding layer was comprised of 50 linear units, and a tanh function was used for the activation function. An output layer is comprised of linear units, and a softmax function was used for the activation function. The probability distribution of 20 types of amino acids in each unit was outputted.

The resulting embedding layer was used as a high dimensional vector expressing a TCR sequence, and TCR sequences were clustered using a clustering algorithm DBSCAN to perform antigen specific clustering, of TCRs.

Evaluation was performed using an entry contained in VDJdb. The entry contained information on the TCR β chain sequence and peptide-MHC complex recognizing the sequence. While VDJdb has entries containing an α chain, only information for β chains was used.

### (Results)

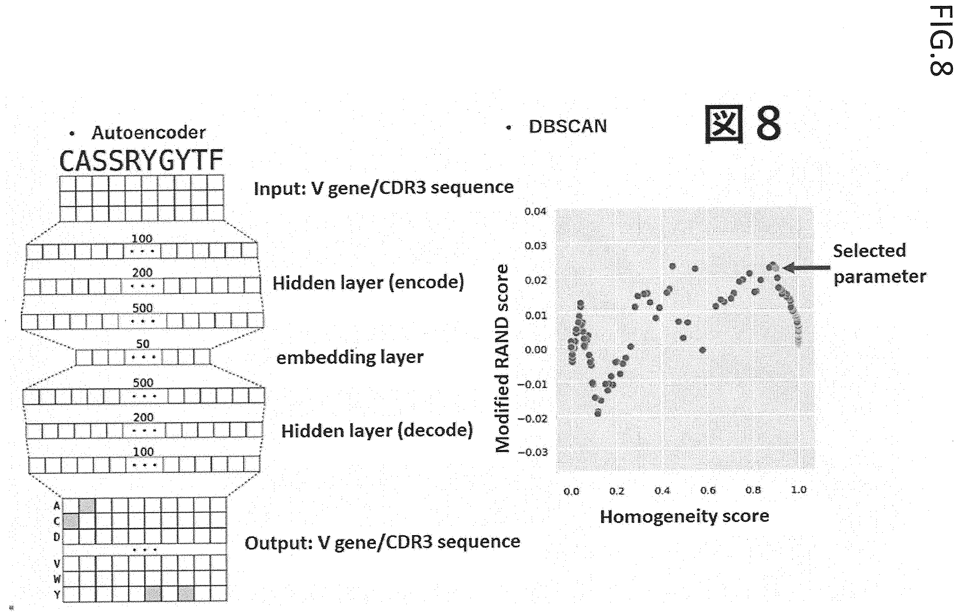

The optimal parameters for DBSCAN were obtained by grid search. Clustering was evaluated according to the modified RAND score while the homogeneity score was > 0.9. In this regard, a homogeneity score represents the ratio of the maximum of peptides and MHC recognized by TCRs contained in the cluster. The resulting RAND score was 0.022 (Figure **8****).**

### (Example 6: Diagnosis combining biological information other than TCR/BCR)

This Example shows that expression or mutation of a gene used in the selection of therapy for breast cancer is linked to an immune response.

### (Data set)

The same data set as Example 4 was used. The machine learning model optimized in Example 5 was applied. However, clustering was performed as follows. First, a linkage matrix was created from 50 dimensional data by unweighted pair group method with arithmetic mean (UPGMA) using a Scipy module. In this regard, Euclidian was used as the Metric. Next, hierarchical clustering (fixed length: t = 0.97 was used as the threshold value) was performed from the linkage matrix. Only clusters consisting of 4 or more samples were used in subsequent calculation. For information associated with genes, the columns of HER2+, ER+, and PR+ described in Table 1 of D. J. Munson, et al., PNAS 113(29) 8272-8277, 2016 were used.

### (Results)

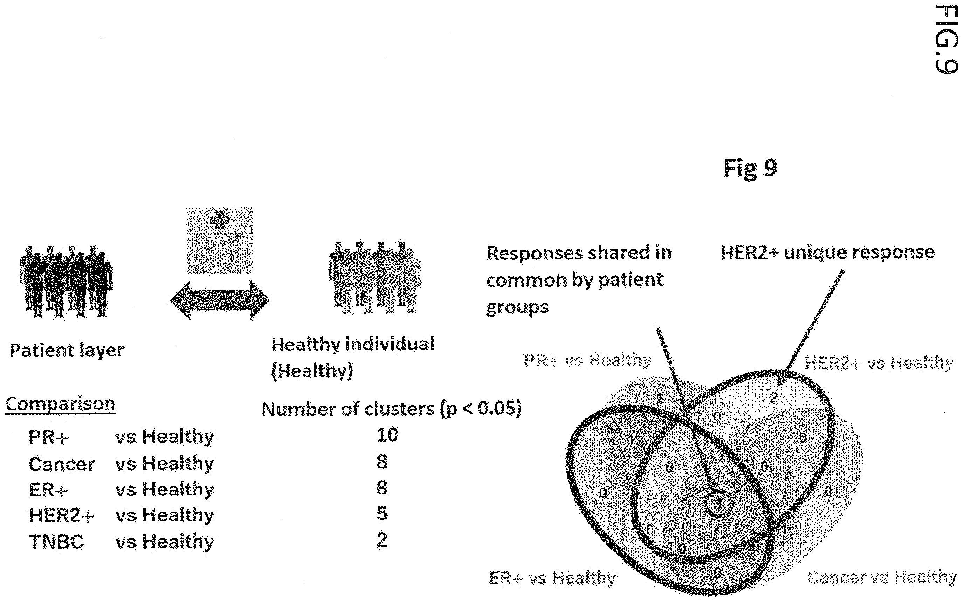

The patient group was divided into Cancer (all patients), HER2+ (HER2+ patients) ER+ (ER+ patients), and PR+ (PR+ patients), including duplicates. A cluster with a statistically significant difference in expression from Healthy (healthy individuals) was searched. The machine learning model optimized in Example 2 was applied, and the difference in expression was estimated by Fisher's exact test (p < 0.05). As a result, immune responses of the cancer patients were separated into immune responses unique to each cancer patient group and common immune responses (Figure **9****).**

### (Example 7: TCR clustering based on sequence similarity)

This Example extracted features using the sequence similarity of CDR3 for clustering, as a modified method of Example 4.

### (Data set)

The same peripheral blood CD8+ T cell receptor β chain sequence information as Example 4 on 20 breast cancer patients and 6 healthy individuals that was reported in D.J. Munson, et al., PNAS 113(29) 8272-8277, 2016 and uploaded to and published on the Gene Expression Omnibus (GEO) database was used.

### (Implementation)

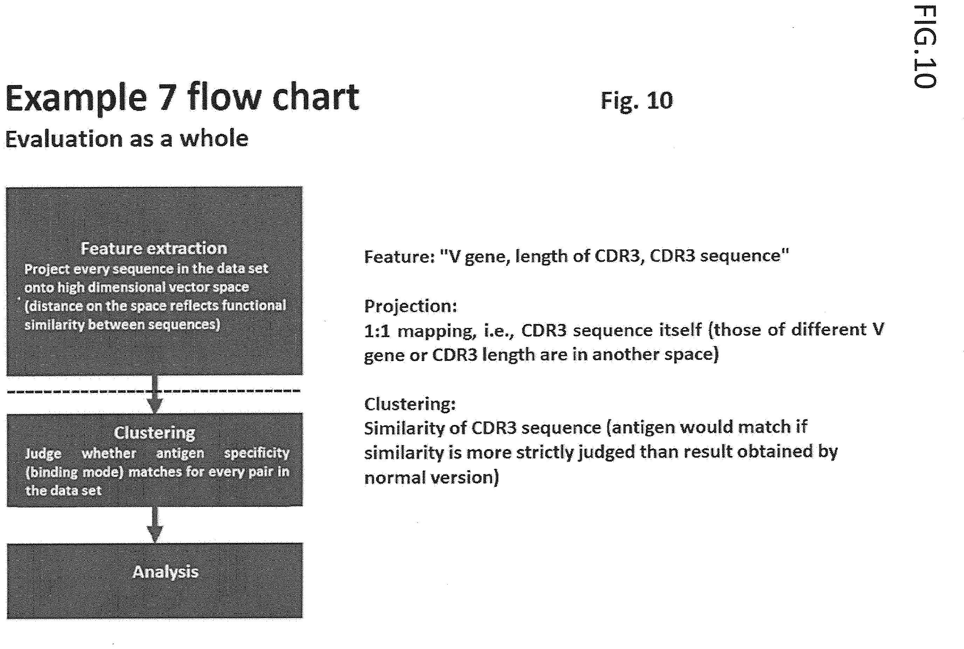

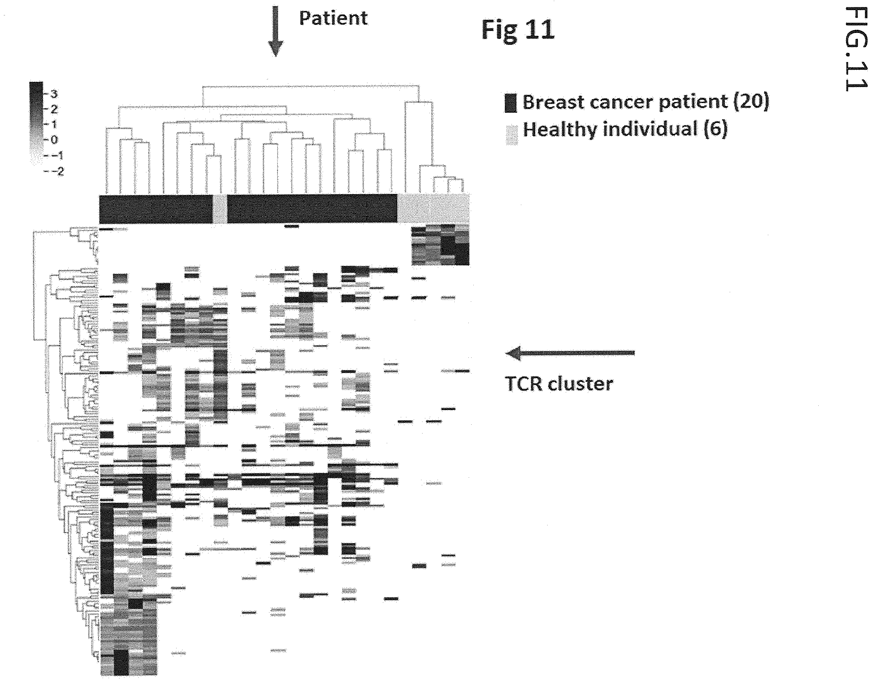

The peripheral blood CD8+ T cell receptor β chain sequence information of the data set was divided by V gene and CDR3 lengths. In this regard, the V gene sequence and the amino acid sequence of CDR3 region are based on the definition of IMGT. Each of the divided data set was clustered based on sequence homology with CD-HIT. In this regard, CD-HIT was applied to the CDR3 sequence, and the threshold value for sequence homology as set to 80%. Only clusters that appeared in 4 or more donors were analyzed. Each donor was phylogenetically analyzed based on the clusters (Figure **10****).** In this regard, phylogenetic analysis used UPGMA (Figure **11****).**

Breast cancer patients and healthy individuals were able to be divided using peripheral blood CD8+ T cell receptor β chain sequence information

### (Application to diagnosis)

When the results of this Example was applied to the same diagnosis in Example 6, the immune responses of cancer patients were confirmed to be divided into immune responses unique to each cancer patient group and common immune responses in the same manner as Example 6, demonstrating the high versatility of the present invention.

### (Example 8: Prediction of immune checkpoint inhibitor side effect)

This Example identified TCR clusters unique to a specific side effect by comparison with a healthy individual sample to perform side effect prediction and diagnosis.

### (Data set)

An immune checkpoint inhibitor was administered to lung cancer patients responsive to an immune checkpoint inhibitor. After two weeks from administration, and after 1 month or 3 months as a follow up, mononuclear cells (PBMC) were obtained from peripheral blood. Since a specific side effect was subsequently manifested in two patients, specimens obtained from these two patients were used. The T cell receptor β chain sequence used in Example 4 was also used as a comparison.

### (Results)

Patient HLA typing was performed from two lung cancer patient specimens. Furthermore, the HLA supertype suspected of being associated with said side effect was identified by referring to a known document on patients with a similar side effect. Next, data for a donor with the same HLA supertype was extracted from the data set referenced in Example 4 to prepare a comparison set. There were 65 cases of said donor.

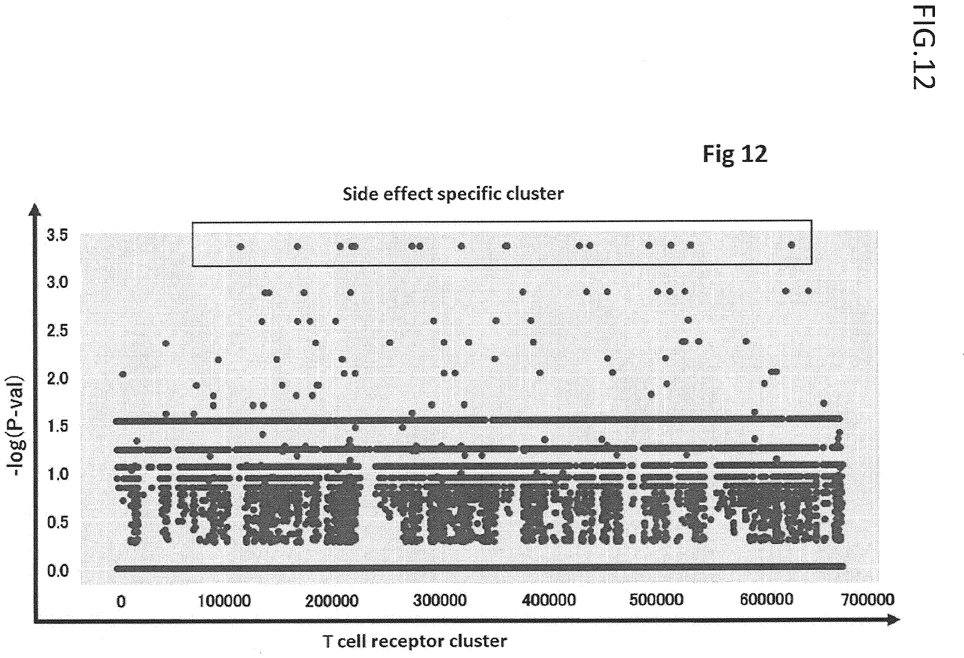

The comparison set and lung cancer patient specimens (after two weeks from administration and follow up) were clustered. The same clustering used in Example 5 was applied. The number of sequences of the side effect group and the healthy individual group contained in each cluster was compared to identify a cluster with a significantly high number of sequences of lung cancer patient specimens. In this regard, Fisher's exact test was used to evaluate the significant difference. As a result, 18 clusters unique to the side effect group were found (Figure **12****).**

### (Example 9: Pathogen is identified from immune cell receptor cluster obtained from a pathological specimen or peripheral blood specimen suspected of having infection)

### (Data set)

Pathological or peripheral blood specimen suspected of having infection, and data (reference data) for B cell/T cell receptor sequences with a known relationship with a specific infection were used.

### (Results)

In cases where infection is suspected but a pathogen cannot be identified by known methods such as PCR, reference data and said specimen derived sequence can be simultaneously clustered to identify the source of infection from the presence of a pathogen specific immune cell and render a definitive diagnosis.

### (Example 10: Identification of tumor-specific T cell from tumor-infiltrating T cells)

T cells that have infiltrated tumors can be divided into cells that are and are not tumor-specific. Such T cells are separated by T cell receptor clustering.

### (Data set)

Tumor-infiltrating T cells (TIL) derived from one case of melanoma patient were subjected to single cell sequencing, and the T cell receptor sequence was obtained for each cell. Reference data set used in Example 4 was utilized.

HLA typing was performed on cancer patients. Data of a donor with at least one matching HLA supertype was selected from the reference data set. As a result, data for 523 cases was obtained as a comparative data set. The comparative data set and cancer patient derived T cell receptor β chain sequence were subjected to clustering analysis. The same clustering used in Example 5 was applied. Overlap with the healthy individual derived comparative data set was found in many clusters. However, the cell count of TIL thereof was low. Meanwhile, a cluster with little overlap with the comparative data set had high cell count in TIL, indicating its tumor-specificity. Furthermore, tumor-specific clusters can be further narrowed down by studying same patient peripheral blood derived TCR clusters and excluding a cluster with a relative increase in peripheral blood. This enables identification of tumor-specific T cells by using a comparative data set.

### (Example 11: Evaluation of drug efficacy using tumor specific T cells)

The efficacy of an immune checkpoint agent or another anticancer agent is evaluated by using tumor-specific T cells identified by an Example described above or another method (e.g., sequence unique to a cancer patient obtained experimentally or by comparison with a healthy individual).

### (Data set)

The number of tumor-specific T cell clusters after administration of a specific agent or the number of sequences is measured by using T cell receptor sequence derived from cancer tissue or peripheral blood obtained from a patient administered with the agent. An efficacy evaluation indicator for an agent can be constructed by linking the correlation of efficacy of agent with the presence of a specific cluster, number of tumor-specific T cell clusters, or the number of sequences.

### (Notes)

### Abbreviations

- TCR:: T cell receptor
- ML:: Machine learning
- CDR:: Complementarity-determining region(s)
- MCC:: Matthews correlation coefficient
- BLOSUM:: BLOcks SUbstitution Matrix
- a.a.:: amino acid

### (Notes)

As disclosed above, the present invention has been exemplified by the use of its preferred embodiments. However, it is understood that the scope of the present invention should be interpreted based solely on the Claims. It is also understood that any patent, any patent application, and any other references cited herein should be incorporated herein by reference in the same manner as the contents are specifically described herein. The present application claims priority to Japanese Patent Application No. 2018-49440 filed on March 16, 2018 with the Japan Patent Office. The entire content thereof is incorporated herein by reference.

### [Industrial Applicability]

Highly accurate clinical application to immunity related diseases is possible.

### [Sequence Listing Free Text]

SEQ ID NO: 1: EBV derived epitope (FLRGRAYGL)

## Claims

1. A method of analyzing a collection of immunological entities, comprising the steps of:
(i) providing a feature of at least two immunological entities;
(ii) subjecting analysis of antigen specificity or binding mode of the immunological entities to machine learning without specifying antigen specificity or binding mode based on the feature; and
(iii) classifying the antigen specificity or binding mode or determining whether the antigen specificity or binding mode is the same/different.

2. A method of analyzing a collection of immunological entities, the method comprising the steps of:
(a) extracting a feature for at least a pair of members of the collection of immunological entities;
(b) computing a distance between antigen specificities or binding modes or judging whether the antigen specificities or binding modes match for the pair by machine learning using the feature;
(c) clustering the collection of immunological entities, based on the distance; and
(d) optionally analyzing based on a classification by the clustering.

3. A method of analyzing a collection of immunological entities, the method comprising the steps of:
(aa) extracting a feature for each sequence constituting at least a pair of members of the collection of immunological entities;
(bb) projecting the feature onto a high dimensional vector space, wherein a distance on the space between the members reflects functional similarity of the members;
(cc) clustering the collection of immunological entities based on the distance; and
(dd) optionally analyzing based on a classification by the clustering.

4. The method of claim 1 or 2, wherein the feature comprises at least one selected from the group consisting of sequence information, lengths of CDR1-3 sequences, a degree of match between sequences, a degree of match between sequences of framework regions, a total charge/hydrophilicity/hydrophobicity/number of aromatic amino acids of a molecule, a charge/hydrophilicity/hydrophobicity/number of aromatic amino acids of each CDR or framework region, number of each amino acid, a combination of heavy chain-light chain, number of somatic hypermutations, a position of a mutation, presence/degree of match of an amino acid motif, a degree of rarity with respect to a reference sequence set, and odds of bound HLA according to a reference sequence.

5. The method of any one of claims 1, 2, and 4, wherein the immunological entities are antibodies, antigen binding fragments of an antibody, B cell receptors, fragments of a B cell receptor, T cell receptors, fragments of a T cell receptor, chimeric antigen receptors (CARs), or cells comprising any one or more of the same.

6. The method of claim 2,
wherein calculation by machine learning uses the feature as an input and is performed by random forest or boosting, and
wherein the clustering is performed based on a simple threshold value based on a binding distance, or by a hierarchical clustering method or a non-hierarchical clustering method.

7. The method of claim 2 or 6, wherein the analysis comprises one or more of identification of a biomarker and identification of an immunological entity that is a therapeutic target or a cell comprising the immunological entity.

8. The method of any one of claims 1-2 and 4-7, wherein the machine learning is selected from the group consisting of machine learning algorithms such as a regressive scheme, a neural network method, support vector machine, and random forest.

9. The method of claim 3, wherein the feature comprises at least one selected from the group consisting of sequence information, lengths of CDR1-3 sequences, a degree of match between sequences, a degree of match between sequences of framework regions, a total charge/hydrophilicity/hydrophobicity/number of aromatic amino acids of a molecule, a charge/hydrophilicity/hydrophobicity/number of aromatic amino acids of each CDR or framework region, number of each amino acid, a combination of heavy chain-light chain, number of somatic hypermutations, a position of a mutation, presence/degree of match of an amino acid motif, a degree of rarity with respect to a reference sequence set, and odds of bound HLA according to a reference sequence.

10. The method of claim 3 or 9, wherein the immunological entities are antibodies, antigen binding fragments of an antibody, B cell receptors, fragments of a B cell receptor, T cell receptors, fragments of a T cell receptor, chimeric antigen receptors (CARs), or cells comprising any one or more of the same.

11. The method of any one of claims 3, 9, and 10,
wherein the step of projecting calculation onto a high dimensional vector space (bb) is performed by a supervised, semi-supervised (Siamese network), or unsupervised (Autoencoder) method, and
wherein the step of clustering (cc) is performed based on a simple threshold value based on a distance on a high dimensional space, or by a hierarchical clustering method or a non-hierarchical clustering method.

12. The method of any one of claims 3 and 9 to 11, wherein the analysis comprises one or more of identification of a biomarker and identification of an immunological entity that is a therapeutic target or a cell comprising the immunological entity.

13. A program for having a computer execute the method of any one of claims 1 to 12.

14. A recording medium storing a program for having a computer execute the method of any one of claims 1 to 12.

15. A system comprising a program for having a computer execute the method of any one of claims 1 to 12.

16. The method of any one of claims 1 to 2 and 4 to 7, comprising the step of associating the antigen specificity or binding mode with biological information.

17. A method of generating a cluster of antigen specificity or binding mode, comprising the step of classifying immunological entities with the same antigen specificity or binding mode to the same cluster using the method of any one of claims 1 to 2 and 4 to 7.

18. A method of identifying a disease, disorder, or biological condition, comprising the step of associating a carrier of the immunological entity with a known disease, disorder, or biological condition based on a cluster generated by the method of claim 17.

19. A composition for identifying the biological information, comprising an immunological entity with antigen specificity or binding mode identified based on the method of claim 16.

20. A composition for diagnosing a disease, disorder, or biological condition, comprising an immunological entity with antigen specificity or binding mode identified based on the method of any one of claims 1 to 12.

21. A composition for treating or preventing a disease, disorder, or biological condition, comprising an immunological entity with antigen specificity or binding mode identified based on the method of any one of claims 1 to 12.

22. A composition for diagnosing a disease, disorder, or biological condition, comprising an immunological entity binder corresponding to an epitope identified based on the method of any one of claims 1 to 12.

23. A composition for treating or preventing a disease, disorder, or biological condition, comprising an immunological entity binder corresponding to an epitope identified based on the method of any one of claims 1 to 12.

24. The composition of claim 23, wherein the composition comprises a vaccine.

25. A method for diagnosing a disease, disorder, or biological condition, comprising the step of diagnosing based on an immunological entity with antigen specificity or binding mode identified based on the method of any one of claims 1 to 12.

26. A method for judging an adverse event for a disease, disorder, or biological condition, comprising the step of determining an adverse event based on an immunological entity with antigen specificity or binding mode identified based on the method of any one of claims 1 to 12.

27. A method for diagnosing a disease, disorder, or biological condition, comprising the step of diagnosing based on an immunological entity with antigen specificity or binding mode identified based on the method of any one of claims 1 to 12, wherein the at least two immunological entities or the collection of immunological entities comprise at least one immunological entity derived from a healthy individual.

28. A method for treating or preventing a disease, disorder, or biological condition, comprising the step of administering an effective amount of an immunological entity with antigen specificity or binding mode identified based on the method of any one of claims 1 to 12.

29. A method for treating or preventing a disease, disorder, or biological condition, comprising the step of administering to a subject an effective amount of an immunological entity with antigen specificity or binding mode identified based on the method of any one of claims 1 to 12, wherein the subject excludes a subject determined as a subject who can have an adverse event based on the method of any one of claims 1 to 12.

30. A method for treating or preventing a disease, disorder, or biological condition, comprising the step of administering an effective amount of an immunological entity with antigen specificity or binding mode identified based on the method of any one of claims 1 to 12, wherein the at least two immunological entities or the collection of immunological entities comprise at least one immunological entity derived from a healthy individual.

31. A method for diagnosing a disease, disorder, or biological condition, comprising the step of diagnosing based on an immunological entity binder corresponding to an epitope identified based on the method of any one of claims 1 to 12.

32. A method for judging an adverse event for a disease, disorder, or biological condition, comprising the step of determining an adverse event based on an immunological entity binder corresponding to an epitope identified based on the method of any one of claims 1 to 12.

33. A method for diagnosing a disease, disorder, or biological condition, comprising the step of diagnosing based on an immunological entity binder corresponding to an epitope identified based on the method of any one of claims 1 to 12, wherein the at least two immunological entities or the collection of immunological entities comprise at least one immunological entity derived from a healthy individual.

34. A method for treating or preventing a disease, disorder, or biological condition, comprising the step of administering an effective amount of an immunological entity binder corresponding to an epitope identified based on the method of any one of claims 1 to 12.

35. A method for treating or preventing a disease, disorder, or biological condition, comprising the step of administering an effective amount of an immunological entity binder corresponding to an epitope identified based on the method of any one of claims 1 to 12, wherein the subject excludes a subject determined as a subject who can have an adverse event based on the method of any one of claims 1 to 12.

36. A method for treating or preventing a disease, disorder, or biological condition, comprising the step of administering an effective amount of an immunological entity binder corresponding to an epitope identified based on the method of any one of claims 1 to 12, wherein the at least two immunological entities or the collection of immunological entities comprise at least one immunological entity derived from a healthy individual.

37. The method of any one of claims 34 to 36, wherein the immunological entity binder comprises a vaccine.

38. A method for diagnosing a disease, disorder, or biological condition, comprising the steps of:
(i) providing a feature of at least two immunological entities;
(ii) subjecting analysis of antigen specificity or binding mode of the immunological entities to machine learning without specifying antigen specificity or binding mode based on the feature;
(iii) classifying the antigen specificity or binding mode or determining whether the antigen specificity or binding mode is the same/different; and
(iv) judging a disease, disorder, or biological condition based on the immunological entities classified or determined in (iii).

39. A method for diagnosing a disease, disorder, or biological condition, the method comprising the steps of:
(a) extracting a feature for at least a pair of members of the collection of immunological entities;
(b) computing a distance between antigen specificities or binding modes or judging whether the antigen specificities or binding modes match for the pair by machine learning using the feature;
(c) clustering the collection of immunological entities based on the distance;
(d) analyzing based on a classification by the clustering; and
(e) judging a disease, disorder, or biological condition based on the immunological entities analyzed in (d).

40. A method for diagnosing a disease, disorder, or biological condition, method comprising the steps of:
(aa) extracting a feature for each sequence constituting at least a pair of members of the collection of immunological entities;
(bb) projecting the feature onto a high dimensional vector space, wherein a distance on the space between the members reflects functional similarity of the members;
(cc) clustering the collection of immunological entities based on the distance;
(dd) analyzing based on a classification by the clustering; and
(ee) judging a disease, disorder, or biological condition based on the immunological entities analyzed in (dd).

41. A method for treating or preventing a disease, disorder, or biological condition, comprising the steps of:
(i) providing a feature of at least two immunological entities;
(ii) subjecting analysis of antigen specificity or binding mode of the immunological entities to machine learning without specifying antigen specificity or binding mode based on the feature;
(iii) classifying the antigen specificity or binding mode or determining whether the antigen specificity or binding mode is the same/different; and
(iv) administering the immunological entities classified or determined in (iii) or an immunological entity binder corresponding to the immunological entities.

42. A method for treating or preventing a disease, disorder, or biological condition, the method comprising the steps of:
(a) extracting a feature for at least a pair of members of the collection of immunological entities;
(b) computing a distance between antigen specificities or binding modes or judging whether the antigen specificities or binding modes match for the pair by machine learning using the feature;
(c) clustering the collection of immunological entities based on the distance;
(d) optionally analyzing based on a classification by the clustering; and
(e) administering the immunological entities analyzed in (d) or an immunological entity binder corresponding to the immunological entities.

43. A method for treating or preventing a disease, disorder, or biological condition, the method comprising the steps of:
(aa) extracting a feature for each sequence constituting at least a pair of members of the collection of immunological entities;
(bb) projecting the feature onto a high dimensional vector space, wherein a distance on the space between the members reflects functional similarity of the members;
(cc) clustering the collection of immunological entities based on the distance;
(dd) optionally analyzing based on a classification by the clustering; and
(ee) administering the immunological entities analyzed in (dd) or an immunological entity binder corresponding to the immunological entities.

44. A method for diagnosing a disease, disorder, or biological condition, comprising the steps of:
(i) providing a -feature of at least two immunological entities, wherein the at least two immunological entities comprise at least one immunological entity derived from a healthy individual;
(ii) subjecting analysis of antigen specificity or binding mode of the immunological entities to machine learning without specifying antigen specificity or binding mode based on the feature;
(iii) classifying the antigen specificity or binding mode or determining whether the antigen specificity or binding mode is the same/different; and
(iv) judging a disease, disorder, or biological condition based on the immunological entities classified or determined in (iii).

45. The method of claim 38 or 44, wherein the disease, disorder, or biological condition comprises an adverse event.

46. A method for diagnosing a disease, disorder, or biological condition, the method comprising the steps of:
(a) extracting a feature for at least a pair of members of the collection of immunological entities, wherein the collection of immunological entities comprises at least one immunological entity derived from a healthy individual;
(b) computing a distance between antigen specificities or binding modes or judging whether the antigen specificities or binding modes match for the pair by machine learning using the feature;
(c) clustering the collection of immunological entities based on the distance;
(d) analyzing based on a classification by the clustering; and
(e) judging a disease, disorder, or biological condition based on the immunological entities analyzed in (d).

47. The method of claim 39 or 46, wherein the disease, disorder, or biological condition comprises an adverse event.

48. A method for diagnosing a disease, disorder, or biological condition, the method comprising the steps of:
(aa) extracting a feature for each sequence constituting at least a pair of members of the collection of immunological entities, wherein the collection of immunological entities comprises at least one immunological entity derived from a healthy individual;
(bb) projecting the feature onto a high dimensional vector space, wherein a distance on the space between the members reflects functional similarity of the members;
(cc) clustering the collection of immunological entities based on the distance;
(dd) analyzing based on a classification by the clustering; and
(ee) judging a disease, disorder, or biological condition based on the immunological entities analyzed in (dd).

49. The method of claim 40 or 48, wherein the disease, disorder, or biological condition comprises an adverse event.

50. A method for treating or preventing a disease, disorder, or biological condition, comprising the steps of:
(i) providing a feature of at least two immunological entities, wherein the at least two immunological entities comprise at least one immunological entity derived from a healthy individual;
(ii) subjecting analysis of antigen specificity or binding mode of the immunological entities to machine learning without specifying antigen specificity or binding mode based on the feature;
(iii) classifying the antigen specificity or binding mode or determining whether the antigen specificity or binding mode is the same/different; and
(iv) administering the immunological entities classified or determined in (iii) or an immunological entity binder corresponding to the immunological entities.

51. A method of claim 41 or 50, wherein the disease, disorder, or biological condition comprises an adverse event, or the treatment or prevention comprises treating or preventing while avoiding an adverse event.

52. A method for treating or preventing a disease, disorder, or biological condition, the method comprising the steps of:
(a) extracting a feature for at least a pair of members of the collection of immunological entities, wherein the collection of immunological entities comprises at least one immunological entity derived from a healthy individual;
(b) computing a distance between antigen specificities or binding modes or judging whether the antigen specificities or binding modes match for the pair by machine learning using the feature;
(c) clustering the collection of immunological entities based on the distance;
(d) optionally analyzing based on a classification by the clustering; and
(e) administering the immunological entities analyzed in (d) or an immunological entity binder corresponding to the immunological entities.

53. The method of claim 42 or 52, wherein the disease, disorder, or biological condition comprises an adverse event, or the treatment or prevention comprises treating or preventing while avoiding an adverse event.

54. A method for treating or preventing a disease, disorder, or biological condition, the method comprising the steps of:
(aa) extracting a feature for each sequence constituting at least a pair of members of the collection of immunological entities, wherein the collection of immunological entities comprises at least one immunological entity derived from a healthy individual;
(bb) projecting the feature onto a high dimensional vector space, wherein a distance on the space between the members reflects functional similarity of the members;
(cc) clustering the collection of immunological entities based on the distance;
(dd) optionally analyzing based on a classification by the clustering; and
(ee) administering the immunological entities analyzed in (dd) or an immunological entity binder corresponding to the immunological entities.

55. The method of claim 53 or 54, wherein the disease, disorder, or biological condition comprises an adverse event, or the treatment or prevention comprises treating or preventing while avoiding an adverse event.
